# EUROPEAN PATENT APPLICATION

(11) **EP 4 706 780 A2**
(43) Date of publication of application: **11.03.2026**
(21) Application number: 26152331.0
(22) Date of filing: 09.12.2021
(51) Int. Cl.: A61Q 17/04

(54) **COMPOSITIONS COMPRISING UV-FILTERS AND ONE OR MORE (BIO)-ALKANEDIOLS**

(30) Priority: 09.12.2020 WO PCT/EP2020/085167
(62) Divisional of application: 21830677.7
(71) Applicant: Symrise AG, 37603 Holzminden (DE)
(72) Inventor: LANGE, Sabine, 37603 Holzminden (DE); BUGDAHN, Nikolas, 37603 Holzminden (DE); SCHADE, Vanessa, 37603 Holzminden (DE)
(74) Representative: Global IP Europe Patentanwaltskanzlei

(57) **Abstract**

The present invention refers in general to the area of sun care products and relates in particular to a cosmetic or pharmaceutical, in particular dermatological, composition or a homecare product comprising or consisting of a solid UV filter and an effective amount of 1,2-heptanediol and/or 2,3-heptanediol or of a specific alkanediol or a mixture of two or more different specific alkanediols and the use of said compositions as a cosmetic, for personal care, as a pharmaceutical, for animal care or as a homecare product. Additionally, the present invention relates to the use of 1,2-heptanediol and/or 2,3-heptanediol or of a specific alkanediol or a mixture of two or more different specific alkanediols for improving the solubility of a solid UV filter, and/or for improving the availability of a solid UV filter in a cosmetic or pharmaceutical composition or homecare product.

## Description

### Technical field

The present invention refers in general to the area of sun care products and relates in particular to a cosmetic or pharmaceutical, in particular dermatological, composition or a homecare product comprising or consisting of a solid UV filter and an effective amount of 1,2-heptanediol and/or 2,3-heptanediol or of a specific alkanediol or a mixture of two or more different specific alkanediols and the use of said compositions as a cosmetic, for personal care, as a pharmaceutical, for animal care or homecare. Additionally, the present invention relates to the use of 1,2-heptanediol and/or 2,3-heptanediol or of a specific alkanediol or a mixture of two or more different specific alkanediols for improving the solubility of a solid UV filter, and/or for improving the availability of a solid UV filter in a cosmetic or pharmaceutical composition or homecare product.

### Background Art

UV filters are compounds which have a pronounced absorption capacity for ultraviolet radiation. They are used especially as sunscreens in cosmetic, dermatological and pharmacological preparations, but also to improve the light fastness of industrial products, such as paints, varnishes, plastics, textiles, polymers such as, for example, polymers and copolymers of mono- and di-olefins, polystyrenes, polyurethanes, polyamides, polyesters, polyureas and polycarbonates, packaging materials and rubbers.

UV rays are classified according to their wavelength as UVA rays (320 to 400 nm) or UVB rays (280 to 320 nm). UV rays can cause acute and chronic damage to the skin, the type of damage depending on the wavelength of the radiation. For instance, UVB radiation can cause sunburn (erythema) extending to most severe burning of the skin. Other harmful effects of UVB rays include reduction in enzyme activities, weakening of the immune system, disturbances of the DNA structure and changes in the cell membrane. UVA rays penetrate into deeper layers of the skin where they can accelerate the aging process of the skin. The shorter wave UVA radiation additionally contributes to the development of sunburn. Moreover, UVA radiation can trigger phototoxic or photo allergic skin reactions. Frequent and unprotected irradiation of the skin by sunlight leads to a loss of skin elasticity and to increased development of wrinkles and in extreme cases, pathogenic changes in the skin extending to skin cancer are observed. To attenuate these negative effects of UV radiation, substances which absorb or reflect UV light, generally called UV filters, are used in cosmetic, dermatological and pharmacological preparations. The UV filters are classified as UVA and UVB filters depending on the location of their absorption maxima; if a UV filter absorbs both UVA and UVB, it is referred to as a UVA/B broadband absorber.

The degree of efficacy of cosmetic, especially dermatological, and pharmacological preparations for protection of the human skin from the erythema which is induced by UV radiation is determined by their Sun Protection Factor (SPF), which is the ratio of the energy required to show the first defined redness (erythema) of human skin which has been protected to the energy required to show the first defined redness of human skin which has not been protected. The amount of energy required to the first signs of erythema on human skin of Fitzpatrick classification 2 (light Caucasian) is 200J/m² which is also known as the Minimal Erythemal Dose (MED). Natural sunlight at 40 °N on a clear day will deliver this dose in about 10 minutes. Thus, a cosmetic, dermatological or pharmacological preparation for the protection of the human skin from the erythema with an SPF of 50 will theoretically protect skin from burning for 500 minutes. In addition to the SPF most regulatory authorities also stipulate that the formulations designed to protect human skin from erythema should also have sufficient absorption in the UVA range of the spectrum.

In order to achieve the desired protection from UV radiation cosmetic, especially dermatological, and pharmacological preparations contain a mixture of UV filters with varying concentrations and the choice of UV filters used is determined by the legislation within the country or economic area. For example, UV filters which can be used for the protection of skin are regulated in the USA by the America FDA via their OTC monograph system and are regulated in the European Union by the Cosmetic Regulation. Regulations covering the use of UV filters exist in other countries and regions as well. These regulations not only stipulate the filters which can be used but also fix a maximum usage level for each UV filter. Thus, there are limited UV filters available to achieve high efficacy with respect to both SPF and UVA or UVB protection.

One of the ways of achieving high SPF is to incorporate high levels of UVA and UVB sunscreens. A disadvantage of incorporating high levels of sunscreens is the high cost of such sunscreens thus making the composition expensive. Sensory properties are also reported to get affected of incorporation of high levels of sunscreens. Hence, there is a problem in achieving high SPF, while keeping the total amount of sunscreens in the compositions relatively low.

Furthermore, the most common UV filters available on the market are solids and they have melting points above 40 °C rendering them with a compromised solubility when used at the maximum allowed concentrations. In comparison to water soluble UV filters, solid UV filter have a poor solubility. In particular in dispersions, preferably emulsions, solid UV filters have a poor solubility or tend to recrystallize out during storage. Simultaneously, sunscreen and suncare products such as creams, lotions and oils should have to feel pleasant, so that end users enjoy using them. Therefore, sufficiently cosmetically accepted solvents must be used to ensure that they remain dissolved in a final formulation. These solid UV filters are applied to the heated oil phase in the manufacture of the formulation and this incorporation of high melting point solids requires energy and time, resulting in significant additional cost to the manufacturing process. However, solid UV filters itself are stable and storable and represent a considerable number of broadband filters, which makes them attractive for the preparation of sun care products.

EP 1 779 839 A1 discloses a cosmetic composition, comprising one or more 1,2-alkanediole and one or more specific triazin UV filter. The suggested 1,2-hexanediol, however, shows only a limited solubility for solid UV filters.

Thus, there is still an ongoing need to further improve the solubility of solid UV filters in sunscreen compositions in order to increase the availability in the composition and, thus, on skin, and in order to avoid recrystallization. The cosmetic or pharmaceutical industry is permanently searching for ingredients which can improve this negative aspect.

When searching for agents for improving solubility it must be taken into account that the substances used in the cosmetic or pharmaceutical sector must be toxicologically acceptable, well tolerated by the skin, stable, especially in the customary cosmetic and/or pharmaceutical formulations, substantially and preferably odourless and able to be prepared inexpensively, i.e. using standard methods.

However, there is no clear dependency between the chemical structure of a substance, on the one hand, and its capability in improving solubility, on the other hand, which makes the search for suitable substances more difficult. Furthermore, there is no predictable relationship between solubility improvement, toxicological acceptability, tolerability and the stability of a substance.

It is therefore an object of the present invention to provide a cosmetic or pharmaceutical, in particular dermatological, composition, comprising or consisting of a solid UV filter which has an improved solubility in the composition and which is stable, i.e. in which the UV filter remains solubilized and in which undesired crystallizing-out or precipitation of the UV filter is prevented even when stored over long time at low temperature.

In addition, it is an object of the present invention to provide a cosmetic or pharmaceutical sunscreen composition, which has excellent skin sensation upon topical application, i.e. improved spreading behaviour and/or reduced fatty/greasy feel and/or improved absorption on the skin.

It is another object of the present invention to provide a substance, which is capable of improving the solubility of UV filters and which improves the complex sensory properties of topical composition comprising a UV filter.

Surprisingly, it has been found that the addition of an effective amount of 1,2-heptanediol or 2,3-heptanediol or a mixture comprising 1,2-heptanediol and 2,3-heptanediol or a specific alkanediol or a mixture of two or more different specific alkanediols as defined to a cosmetic or pharmaceutical composition or homecare product comprising a solid UV filter significantly improves the solubility of the solid UV filter and results in a stable mixture without exhibiting any recrystallization of the solid UV filter component after preparation or during storage.

In particular it has been found that specific alkanediol mixtures as defined are outstanding suitable to improve solubility of a solid UV filter resulting in stable formulations which can be stored without recrystallization or precipitation of the UV filter.

In addition, the combination according to the present invention results in cosmetic or pharmaceutical compositions with excellent skin sensation upon application, too.

### Summary of the invention

In order to accomplish the above problem, the present invention provides in a first aspect a cosmetic or pharmaceutical composition or a homecare product, comprising or consisting of
(a1) 1,2-heptanediol;
(b1) at least one solid UV filter; and
(c1) optionally at least one active substance for a cosmetic or pharmaceutical composition or homecare product and/or additive;
   or
(a2) 2,3-heptanediol;
(b2) at least one solid UV filter; and
(c2) optionally at least one active substance for a cosmetic or pharmaceutical composition or homecare product and/or additive;
   or
(a3) a mixture comprising 1,2-heptanediol and 2,3-heptanediol;
(b3) at least one solid UV filter; and
(c3) optionally at least one active substance for a cosmetic or pharmaceutical composition or homecare product and/or additive.

In a second aspect, the present invention provides a cosmetic or pharmaceutical composition or a homecare product, comprising or consisting of
(a) at least one linear alkanediol having a carbon chain of 5 to 14 carbon atoms or a mixture comprising at least one first linear alkanediol having a carbon chain of 5 to 14 carbon atoms and one or more second linear alkanediol having a carbon chain of 5 to 14 carbon atoms which is different from the first linear alkanediol;
(b) at least one solid UV filter; and
(c) optionally at least one active substance for a cosmetic or pharmaceutical composition or homecare product and/or additive.

In a further aspect, the present invention provides for the use of the composition as cosmetic, for personal care, in particular for skin, hair, scalp or nail care, as pharmaceutical, for animal care or for homecare.

In a further aspect, the present invention provides for the use of 1,2-heptanediol or 2,3-heptanediol or a mixture comprising 1,2-heptanediol and 2,3-heptanediol or at least one linear alkanediol or a mixture comprising at least one first linear alkanediol and one or more second linear alkanediol for improving the solubility of a UV filter in a cosmetic or pharmaceutical composition, and, thus for improving the availability of a solid UV filter in a cosmetic or pharmaceutical composition.

### Description of Figures

Figure 1 shows the solubility of samples of Bis-Ethylhexyloxyphenol Methoxyphenyl Triazine (Bemotrizinol) samples in different 1,2-alkanediols.
Figure 2 shows the solubility of samples of Butyl Methoxydibenzoylmethane (Avobenzon) in different 1,2-alkanediols.
Figure 3 shows the solubility of samples of 4-Methylbenzyliden Camphor in different 1,2-alkanediols.
Figure 4 shows the solubility of samples of Ethylhexyl Triazone in different 1,2-alkanediols.
Figure 5 shows the solubility of samples of Diethylamino Hydroxybenzoyl Hexyl Benzoate in different 1,2-alkanediols.
Figure 6 is a microscopical image of O/W emulsion samples comprising 5 % Bis-Ethylhexyloxyphenol Methoxyphenyl Triazine (Neo Heliopan BMT) in combination with different 1,2-alkanediols after two weeks storage.
Figure 7 is a microscopical image of O/W emulsion samples comprising 3 % Ethylhexyl Triazone (Uvinul T150) in combination with different 1,2-alkanediols after two weeks storage.
Figure 8 shows the solubility of samples of Bis-Ethylhexyloxyphenol Methoxyphenyl Triazine (Neo Heliopan BMT) in 1,2-heptanediol and 2,3-heptanediol and different mixtures of 1,2-heptanediol and 2,3-heptanediol.
Figure 9 shows the solubility of samples of Bis-Ethylhexyloxyphenol Methoxyphenyl Triazine (Neo Heliopan BMT) in 1,2-octanediol and 2,3-octanediol and different mixtures of 1,2-octanediol and 2,3-octanediol.

### Detailed description of the invention

The present invention is specified in the appended claims. The invention itself, and its preferred variants, other objects and advantages, are however also apparent from the following detailed description in conjunction with the accompanying examples and figures.

The present invention relates in a first aspect to a cosmetic or pharmaceutical composition or homecare product, comprising or consisting of:
(a1) 1,2-heptanediol;
(b2) at least one solid UV filter; and
(c2) optionally at least one active substance for a cosmetic or pharmaceutical composition or homecare product and/or additive.

Alternatively, the present invention relates in a first aspect to a cosmetic or pharmaceutical composition or homecare product, comprising or consisting of:
(a2) 2,3-heptanediol;
(b2) at least one solid UV filter; and
(c2) optionally at least one active substance for a cosmetic or pharmaceutical composition or homecare product and/or additive.

In a further alternative, the present invention relates in a first aspect to a cosmetic or pharmaceutical composition or homecare product, comprising or consisting of:
(a3) a mixture comprising 1,2-heptanediol and 2,3-heptanediol;
(b3) at least one solid UV filter; and
(c3) optionally at least one active substance for a cosmetic or pharmaceutical composition or homecare product and/or additive.

In a second aspect, the present invention relates to a cosmetic or pharmaceutical composition or homecare product, comprising or consisting of:
(a) at least one linear alkanediol having a carbon chain of 5 to 14 carbon atoms or a mixture comprising at least one first linear alkanediol having a carbon chain of 5 to 14 carbon atoms and one or more second linear alkanediol having a carbon chain of 5 to 14 carbon atoms which is different from the first linear alkanediol;
(b) at least one solid UV filter; and
(c) optionally at least one active substance for a cosmetic or pharmaceutical composition or homecare product and/or additive.

In a more preferred variant of the second aspect, the present invention relates to a cosmetic or pharmaceutical composition or homecare product, comprising or consisting of:
(a') a mixture comprising at least one first linear alkanediol having a carbon chain of 5 to 14 carbon atoms and one or more second linear alkanediol having a carbon chain of 5 to 14 carbon atoms, wherein the number of the carbon atoms of the first and the second alkanediol is either same or different;
(b') at least one solid UV filter; and
(c') optionally at least one active substance for a cosmetic or pharmaceutical composition or homecare product and/or additive.

The term "comprising" means that the named components are essential, but other components may be added and is still embraced by the present invention.

The term "consisting of" as used according to the present invention means that the total amount of components (a) to (c) adds up to 100 % by weight, based on the total weight of the cosmetic or pharmaceutical composition, and signifies that the subject matter is closed-ended and can only include the limitations that are expressly recited.

Whenever reference is made to "comprising" it is intended to cover both meanings as alternatives, that is the meaning can be either "comprising" or "consisting of" unless the context dictates otherwise.

The term "at least one solid UV filter" means that the cosmetic or pharmaceutical composition or homecare product according to the present invention can comprise either one solid UV filter or a mixture of two, three, four, five, six or even more different solid UV filters.

The term "optionally" means that the subsequently described compound may but need not to be present in the composition, and that the description includes variants, were the compound is included or variants, were the compound is absent.

Alkanediols are glycols, i.e. any of a class of organic compounds belonging to the alcohol family; in the molecule of a glycol, two hydroxyl (-OH) groups are attached to different carbon atoms of a carbon chain.

The component (a) in the cosmetic or pharmaceutical composition or homecare product according to the first aspect of the present invention is either 1,2-heptanediol (a1) or 2,3-heptanediol (a2), or a mixture comprising both heptanediols, i.e. 1,2-heptanediol plus 2,3-heptanediol (a3).

1,2-heptandediol belongs to the category of alkanediols and is a straight chain alkanediol with the general formula: 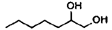 It has a carbon chain with 7 carbon atoms and the two functional OH groups of the alkanediol are in alpha,beta position and chemically bonded to the C1 and C2 carbon atoms in the alkanediol chain.

2,3-heptanediol belongs to the category of alkanediols and is a straight chain alkanediol with the general formula: 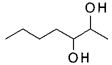 It has a carbon chain with 7 carbon atoms and the two functional OH groups of the alkanediol are in beta,gamma position and chemically bonded to the C2 and C3 carbon atoms in the alkanediol chain.

Straight chain 1,2-alkanediols have been used for more than 15 years as multifunctional actives. Short chain 1,2-alkanediols are amphiphilic compounds and thus, like 1,2-pentanediol and 1,2-hexanediol, are soluble both in water and cosmetic oils. In contrast, 1,2-octanediol is a solid and tends to precipitate or recrystallize in oily solutions. On the other hand, 1,2-decanediol is a solid and soluble only in cosmetic oils. Apart from moisturizing, some 1,2-alkanediols are used as viscosity modifiers.

The component (a) in the cosmetic or pharmaceutical composition or homecare product according to the second aspect of the present invention is in a first alternative at least one linear alkanediol having a carbon chain of 5 to 14 carbon atoms.

This second aspect linear alkanediol is preferably a 2,3-alkanediol. In such cases, an optimum balance between various properties such as skin feel of the alkanediol and the formulability is achieved for compositions comprising 2,3-alkanediols with a chain length of from C8 to C10, thus in particular C8, C9 and C10, although C11 and C12 are also possible, thus giving also preferably C8 to C12. This is advantageous for cosmetic and pharmaceutical compositions or the application of these 2,3-alkandiols in cosmetic or pharmaceutical formulations or homecare products. Further, the higher 2,3-alkandiols show good skin feel parameters. This advantage combining with strong solubility advantages affords 2,3-alkanediols that are very useful in cosmetic formulations for themselves, but also as combination partners with other alkanediols, as they improve the formulability while maintaining or even strengthening additional properties such as the formulability with UV filters, as shown in the following. Improving the skin feel of UV filter compositions is also an important factor in such applications and the 2,3-alkandiols as mentioned herein provide excellent improvements in this respect. Further antimicrobial properties are also useful.

Moreover, in a second alternative of this second aspect, the component (a) may include a first linear alkanediol in combination with a second linear alkanediol, preferably at least one first linear alkanediol having a carbon chain of 5 to 14 carbon atoms and at least one second linear alkanediol having a carbon chain of 5 to 14 carbon atoms. Therefore, the invention also entails a mixture of a first and a second alkanediol as described herein.

Since the first linear alkanediol of the invention can be selected from the same lists and types of compounds as the linear alkanediol according to the first alternative of the second aspect, in the following, reference is made to the linear alkanediol in general, which can also be the first linear alkanediol. In mixtures of first and second linear alkanediols, where these are specifically different, the specific terms "first" and "second" will be used to distinguish the two alkanediol components.

As used in this document, the phrase "at least one linear alkanediol" or "at least one first linear alkanediol" means that the composition can comprise one linear alkanediol having a carbon chain of 5 to 14 carbon atoms or can comprise one first linear alkanediol having a carbon chain of 5 to 14 carbon atoms or can comprise more than one linear alkanediol or can comprise more than one first linear alkanediol having a carbon chain of 5 to 14 carbon atoms, i.e. two, three, four or more different linear alkanediols or first linear alkanediols having a carbon chain of 5 to 14 carbon atoms.

The linear alkanediol or the first linear alkanediol consist of a chain of 5 to 14 carbon atoms joined to each other by single covalent bonds with two OH functional groups attached to two different carbon atoms in the chain.

The at least one linear alkanediol or the at least one first linear alkanediol having a carbon chain of 5 to 14 carbon atoms in the cosmetic or pharmaceutical composition or homecare product according to the second aspect of the present invention is preferably selected from the group consisting of pentanediol, hexanediol heptanediol, octanediol, nonanediol, decanediol, undecanediol, dodecanediol, tridecanediol, tetradecanediol, and mixtures thereof.

In a preferred variant, the at least one linear alkanediol or the at least one first linear alkanediol is selected from the group consisting of pentanediol, hexanediol, heptanediol, octanediol, nonanediol, decanediol, undecanediol, dodecanediol, tridecanediol, and mixtures thereof.

In a more preferred variant, the at least one linear alkanediol or the at least one first linear alkanediol is selected from the group consisting of alkanediols having an uneven number of carbon atoms of 5 to 13, i.e. pentanediol, heptanediol, nonanediol, undecanediol, tridecanediol, and mixtures thereof.

In a still more preferred variant, the at least one linear alkanediol or the at least one first linear alkanediol is selected from the group consisting of alkanediols having an uneven number of carbon atoms of 7, 9, 11 and 13, i.e. heptanediol, nonanediol, undecanediol and tridecanediol, or is selected from the group consisting of alkanediols having an uneven number of carbon atoms of 9 and 11, i.e. nonanediol and undecanediol, or is selected from the group consisting of alkanediols having an uneven number of carbon atoms of 9 and 13, i.e. nonanediol and tridecanediol, or is selected from the group consisting of alkanediols having an uneven number of carbon atoms of 11 and 13, i.e. undecanediol and tridecanediol.

In a still more preferred variant, the at least one linear alkanediol or the at least one first linear alkanediol is either pentanediol, hexanediol, heptanediol or octanediol.

In a most preferred variant, the at least one linear alkanediol or the at least first linear alkanediol is heptanediol.

In an alternative, the cosmetic or pharmaceutical composition or the homecare product according to the second aspect of the present invention comprises a mixture comprising at least one first linear alkanediol having a carbon chain of 5 to 14 carbon atoms and one or more second linear alkanediol having a carbon chain of 5 to 14 carbon atoms which is/are different from the first linear alkanediol.

This means that the alkanediol mixture is a mixture comprising at least one first linear alkanediol having a carbon chain of 5 to 14 carbon atoms and one or more, i.e. two, three, four or more second linear alkanediols having a carbon chain of 5 to 14 carbon atoms. In this alternative, the first linear alkanediol can be any of the alkanediols as described herein for the linear alkanediol in general and in particular for the first alternative of the second aspect.

For example, the mixture can include one first linear alkanediol and one, two, three or more second linear alkanediols; or the mixture can include two first linear alkanediols and one, two, three or more second linear alkanediols, etc. with the proviso, that in each mixture, the first linear alkanediols and the second linear alkanediols are different from each other.

The phrase "different from each other means" that the first linear alkanediols and the second linear alkanediols in the mixture are either different with regard to the length of their carbon chain, i.e. number of the carbon atoms, and/or with regard to their constitutional isomerism or with regard to their stereoisomerism.

Likewise, the number of the carbon atoms of the first linear alkanediols and the second linear alkanediols in the mixture can also be same. For example, the first linear alkanediol and the second linear alkanediol have a carbon chain of 7 carbon atoms, but the first linear alkanediol and the second linear alkanediol are different with regard to their constitutional isomerism or with regard to their stereoisomerism.

The second linear alkanediol preferably consists of a chain of 5 to 14 carbon atoms joined to each other by single covalent bonds with two OH functional groups attached to two different carbon atoms in the chain.

The at least one second linear alkanediol having a carbon chain of 5 to 14 carbon atoms in the cosmetic or pharmaceutical composition or the homecare product according to the second aspect of the present invention is selected from the group consisting of pentanediol, hexanediol, heptanediol, octanediol, nonanediol, decanediol, undecanediol, dodecanediol, tridecanediol, tetradecanediol and mixtures thereof.

In a preferred variant, the at least one second linear alkanediol is selected from the group consisting of pentanediol, hexanediol, heptanediol, octanediol, nonanediol, decanediol, undecanediol, dodecanediol and tridecanediol. If both the first and the second alkanediol is for example heptanediol, then the second linear alkanediol heptanediol is a different constitutional isomer or stereoisomer from the first linear alkanediol.

In a more preferred variant, the at least one second linear alkanediol is selected from the group consisting of pentanediol, hexanediol, heptanediol, octanediol and nonanediol, most preferably heptanediol, octanediol and nonanediol.

The term "alkanediol" within the context of the present invention also includes its constitutional isomers or position isomers. Constitutional isomers are compounds that have the same molecular formula and different connectivity. Position isomers, a particular form of constitutional isomerism, are structural isomers that can be viewed as differing only on the position of a functional group on a parent structure, which in this case is the position of the two alcohol functions.

Depending on the number of the carbon atoms of the carbon chain of the alkanediol, there are various position isomers of the alkanediol: (x,x+1) constitutional isomers; (x,x+2) constitutional isomers; (x,x+3) constitutional isomers; etc. and alpha,omega constitutional isomers when the alcohol functions are at the terminal ends of the carbon chain, wherein x stands for the number of the carbon atom in the alkanediol chain, to which the OH groups of the alkanediol are chemically bonded. For example: if x is 1, the two OH groups of the alkanediol are chemically bonded to the C1 and C2 carbon atoms in the alkanediol chain; if x is 2, the two OH groups of the alkanediol are chemically bonded to the C2 and C3 carbon atoms in the alkanediol chain, etc.

In the (x,x+1) constitutional isomers, the two OH functional groups are vicinal attached to two different adjacent carbon atoms in the chain. In the (x,x+2) constitutional isomers, the two OH functional groups are attached to two different carbon atoms in the chain where the two carbon atoms are separated by one C atom. In the (x,x+3) constitutional isomers, the two OH functional groups are attached to two different carbon atoms in the chain where the two carbon atoms are separated by two C atoms. In the alpha,omega constitutional isomers, the two functional groups are attached to the first C atom and to the terminal C atom.

In a preferred variant of the cosmetic or pharmaceutical composition or homecare product according to the second aspect of the present invention, the linear alkanediol having a carbon chain of 5 to 14 carbon atoms, is preferably a vicinal (x,x+1) diol, selected from the group consisting of a 1,2-diol, 2,3-diol, 3,4-diol, 4,5-diol, further (x,x+1) diols, and mixtures thereof, preferably an alpha,beta 1,2 constitutional isomer.

In a preferred variant of the cosmetic or pharmaceutical composition or homecare product according to the second aspect of the present invention, the first linear alkanediol and/or the second linear alkanediol having a carbon chain of 5 to 14 carbon atoms is a vicinal (x,x+1) diol, selected from the group consisting of a 1,2-diol, 2,3-diol, 3,4-diol, 4,5-diol, further (x,x+1) diols, and mixtures thereof, preferably an alpha,beta 1,2 constitutional isomer.

In a further preferred variant of the cosmetic or pharmaceutical composition or homecare product according to the second aspect of the present invention, the linear alkanediol having a carbon chain of 5 to 14 carbon atoms, is preferably a non-vicinal (x,x+2) diol, selected from the group consisting of a 1,3-diol, 2,4-diol, 3,5-diol, further (x,x+2) diols, and mixtures thereof, preferably an alpha,gamma 1,3 constitutional isomer.

In a preferred variant of the cosmetic or pharmaceutical composition or homecare product according to the second aspect of the present invention, the first linear alkanediol and/or the second linear alkanediol having a carbon chain of 5 to 14 carbon atoms is a non-vicinal (x,x+2) diol, selected from the group consisting of a 1,3-diol, 2,4-diol, 3,5-diol, 4,6-diol, further (x,x+2) diols, and mixtures thereof, preferably an alpha,gamma 1,3-consitutional isomer.

In a further preferred variant of the cosmetic or pharmaceutical composition or homecare product according to the second aspect of the present invention, the linear alkanediol having a carbon chain of 5 to 14 carbon atoms, is preferably a non-vicinal (x,x+3) diol, selected from the group consisting of a 1,4 diol, 2,5 diol, further (x,x+3) diols, and mixtures thereof, preferably an alpha,delta 1,4 constitutional isomer.

In a preferred variant of the cosmetic or pharmaceutical composition or homecare product according to the second aspect of the present invention, the first linear alkanediol and/or the second linear alkanediol having a carbon chain of 5 to 14 carbon atoms is a non-vicinal (x,x+3) diol, selected from the group consisting of a 1,4 diol, 2,5 diol, further (x,x+3) diols, or mixtures thereof, preferably an alpha,delta 1,4 constitutional isomer.

In a preferred variant of the cosmetic or pharmaceutical composition or homecare product according to the second aspect of the present invention, the linear alkanediol or the first linear alkanediol and/or the second linear alkanediol is preferably an alpha,omega alkanediol, more preferably, 1,7-heptanediol or 1,8-octanediol.

According to the present invention, vicinal (x,x+1) diols are most preferred, such as alpha,beta or beta,gamma or gamma,delta etc.

In a still preferred variant of the cosmetic or pharmaceutical composition or homecare product according to the second aspect of the present invention, the linear alkanediol and in particular the first linear alkanediol and/or the second linear alkanediol, is a 1,2-alkanediol, a 2,3-alkanediol, a 3,4-alkanediol, or mixtures thereof, more preferred 2,3-alkanediol.

The 1,2-alkanediols of the linear alkanediol, in particular the first linear alkanediol having a carbon chain of 5 to 14 carbon atoms and/or the second linear alkanediol having a carbon chain of 5 to 14 carbon atoms, can preferably be those as represented by the following formulae:

| | |
|---|---|
| 1,2-pentanediol | 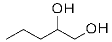 |
| 1,2-hexanediol | 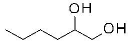 |
| 1,2-heptanediol | 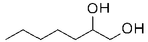 |
| 1,2-octanediol | 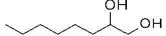 |
| 1,2-nonanediol | 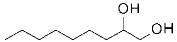 |
| 1,2-decanediol | 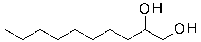 |
| 1,2-undecanediol | 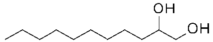 |
| 1,2-dodecanediol | 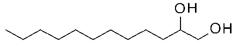 |
| 1,2-tridecanediol | 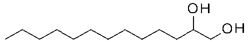 |
| 1,2-tetradecanediol | 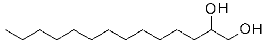 |

The 2,3-alkanediols of the linear alkanediol of the invention, in particular the first linear alkanediol having a carbon chain of 5 to 14 carbon atoms and/or the second linear alkanediol having a carbon chain of 5 to 14 carbon atoms, can preferably be those as represented by the following formulae:

| | |
|---|---|
| 2,3-pentanediol | 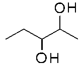 |
| 2,3-hexanediol | 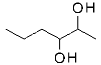 |
| 2,3-heptanediol | 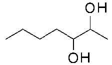 |
| 2,3-octanediol | 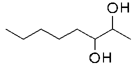 |
| 2,3-nonanediol | 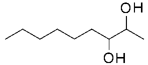 |
| 2,3-decanediol | 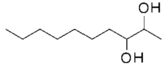 |
| 2,3-undecanediol | 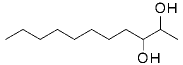 |
| 2,3-dodecanediol | 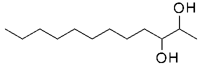 |
| 2,3-tridecanediol | 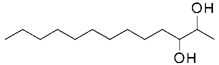 |
| 2,3-tetradecanediol | 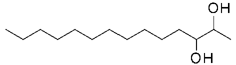 |

The 3,4-alkanediols of the linear alkanediol, preferably the first linear alkanediol having a carbon chain of 5 to 14 carbon atoms and/or the second linear alkanediol having a carbon chain of 5 to 14 carbon atoms, can preferably be those as represented by the following formulae:

| | |
|---|---|
| 3,4-hexanediol | 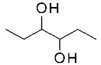 |
| 3,4-heptanediol | 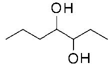 |
| 3,4-octanediol | 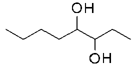 |
| 3,4-nonanediol | 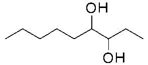 |
| 3,4-decanediol | 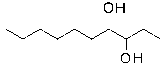 |
| 3,4-undecanediol | 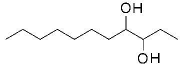 |
| 3,4-dodecanediol | 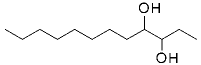 |
| 3,4-tridecanediol | 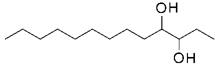 |
| 3,4-tetradecanediol | 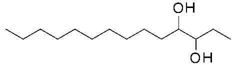 |

In a preferred variant, the cosmetic or pharmaceutical composition according to the second aspect of the present invention comprises a mixture comprising at least one first linear alkanediol having a carbon chain of 5 to 14 carbon atoms and one or more second linear alkanediol having a carbon chain of 5 to 14 carbon atoms wherein the number of the carbon atoms of the first and the second alkanediol is either same or different.

If both, the first and the second alkanediol have the same number of carbon atoms, such an alkanediol combination is herein also referred to as "homo alkanediol mixture" or "homo combination". For example, the first linear alkanediol and the second linear alkanediol have a carbon chain of 7 carbon atoms, but the first linear alkanediol and the second linear alkanediol are different with regard to their constitutional isomerism or with regard to their stereoisomerism.

If the first and the second alkanediol have a different number of carbon atoms, such an alkanediol combination is herein also referred to as "hetero alkanediol mixture" or "hetero combination". For example, the first linear alkanediol has a carbon chain of 7 carbon atoms and the second linear alkanediol has a carbon chain of 8 carbon atoms. However, beyond that, the first linear alkanediol and the second linear alkanediol can be different with regard to their constitutional isomerism or with regard to their stereoisomerism.

In a preferred variant of the cosmetic or pharmaceutical composition according to the second aspect of the present invention, the linear alkanediol or the first linear alkanediol is selected from the group consisting of:
1,2-pentanediol, 2,3-pentanediol, 3,4-pentanediol,
1,2-hexanediol, 2,3-hexanediol, 3,4-hexanediol,
1,2-heptanediol, 2,3-heptanediol, 3,4-heptanediol,
1,2-octanediol, 2,3-octanediol, 3,4-octanediol,
1,2-nonanediol, 2,3-nonanediol, 3,4-nonanediol,
1,2-decanediol, 2,3-decanediol, 3,4-decanediol,
1,2-undecanediol, 2,3-undecanediol, 3,4-undecanediol,
1,2-dodecanediol, 2,3-dodecanediol, 3,4-dodecanediol,
1,2-tridecanediol, 2,3-tridecanediol, 3,4-tridecanediol, and mixtures thereof.

Of the aforesaid linear alkanediols or first linear alkanediols, the following (x,x+1) constitutional isomers are preferred: 1,2-pentanediol, 2,3-pentanediol, 1,2-hexanediol, 2,3-hexanediol, 1,2-heptanediol, 2,3-heptanediol, 2,3-octanediol, 2,3-nonanediol, 2,3-decanediol, 2,3-undecanediol, 2,3-dodecanediol, 2,3-tridecanediol or mixtures thereof. Said alkanediols are liquid at a purity of 90 to 99 %.

Of the aforesaid liquid alkanediols 1,2-pentanediol, 2,3-pentanediol, 1,2-hexanediol, 2,3-hexanediol, 1,2-heptanediol, 2,3-heptanediol, 2,3-octanediol, 2,3-nonanediol or mixtures of said liquid alkanediols are particularly preferred. Said alkanediols can be easier incorporated into semi-finished products or final products containing a solid UV filter.

In a more preferred variant, the at least one linear alkanediol or the at least one first linear alkanediol is selected from the group consisting of 1,2-alkanediols having an uneven number of carbon atoms of 5 to 13, i.e. 1,2-pentanediol, 1,2-heptanediol, 1,2-nonanediol, 1,2-undecanediol and 1,2-tridecanediol.

In a more preferred variant, the linear alkanediol or the first linear alkanediol is selected from the group consisting of 1,2-alkanediols having an uneven number of carbon atoms of 7, 9, 11 and 13, i.e. 1,2-heptanediol, 1,2-nonanediol, 1,2-undecanediol or 1,2-tridecanediol, or is selected from the group consisting of 1,2-alkanediols having an uneven number of carbon atoms of 9 and 11, i.e. 1,2-nonanediol and 1,2-undecanediol, or is selected from the group consisting of 1,2-alkanediols having an uneven number of carbon atoms of 9 and 13, i.e. 1,2-nonanediol and 1,2-tridecanediol, or is selected from the group consisting of 1,2-alkanediols having an uneven number of carbon atoms of 11 and 13, i.e. 1,2-undecanediol and 1,2-tridecanediol.

In a more preferred variant, the at least one linear alkanediol or the at least one first linear alkanediol is selected from the group consisting of 2,3-alkanediols having an uneven number of carbon atoms of 5 to 13, i.e. 2,3-pentanediol, 2,3-heptanediol, 2,3-nonanediol, 2,3-undecanediol and 2,3-tridecanediol.

In a still more preferred variant, the linear alkanediol or the first linear alkanediol is selected from the group consisting of 2,3-alkanediols having an uneven number of carbon atoms of 7, 9, 11 and 13, i.e. 2,3-heptanediol, 2,3-nonanediol, 2,3-undecanediol or 2,3-tridecanediol, or is selected from the group consisting of 2,3-alkanediols having an uneven number of carbon atoms of 9 and 11, i.e. 2,3-nonanediol and 2,3-undecanediol, or is selected from the group consisting of 2,3-alkanediols having an uneven number of carbon atoms of 9 and 13, i.e. 2,3-nonanediol and 2,3-tridecanediol, or is selected from the group consisting of 2,3-alkanediols having an uneven number of carbon atoms of 11 and 13, i.e. 2,3-undecanediol and 2,3-tridecanediol.

In a further preferred variant of the cosmetic or pharmaceutical composition or homecare product according to the second aspect of the present invention, the linear alkanediol or the first linear alkanediol is selected from the group consisting of:
1,2-pentanediol, 2,3-pentanediol, 3,4-pentanediol,
1,2-hexanediol, 2,3-hexanediol, 3,4-hexanediol,
1,2-heptanediol, 2,3-heptanediol, 3,4-heptanediol,
1,2-octanediol, 2,3-octanediol, 3,4-octanediol,
1,2-nonanediol, 2,3-nonanediol, 3,4-nonanediol and mixtures thereof.

More preferred, in the cosmetic or pharmaceutical composition or homecare product according to the second aspect of the present invention, the linear alkanediol or the first linear alkanediol is selected from the group consisting of 1,2-pentanediol, 2,3-pentanediol, 3,4-pentanediol and mixtures thereof or is selected from the group consisting of 1,2-heptanediol, 2,3-heptanediol, 3,4-heptanediol, and mixtures thereof. However, the linear alkanediol or the first linear alkanediol can also preferably be selected from the group consisting of 1,2-octanediol, 2,3-octanediol, 3,4-octanediol, and mixtures thereof.

Even more preferred, in the cosmetic or pharmaceutical composition or the homecare product according to the second aspect of the present invention, the linear alkanediol or the first linear alkanediol is an alpha,beta or a beta,gamma diol as either 1,2-heptanediol, 2,3-heptanediol, or a mixture thereof. However, the linear alkanediol or the first linear alkanediol can also preferably be an alpha,beta or beta,gamma diol as either 1,2-octanediol, 2,3-octanediol, or a mixture thereof. A mixture comprising 1,2-heptanediol and 2,3-octanediol or a mixture comprising 1,2-octanediol and 2,3-heptanediol is also possible.

Most preferred, the linear alkanediol or the first linear alkanediol is 1,2-pentanediol, 1,2-hexanediol, 1,2-heptanediol, 1,2-octanediol or 1,2-nonanediol.

Likewise, in a further preferred variant of the cosmetic or pharmaceutical composition or homecare product according to the second aspect of the present invention, the linear alkanediol or the first linear alkanediol is selected from the group consisting of:
1,2-nonanediol, 2,3-nonanediol, 3,4-nonanediol,
1,2-decanediol, 2,3-decanediol, 3,4-decanediol,
1,2-undecanediol, 2,3-undecanediol, 3,4-undecanediol, and mixtures thereof.

More preferred, in the cosmetic or pharmaceutical composition or the homecare product according to the second aspect of the present invention, the linear alkanediol or the first linear alkanediol is selected from the group consisting of 1,2-nonanediol, 2,3-nonanediol, 3,4-nonanediol, and mixtures thereof, or can also be preferably selected from the group consisting of 1,2-decanediol, 2,3-decanediol, 3,4-decanediol, and mixtures thereof, or can also be preferably selected from the group consisting of 1,2-undecanediol, 2,3-undecanediol, 3,4-undecanediol, and mixtures thereof.

Even more preferred, in the cosmetic or pharmaceutical composition or homecare product according to the second aspect of the present invention, the linear alkanediol or the first linear alkanediol is an alpha,beta or a beta,gamma diol as either 1,2-nonanediol, 2,3-nonanediol, or a mixture thereof. However, the linear alkanediol or the first linear alkanediol can also preferably be 1,2-decanediol, 2,3-decanediol, or a mixture thereof.

Additionally, the linear alkanediol or the first linear alkanediol can also preferably be 1,2-undecanediol, 2,3-undecanediol, or a mixture thereof. A mixture comprising 1,2-nonanediol and/or 2,3-decanediol and/or 2,3-undecanediol or a mixture comprising 1,2-decanediol and/or 2,3-nonanediol and/or 2,3-undecanediol or a mixture comprising 1,2-undecanediol and/or 2,3-nonanediol and 2,3-decanediol is also possible.

In a preferred variant of the cosmetic or pharmaceutical composition or the homecare product according to the second aspect of the present invention, the second linear alkanediol is selected from the group consisting of:
1,2-pentanediol, 2,3-pentanediol, 3,4-pentanediol,
1,2-hexanediol, 2,3-hexanediol, 3,4-hexanediol,
1,2-heptanediol, 2,3-heptanediol, 3,4-heptanediol,
1,2-octanediol, 2,3-octanediol, 3,4-octanediol,
1,2-nonanediol, 2,3-nonanediol, 3,4-nonanediol,
1,2-decanediol, 2,3-decanediol, 3,4-decanediol,
1,2-undecanediol, 2,3-undecanediol, 3,4-undecanediol,
1,2-dodecanediol, 2,3-dodecanediol, 3,4-dodecanediol,
1,2-tridecanediol, 2,3-tridecanediol, 3,4-tridecanediol, and mixtures thereof.

Of the aforesaid second linear alkanediols, the following (x,x+1) constitutional isomers are preferred: 1,2-pentanediol, 2,3-pentanediol, 1,2-hexanediol, 2,3-hexanediol, 1,2-heptanediol, 2,3-heptanediol, 2,3-octanediol, 2,3-nonanediol, 2,3-decanediol, 2,3-undecanediol, 2,3-dodecanediol, 2,3-tridecanediol, or mixtures thereof. Said alkanediols are liquid at a purity of 90 to 99 %.

Of the aforesaid liquid alkanediols 1,2-pentanediol, 2,3-pentanediol, 1,2-hexanediol, 2,3-hexanediol, 1,2-heptanediol, 2,3-heptanediol, 2,3-octanediol, 2,3-nonanediol or mixtures of said liquid alkanediols are particularly preferred. Said alkanediols can be easier incorporated into semi-finished products or final products containing a solid UV filter.

In a more preferred variant, the second linear alkanediol is selected from the group consisting of 1,2-alkanediols having an uneven number of carbon atoms of 5 to 13, i.e. 1,2-pentanediol, 1,2-heptanediol, 1,2-nonanediol, 1,2-undecanediol and 1,2-tridecanediol.

In a more preferred variant, the second linear alkanediol is selected from the group consisting of 1,2-alkanediols having an uneven number of carbon atoms of 7, 9, 11 and 13, i.e. 1,2-heptanediol, 1,2-nonanediol, 1,2-undecanediol and 1,2-tridecanediol, or is selected from the group consisting of 1,2-alkanediols having an uneven number of carbon atoms of 9 and 11, i.e. 1,2-nonanediol and 1,2-undecanediol, or is selected from the group consisting of 1,2-alkanediols having an uneven number of carbon atoms of 9 and 13, i.e. 1,2-nonanediol and 1,2-tridecanediol, or is selected from the group consisting of 1,2-alkanediols having an uneven number of carbon atoms of 11 and 13, i.e. 1,2-undecanediol and 1,2-tridecanediol.

In a more preferred variant, the second linear alkanediol is selected from the group consisting of 2,3-alkanediols having an uneven number of carbon atoms of 5 to 13, i.e. 2,3-pentanediol, 2,3-heptanediol, 2,3-nonanediol, 2,3-undecanediol and 2,3-tridecanediol.

In a still more preferred variant, the second linear alkanediol is selected from the group consisting of 2,3-alkanediols having an uneven number of carbon atoms of 7, 9, 11 and 13, i.e. 2,3-heptanediol, 2,3-nonanediol, 2,3-undecanediol and 2,3-tridecanediol, or is selected from the group consisting of 2,3-alkanediols having an uneven number of carbon atoms of 9 and 11, i.e. 2,3-nonanediol and 2,3-undecanediol, or is selected from the group consisting of 2,3-alkanediols having an uneven number of carbon atoms of 9 and 13, i.e. 2,3-nonanediol and 2,3-tridecanediol, or is selected from the group consisting of 2,3-alkanediols having an uneven number of carbon atoms of 11 and 13, i.e. 2,3-undecanediol and 2,3-tridecanediol.

In a more preferred variant of the cosmetic or pharmaceutical composition or homecare product according to the second aspect of the present invention, the second linear alkanediol is selected from the group consisting of:
1,2-pentanediol, 2,3-pentanediol, 3,4-pentanediol,
1,2-hexanediol, 2,3-hexanediol, 3,4-hexanediol,
1,2-heptanediol, 2,3-heptanediol, 3,4-heptanediol,
1,2-octanediol, 2,3-octanediol, 3,4-octanediol,
1,2-nonanediol, 2,3-nonanediol, 3,4-nonanediol and mixtures thereof.

More preferred, in the cosmetic or pharmaceutical composition according to the second aspect of the present invention, the second linear alkanediol is selected from the group consisting of 1,2-pentanediol, 2,3-pentanediol, 3,4-pentanediol, and mixtures thereof. However, the second linear alkanediol can also preferably be selected from the group consisting of 1,2-hexanediol, 2,3-hexanediol, 3,4-hexanediol, and mixtures thereof or can also preferably be selected from the group consisting of 1,2-heptanediol, 2,3-heptanediol, 3,4-heptanediol, and mixtures thereof.

Even more preferred, in the cosmetic or pharmaceutical composition or homecare product according to the second aspect of the present invention, the second linear alkanediol is an alpha,beta or a beta,gamma diol as either 1,2-pentanediol, 2,3-pentanediol or a mixture thereof. However, the second linear alkanediol can also preferably be 1,2-hexanediol or 2,3-hexanediol or a mixture thereof. A mixture comprising 1,2-pentanediol and 2,3-hexanediol or a mixture comprising 2,3-pentanediol and 1,2-hexanediol is also possible.

Most preferred, the second linear alkanediol is 1,2-pentanediol or 2,3-pentanediol or 1,2-hexanediol or 2,3-hexanediol or 1,2-heptanediol or 2,3-heptanediol or 2,3-octanediol or 2,3-nonanediol.

In a particularly preferred variant, the cosmetic or pharmaceutical composition or the homecare product according to the second aspect of the present invention which comprises a mixture or a combination comprising at least one first linear alkanediol and one or more second linear alkanediol includes any one of the following mixtures/combinations:
▪ a mixture comprising 1,2-pentanediol and 2,3-pentanediol; or
▪ a mixture comprising 1,2-hexanediol and 2,3-hexanediol; or
▪ a mixture comprising 1,2-heptanediol and 2,3-heptanediol; or
▪ a mixture comprising 1,2-octanediol and 2,3-octanediol; or
▪ a mixture comprising 1,2-nonanediol and 2,3-nonanediol; or
▪ a mixture comprising 1,2-decanediol and 2,3-decanediol;
▪ a mixture comprising 1,2-undecanediol and 2,3-undecanediol;
▪ a mixture comprising 1,2-dodecanediol and 2,3-dodecanediol; or
▪ a mixture comprising 1,2-tridecanediol and 2,3-tridecanediol.

In said homo alkanediol mixture the first linear alkanediol and the second linear alkanediol have the same number of carbon atoms.

Particularly favorable is a mixture including 1,2-pentanediol in combination with 2,3-pentanediol or a mixture including 1,2-heptanediol in combination with 2,3-heptanediol and/or 3,4-heptanediol, or a mixture including 1,2-octanediol in combination with 2,3-octanediol and/or 3,4-octanediol.

The above specified alkanediol mixtures according to the second aspect of the present invention, comprising a 1,2-alkanediol and a 2,3-alkanediol either having the same number of carbon atoms, are characterized in that they significantly improve the solubility of a solid UV filter in a cosmetic or pharmaceutical formulation or homecare product compared to the respective single 1,2-alkanediol or 2,3-alkanediol substances which have only a poor solubility for solid UV filters. The solid UV filter does not crystallize out or does not precipitate out during storage and, thus, results in a stable cosmetic or pharmaceutical formulation or homecare product.

Additionally, with the admixture of the corresponding liquid 2,3-alkanediol to a solid 1,2-alkanediol the sensory properties of topical formulations comprising a solid UV filter can be significantly improved compared to the solid UV filter without the addition of the specified alkanediol mixture. The simultaneously use of a mixture comprising a 1,2-alkanediol and 2,3-alkanediol as specified above improves the spreading behaviour and/or significantly reduces the fatty/greasy skin feeling of the solid UV filters of the topical formulation. In parallel, the absorption (less remaining residue) of the solid UV filter was improved too.

Said effect or action of improving the solubility of solid UV filter is found for the homo mixture including 1,2-pentanediol and 2,3-pentanediol. The mixture of said alkanediol mixture and a solid UV filter result in a clear solution and the solid UV filter does not crystallize our or does not precipitate out during storage. **In** addition, an improvement of the sensory properties (spreading behaviour, fatty/greasy skin feeling and absorption (remaining residue on the skin) of a topical formulation comprising a solid UV filter can also be observed when used in combination with said alkanediol mixture.

A solubility improving effect can also be observed for the homo mixture including 1,2-hexanediol and 2,3-hexanediol. The mixture of said alkanediol mixture and a solid UV filter results in a clear solution and the solid UV filter does not crystallize our or does not precipitate out during storage. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

The homo mixture including 1,2-heptanediol and 2,3-heptanediol is beneficial since it has a particular solubility improving effect of solid UV filters. The resulting solution of said alkanediol mixture and a solid UV filter is clear and the solid UV filter does not crystallize out or does not precipitate out during storage. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

The alkanediol mixture comprising 1,2-octanediol and 2,3-octanediol or an alkanediol mixture comprising 1,2-nonanediol and 2,3-nonanediol are particularly advantageous, since they improve solubility of solid UV filters. A solution of said alkanediol mixture and a solid UV filter is stable and the solid UV filter does not crystallize our or does not precipitate out during storage. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

Also, the alkanediol mixture comprising 1,2-decanediol and 2,3-decanediol or the alkanediol mixture comprising 1,2-undecanediol and 2,3-undecanediol significantly improve solubility of solid UV filters. The mixtures of said alkanediol mixtures and a solid UV filter result in clear solutions which are stable during storage. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

A solubility enhancing effect is also true for an alkanediol mixture including 1,2-dodecanediol and 2,3-dodecanediol or an alkanediol mixture including 1,2-tridecanediol and 2,3-tridecanediol. The mixtures of said alkanediol mixture and a solid UV filter result in clear solutions with not precipitation of the UV filter during storage. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

The afore specified homo alkane diol mixtures including a 1,2-alkanediol and the corresponding 2,3-alkanediol display a significant solubility improvement for solid UV filters and are clearly superior to the individually corresponding 1,2-alkanediols or 2,3-alkanediols, having the same concentration, but which show a poorer solubility. All solutions of said 1,2-alkanediol and 2,3-alkanediol mixtures with a solid UV filter are clear and stable and the solid UV filter does not precipitate out even during storage.

In addition, with the simultaneous use of said alkanediol mixture, an improvement of the sensory properties (spreading behaviour, fatty/greasy skin feeling and absorption (remaining residue on the skin) for a solid UV filter containing preparation can also be observed.

Additionally, with the admixture of the corresponding liquid 2,3-alkanediol, even in small amounts, to a solid 1,2-alkanediol, the solid 1,2-alkanedioles, such as 1,2-octanediol, 1,2-nonanediol etc., can be solved, resulting in a liquid alkanediol mixture. The 2,3-alkanediol serves as solvent for the solid 1,2-alkanediols. Such liquid mixtures have the benefit that firstly the availability of the solid 1,2-alkanediol in the mixture is improved and secondly the incorporation of the solid 1,2-alkanediol in semi-finished products or final products is facilitated. This effect is particularly favorably for emulsions, in which lipophilic 1,2-alkanediols having a carbon chain of 8 or more carbon atoms, when used alone, tend to migrate into the oil phase or tend to precipitate or recrystallize.

With the solution of the solid 1,2-octanediol in the liquid 2,3-octanediol or of the solid 1,2-nonanediol in the liquid 2,3-nonanediol, the availability of the 1,2-octanediol or 1,2-nonanediol can be likewise improved in the end use.

Due to its better solubility, the availability of the solid UV filters in the cosmetic or pharmaceutical composition or homecare product is increased by the above alkanediol mixtures including a 1,2-alkanediol and a 2,3-alkanediol. Additionally, due to its better solubility, the solid UV filter can be furnished in a higher concentration in the final formulation or can be furnished in a lower concentration in the final formulation compared to a composition including a solid UV filter with low solubility and without the addition of an alkanediol.

Due to its better solubility, a uniformly solubilized product, where the solid UV filter is uniformly dissolved in the product can be obtained. Thus, the concentration of the solid UV filter is exactly the same through the whole formulation from the very first splash to the very last ml, until the bottle is completely empty. Without the use of an alkanediol as specified herein, the distribution of the solid UV filter would be quite haphazardly in the formulation and would vary from application to application.

In addition, the above alkanediol mixtures comprising a 1,2-alkanediol and the corresponding 2,3-alkanediol render possible the cold preparation of cosmetic or pharmaceutical formulations or homecare products, i.e. no heating during preparation is necessary.

Thus, with the afore-mentioned properties of the specified alkanediol mixtures including a 1,2-alkanediol and the respective 2,3-alkanediol the processability in formulations can be improved.

Hence, a good compromise can be achieved when the alkanediol mixture is combined in such a way as to maintain the solubility improving effect, while improving processability in formulations. The above specified 1,2-alkanediol and corresponding 2,3-alkanediol combinations solve this balancing problem over the 1,2-alkanediol substances or 2,3-alkanediol substances alone. This is well achieved by combinations such as comprising 1,2-pentanediol and 2,3-pentanediol but also for 1,2-hexanediol and 2,3-hexandiol. Also, combinations such as comprising 1,2-heptanediol and 2,3-heptanediol, but also 1,2-octanediol and 2,3-octanediol show this effect. The same also counts for 1,2-nonanediol in combination with 2,3-nonanediol, but also for 1,2-decanediol in combination with 2,3-decanediol.

The afore specified homo alkanediol mixtures including a 1,2-alkanediol and the corresponding 2,3-alkanediol display a remarkably synergistic solubility activity and are clearly superior to the individually corresponding 1,2-alkanediols or 2,3-alkanediols and having the same concentration.

In a particularly preferred variant, the cosmetic or pharmaceutical composition or homecare product according to the second aspect of the present invention which comprises a mixture or a combination comprising at least one first linear alkanediol and one or more second linear alkanediol thus includes any one of the following combinations:
▪ 1,2-pentanediol in combination with one of 1,2-hexanediol, 1,2-heptanediol, 1,2-octanediol, 1,2-nonanediol, 1,2-decanediol, 1,2-undecanediol, 1,2-doedecanediol, or 1,2-tridecanediol;
▪ 1,2-hexanediol in combination with one of 1,2-pentanediol, 1,2-heptanediol, 1,2-octanediol, 1,2-nonanediol, 1,2-decanediol, 1,2-undecanediol, 1,2-doedecanediol, or 1,2-tridecanediol;
▪ 1,2-heptanediol in combination with one of 1,2-pentanediol, 1,2-hexanediol, 1,2-octanediol, 1,2-nonanediol, 1,2-decanediol, 1,2-undecanediol, 1,2-doedecanediol, or 1,2-tridecanediol;
▪ 1,2-octanediol in combination with one of 1,2-pentanediol, 1,2-hexanediol, 1,2-heptanediol, 1,2-nonanediol, 1,2-decanediol, 1,2-undecanediol, 1,2-doedecanediol, or 1,2-tridecanediol;
▪ 1,2-nonanediol in combination with one of 1,2-pentanediol, 1,2-hexanediol, 1,2-heptanediol, 1,2-octanediol, 1,2-decanediol, 1,2-undecanediol, 1,2-doedecanediol, or 1,2-tridecanediol;
▪ 1,2-decanediol in combination with one of 1,2-pentanediol, 1,2-hexanediol, 1,2-heptanediol, 1,2-octanediol, 1,2-nonanediol, 1,2-undecanediol, 1,2-doedecanediol, or 1,2-tridecanediol;
▪ 1,2-undecanediol in combination with one of 1,2-pentanediol, 1,2-hexanediol, 1,2-heptanediol, 1,2-octanediol, 1,2-nonanediol, 1,2-decanediol, 1,2-dodecanediol, or 1,2-tridecanediol;
▪ 1,2-dodecanediol in combination with one of 1,2-pentanediol, 1,2-hexanediol, 1,2-heptanediol, 1,2-octanediol, 1,2-nonanediol, 1,2-decanediol, 1,2-undecanediol, or 1,2-tridecanediol;
▪ 1,2-tridecanediol in combination with one of 1,2-pentanediol, 1,2-hexanediol, 1,2-heptanediol, 1,2-octanediol, 1,2-nonanediol, 1,2-decanediol, 1,2-undecanediol, or 1,2-doedecanediol;
▪ 2,3-pentanediol in combination with one of 1,2-hexanediol, 1,2-heptanediol, 1,2-octanediol, 1,2-nonanediol, 1,2-decanediol, 1,2-undecanediol, 1,2-doedecanediol, or 1,2-tridecanediol;
▪ 2,3-hexanediol in combination with one of 1,2-pentanediol, 1,2-heptanediol, 1,2-octanediol, 1,2-nonanediol, 1,2-decanediol, 1,2-undecanediol, 1,2-doedecanediol, or 1,2-tridecanediol;
▪ 2,3-heptanediol in combination with one of 1,2-pentanediol, 1,2-hexanediol, 1,2-octanediol, 1,2-nonanediol, 1,2-decanediol, 1,2-undecanediol, 1,2-doedecanediol, or 1,2-tridecanediol;
▪ 2,3-octanediol in combination with one of 1,2-pentanediol, 1,2-hexanediol, 1,2-heptanediol, 1,2-nonanediol, 1,2-decanediol, 1,2-undecanediol, 1,2-doedecanediol, or 1,2-tridecanediol;
▪ 2,3-nonanediol in combination with one of 1,2-pentanediol, 1,2-hexanediol, 1,2-heptanediol, 1,2-octanediol, 1,2-decanediol, 1,2-undecanediol, 1,2-doedecanediol, or 1,2-tridecanediol;
▪ 2,3-decanediol in combination with one of 1,2-pentanediol, 1,2-hexanediol, 1,2-heptanediol, 1,2-octanediol, 1,2-nonanediol, 1,2-undecanediol, 1,2-doedecanediol, or 1,2-tridecanediol;
▪ 2,3-undecanediol in combination with one of 1,2-pentanediol, 1,2-hexanediol, 1,2-heptanediol, 1,2-octanediol, 1,2-nonanediol, 1,2-decanediol, 1,2-dodecanediol, or 1,2-tridecanediol;
▪ 2,3-dodecanediol in combination with one of 1,2-pentanediol, 1,2-hexanediol, 1,2-heptanediol, 1,2-octanediol, 1,2-nonanediol, 1,2-decanediol, 1,2-undecanediol, or 1,2-tridecanediol; or
▪ 2,3-tridecanediol in combination with one of 1,2-pentanediol, 1,2-hexanediol, 1,2-heptanediol, 1,2-octanediol, 1,2-nonanediol, 1,2-decanediol, 1,2-undecanediol, or 1,2-dodecanediol;
▪ 2,3-pentanediol in combination with one of 2,3-hexanediol, 2,3-heptanediol, 2,3-octanediol, 2,3-nonanediol, 2,3-decanediol, 2,3-undecanediol, 2,3-doedecanediol, or 2,3-tridecanediol;
▪ 2,3-hexanediol in combination with one of 2,3-pentanediol, 2,3-heptanediol, 2,3-octanediol, 2,3-nonanediol, 2,3-decanediol, 2,3-undecanediol, 2,3-dodecanediol, or 2,3-tridecanediol;
▪ 2,3-heptanediol in combination with one of 2,3-pentanediol, 2,3-hexanediol, 2,3-octanediol, 2,3-nonanediol, 2,3-decanediol, 2,3-undecanediol, 2,3-dodecanediol, or 2,3-tridecanediol;
▪ 2,3-octanediol in combination with one of 2,3-pentanediol, 2,3-hexanediol, 2,3-heptanediol, 2,3-nonanediol, 2,3-decanediol, 2,3-undecanediol, 2,3-dodecanediol, or 2,3-tridecanediol;
▪ 2,3-nonanediol in combination with one of 2,3-pentanediol, 2,3-hexanediol, 2,3-heptanediol, 2,3-octanediol, 2,3-decanediol, 2,3-undecanediol, 2,3-dodecanediol, or 2,3-tridecanediol;
▪ 2,3-decanediol in combination with one of 2,3-pentanediol, 2,3-hexanediol, 2,3-heptanediol, 2,3-octanediol, 2,3-nonanediol, 2,3-undecanediol, 2,3-dodecanediol, or 2,3-tridecanediol;
▪ 2,3-undecanediol in combination with one of 2,3-pentanediol, 2,3-hexanediol, 2,3-heptanediol, 2,3-octanediol, 2,3-nonanediol, 2,3-decanediol, 2,3-dodecanediol, or 2,3-tridecanediol;
▪ 2,3-dodecanediol in combination with one of 2,3-pentanediol, 2,3-hexanediol, 2,3-heptanediol, 2,3-octanediol, 2,3-nonanediol, 2,3-decanediol, 2,3-undecanediol, or 2,3-tridecanediol; or
▪ 2,3-tridecanediol in combination with one of 2,3-pentanediol, 2,3-hexanediol, 2,3-heptanediol, 2,3-octanediol, 2,3-nonanediol, 2,3-decanediol, 2,3-undecanediol, or 2,3-dodecanediol.

In a particular preferred variant, the cosmetic or pharmaceutical composition or homecare product according to the second aspect of the present invention which comprises a mixture or a combination comprising at least one first linear alkanediol and one or more second linear alkanediol thus may include any of the following mixtures/combinations:
▪ mixture comprising 1,2-heptanediol and 1,2-pentanediol; or
▪ mixture comprising 1,2-heptanediol and 1,2-hexanediol; or
▪ mixture comprising 1,2-heptanediol and 1,2-octanediol; or
▪ mixture comprising 1,2-heptanediol and 1,2-nonanediol; or
▪ mixture comprising 1,2-heptanediol and 1,2-decanediol; or
▪ mixture comprising 1,2-heptanediol and 1,2-undecanediol; or
▪ mixture comprising 1,2-heptanediol and 1,2-dodecanediol; or
▪ mixture comprising 1,2-heptanediol and 1,2-tridecanediol.

In a particular preferred variant, the cosmetic or pharmaceutical composition or homecare product according to the second aspect of the present invention which comprises a mixture or a combination comprising at least one first linear alkanediol and one or more second linear alkanediol thus may include any of the following mixtures/combinations:
▪ mixture comprising 1,2-heptanediol and 2,3-pentanediol; or
▪ mixture comprising 1,2-heptanediol and 2,3-hexanediol; or
▪ mixture comprising 1,2-heptanediol and 2,3-octanediol; or
▪ mixture comprising 1,2-heptanediol and 2,3-nonanediol; or
▪ mixture comprising 1,2-heptanediol and 2,3-decanediol; or
▪ mixture comprising 1,2-heptanediol and 2,3-undecanediol; or
▪ mixture comprising 1,2-heptanediol and 2,3-dodecanediol; or
▪ mixture comprising 1,2-heptanediol and 2,3-tridecanediol.

In a particular preferred variant, the cosmetic or pharmaceutical composition or homecare product according to the second aspect of the present invention which comprises a mixture or a combination comprising at least one first linear alkanediol and one or more second linear alkanediol thus may include any of the following mixtures/combinations:
▪ 1,2-pentanediol and 2,3-pentanediol;
▪ 1,2-pentanediol and 2,3-hexanediol;
▪ 1,2-pentanediol and 2,3-heptanediol;
▪ 1,2-pentanediol and 2,3-octanediol;
▪ 1,2-pentanediol and 2,3-nonanediol;
▪ 1,2-pentanediol and 2,3-decanediol;
▪ 1,2-pentanediol and 2,3-undecanediol;
▪ 1,2-pentanediol and 2,3-dodecanediol;
▪ 1,2-pentanediol and 2,3-tridecanediol;
▪ 1,2-hexanediol and 2,3-pentanediol;
▪ 1,2-hexanediol and 2,3-hexanediol;
▪ 1,2-hexanediol and 2,3-heptanediol;
▪ 1,2-hexanediol and 2,3-octanediol;
▪ 1,2-hexanediol and 2,3-nonanediol;
▪ 1,2-hexanediol and 2,3-decanediol;
▪ 1,2-hexanediol and 2,3-undecanediol;
▪ 1,2-hexanediol and 2,3-dodecanediol;
▪ 1,2-hexanediol and 2,3-tridecanediol;
▪ 1,2-heptanediol and 2,3-pentanediol;
▪ 1,2-heptanediol and 2,3-hexanediol;
▪ 1,2-heptanediol and 2,3-heptanediol;
▪ 1,2-heptanediol and 2,3-octanediol;
▪ 1,2-heptanediol and 2,3-nonanediol;
▪ 1,2-heptanediol and 2,3-decanediol;
▪ 1,2-heptanediol and 2,3-undecanediol;
▪ 1,2-heptanediol and 2,3-dodecanediol;
▪ 1,2-heptanediol and 2,3-tridecanediol;
▪ 1,2-octanediol and 2,3-pentanediol;
▪ 1,2-octanediol and 2,3-hexanediol;
▪ 1,2-octanediol and 2,3-heptanediol;
▪ 1,2-octanediol and 2,3-octanediol;
▪ 1,2-octanediol and 2,3-nonanediol;
▪ 1,2-octanediol and 2,3-decanediol;
▪ 1,2-octanediol and 2,3-undecanediol;
▪ 1,2-octanediol and 2,3-dodecanediol;
▪ 1,2-octanediol and 2,3-tridecanediol;
▪ 1,2-nonanediol and 2,3-pentanediol;
▪ 1,2-nonanediol and 2,3-hexanediol;
▪ 1,2-nonanediol and 2,3-heptanediol;
▪ 1,2-nonanediol and 2,3-octanediol;
▪ 1,2-nonanediol and 2,3-nonanediol;
▪ 1,2-nonanediol and 2,3-decanediol;
▪ 1,2-nonanediol and 2,3-undecanediol;
▪ 1,2-nonanediol and 2,3-dodecanediol;
▪ 1,2-nonanediol and 2,3-tridecanediol;
▪ 1,2-decanediol and 2,3-pentanediol;
▪ 1,2-decanediol and 2,3-hexanediol;
▪ 1,2-decanediol and 2,3-heptanediol;
▪ 1,2-decanediol and 2,3-octanediol;
▪ 1,2-decanediol and 2,3-nonanediol;
▪ 1,2-decanediol and 2,3-decanediol;
▪ 1,2-decanediol and 2,3-undecanediol;
▪ 1,2-decanediol and 2,3-dodecanediol;
▪ 1,2-decanediol and 2,3-tridecanediol;
▪ 1,2-undecanediol and 2,3-pentanediol;
▪ 1,2-undecanediol and 2,3-hexanediol;
▪ 1,2-undecanediol and 2,3-heptanediol;
▪ 1,2-undecanediol and 2,3-octanediol;
▪ 1,2-undecanediol and 2,3-nonanediol;
▪ 1,2-undecanediol and 2,3-decanediol;
▪ 1,2-undecanediol and 2,3-undecanediol;
▪ 1,2-undecanediol and 2,3-dodecanediol;
▪ 1,2-undecanediol and 2,3-tridecanediol; or
▪ 1,2-dodecanediol and 2,3-pentanediol;
▪ 1,2-dodecanediol and 2,3-hexanediol;
▪ 1,2-dodecanediol and 2,3-heptanediol;
▪ 1,2-dodecanediol and 2,3-octanediol;
▪ 1,2-dodecanediol and 2,3-nonanediol;
▪ 1,2-dodecanediol and 2,3-decanediol;
▪ 1,2-dodecanediol and 2,3-undecanediol;
▪ 1,2-dodecanediol and 2,3-dodecanediol;
▪ 1,2-dodecanediol and 2,3-tridecanediol.

In a particular preferred variant, the cosmetic or pharmaceutical composition or homecare product according to the second aspect of the present invention which comprises a mixture or a combination comprising at least one first linear alkanediol and one or more second linear alkanediol thus may include any of the following mixtures/combinations:
▪ 2,3-pentanediol and 2,3-hexanediol;
▪ 2,3-pentanediol and 2,3-heptanediol;
▪ 2,3-pentanediol and 2,3-octanediol;
▪ 2,3-pentanediol and 2,3-nonanediol;
▪ 2,3-pentanediol and 2,3-decanediol;
▪ 2,3-pentanediol and 2,3-undecanediol;
▪ 2,3-pentanediol and 2,3-dodecanediol;
▪ 2,3-pentanediol and 2,3-tridecanediol;
▪ 2,3-hexanediol and 2,3-pentanediol;
▪ 2,3-hexanediol and 2,3-heptanediol;
▪ 2,3-hexanediol and 2,3-octanediol;
▪ 2,3-hexanediol and 2,3-nonanediol;
▪ 2,3-hexanediol and 2,3-decanediol;
▪ 2,3-hexanediol and 2,3-undecanediol;
▪ 2,3-hexanediol and 2,3-dodecanediol;
▪ 2,3-hexanediol and 2,3-tridecanediol;
▪ 2,3-heptanediol and 2,3-pentanediol;
▪ 2,3-heptanediol and 2,3-hexanediol;
▪ 2,3-heptanediol and 2,3-octanediol;
▪ 2,3-heptanediol and 2,3-nonanediol;
▪ 2,3-heptanediol and 2,3-decanediol;
▪ 2,3-heptanediol and 2,3-undecanediol;
▪ 2,3-heptanediol and 2,3-dodecanediol;
▪ 2,3-heptanediol and 2,3-tridecanediol;
▪ 2,3-octanediol and 2,3-pentanediol;
▪ 2,3-octanediol and 2,3-hexanediol;
▪ 2,3-octanediol and 2,3-heptanediol;
▪ 2,3-octanediol and 2,3-nonanediol;
▪ 2,3-octanediol and 2,3-decanediol;
▪ 2,3-octanediol and 2,3-undecanediol;
▪ 2,3-octanediol and 2,3-dodecanediol;
▪ 2,3-octanediol and 2,3-tridecanediol;
▪ 2,3-nonaediol and 2,3-pentanediol;
▪ 2,3-nonaediol and 2,3-hexanediol;
▪ 2,3-nonaediol and 2,3-heptanediol;
▪ 2,3-nonaediol and 2,3-octanediol;
▪ 2,3-nonanediol and 2,3-decanediol;
▪ 2,3-nonanediol and 2,3-undecanediol;
▪ 2,3-nonanediol and 2,3-dodecanediol;
▪ 2,3-nonanediol and 2,3-tridecanediol;
▪ 2,3-decanediol and 2,3-pentanediol;
▪ 2,3-decanediol and 2,3-hexanediol;
▪ 2,3-decanediol and 2,3-heptanediol;
▪ 2,3-decanediol and 2,3-octanediol;
▪ 2,3-decanediol and 2,3-nonaediol;
▪ 2,3-decanediol and 2,3-undecanediol;
▪ 2,3-decanediol and 2,3-dodecanediol;
▪ 2,3-decanediol and 2,3-tridecanediol;
▪ 2,3-undecanediol and 2,3-pentanediol;
▪ 2,3-undecanediol and 2,3-hexanediol;
▪ 2,3-undecanediol and 2,3-heptanediol;
▪ 2,3-undecanediol and 2,3-octanediol;
▪ 2,3-undecanediol and 2,3-nonaediol;
▪ 2,3-undecanediol and 2,3-decanediol;
▪ 2,3-undecanediol and 2,3-dodecanediol; or
▪ 2,3-dodecanediol and 2,3-tridecanediol.

Particularly favourable is a mixture including 2,3-hexanediol and 2,3-heptanediol or a mixture including 2,3-hexanediol and 2,3-octanediol or a mixture including 2,3-heptanediol and 2,3-octanediol.

In the hetero alkanediol mixtures described before, the first linear alkanediol and the second linear alkanediol have a different number of carbon atoms.

The above specified hetero alkanediol mixtures according to the second aspect of the present invention, comprising a first linear alkanediol and a second alkanediol are characterized in that they improve the solubility of solid UV filters in a cosmetic or pharmaceutical preparation or homecare product compared to their corresponding single first linear alkanediol or second linear alkanediol substances which have only a poor solubility for solid UV filters. Additionally, the solid UV filter does less crystallize out or does less precipitate out in the cosmetic or pharmaceutical formulation or homecare product.

The use of a hetero alkanediol mixture comprising 1,2-hexanediol and 2,3-octanediol improves the solubility of a solid UV filter and results in a clear solution. The UV filter does not crystallize out or does not precipitate out neither shortly after preparation nor during storage. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

A solubility improving effect can also be observed for the hetero alkanediol mixture comprising 1,2-octanediol and 2,3-hexanediol compared to the corresponding single first linear alkanediol and second linear alkanediol substances. Said hetero alkanediol mixture and a solid UV filter results in a clear solution and the solid UV filter does not crystallize out or does not precipitate out neither shortly after preparation nor during storage. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

The hetero alkane mixture comprising 1,2-octanediol and 2,3-heptanediol is also beneficial since it has a particular solubility improving effect of solid UV filters compared to the corresponding single first linear alkanediol and second linear alkanediol substances. The resulting solution of said alkanediol mixture and a solid UV filter is clear and the solid UV filter does not crystallize out or does not precipitate out neither shortly after preparation nor during storage. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

Furthermore, a distinct improvement in solubility of a solid UV filter can be observed for the following specified alkanediol mixtures, namely a mixture of alkanediols including 1,2-pentanediol and 2,3-hexanediol; or a mixture including 1,2-pentanediol and 1,2-heptanediol; or a mixture including 1,2-pentanediol and 2,3-heptanediol; or a mixture including 1,2-pentanediol and 2,3-octanediol; or a mixture including 1,2-pentanediol and 1,2-nonanediol; or a mixture including 1,2-pentanediol and 2,3-nonanediol, compared to the corresponding single first linear alkanediol and second linear alkanediol substances.

All of the afore specified hetero alkane diol mixtures including a first linear alkanediol and a second linear alkanediol display an improved solubility towards solid UV filters compared to the corresponding single first linear alkanediol and second linear alkanediol substances. The mixtures of said alkanediol mixtures and a solid UV filter result in clear, non-turbid solutions which do not precipitate the UV filter neither shortly after preparation nor during storage. In addition, an improvement of the sensory properties (spreading behaviour, fatty/greasy skin feeling and absorption (remaining residue on the skin) can also be observed for said alkanediol mixtures.

The hetero alkanediol mixture comprising 1,2-pentanediol and 2,3-hexanediol improves the solubility of a solid UV filter compared to the individual 1,2-alkanediol or 2,3-alkanediol. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

Also, the alkanediol mixture comprising 1,2-pentanediol and 1,2-heptanediol improves the solubility of a solid UV filter in a cosmetic or pharmaceutical or homecare product compared to the corresponding single first linear alkanediol and second linear alkanediol substances. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

A solubility enhancing effect towards a solid UV filter can also be observed for the hetero alkanediol mixture comprising 1,2-pentanediol and 2,3-heptanediol compared to the corresponding single first linear alkanediol and second linear alkanediol substances. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

An improvement in the solubility of a solid UV filter can also be observed for the hetero alkanediol mixture comprising 1,2-pentanediol and 2,3-octanediol compared to the corresponding single first linear alkanediol and second linear alkanediol substances. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

A solubility improving effect towards a solid UV filter can also be observed for the alkanediol mixture comprising 1,2-pentanediol and 1,2-nonanediol compared to the corresponding single first linear alkanediol and second linear alkanediol substances. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

Also, the hetero alkanediol mixture including 1,2-pentanediol and 2,3-nonanediol is effective in improving the solubility of a solid UV filter. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

Furthermore, a solubility improving effect of solid UV filters can also be observed for mixtures comprising at least one first linear alkanediol and one or more second linear alkanediol according to the second aspect of the present invention, namely a mixture of alkanediols including 1,2-heptanediol and 1,2-octanediol or a mixture of alkanediols including 1,2-heptanediol and 2,3-octanediol or a mixture of alkanediols including 1,2-heptanediol and 1,2-nonanediol or a mixture of alkanediols including 1,2-heptanediol and 2,3-nonanediol compared to the corresponding single first linear alkanediol and second linear alkanediol substances of the alkanediol mixtures.

All of the afore specified hetero alkane diol mixtures including a first linear alkanediol and a second alkanediol display an improved solubility towards solid UV filters. The mixtures of said alkanediol mixtures and a solid UV filter result in clear, non-turbid solutions which do not precipitate the UV filter neither shortly after preparation nor during storage. In addition, an improvement of the sensory properties (spreading behaviour, fatty/greasy skin feeling and absorption (remaining residue on the skin) can also be observed for said alkanediol mixtures.

The alkanediol mixture including 1,2-heptanediol and 1,2-octanediol or including 1,2-heptanediol and 2,3-octanediol shows an enhanced effect in improving the solubility towards a solid UV filter compared to the corresponding single first linear alkanediol and second linear alkanediol substances. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

Also, the hetero alkanediol mixture including 1,2-heptanediol and 2,3-nonanediol is effective in improving the solubility of a solid UV filter. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

Especially, an alkanediol mixture including 1,2-heptanediol and 1,2-nonanediol shows an improved solubility towards a solid UV filter. In addition, an improvement of the sensory properties (spreading behaviour, fatty/greasy skin feeling and absorption (remaining residue on the skin) of a topical formulation comprising a solid UV filter can also be observed when used in combination with said alkanediol mixture.

Consequently, with the improvement of the solubility of the solid UV filter by said hetero alkanediol mixtures, it is possible to prepare clear, non-turbid solutions, which are stable during storage, i.e. the UV filter does not recrystallize out or does not precipitate out. Additionally, the simultaneously use of said hetero alkanediol mixtures considerably improves the sensory properties (spreading behaviour, fatty/greasy skin feeling and absorption (remaining residue on the skin) of a topical formulation comprising a solid UV filter.

Consequently, with the improvement of the solubility of the UV filter substance the storage stability and, thus, shelf life of the cosmetic or pharmaceutical preparation or homecare product can be considerably prolonged.

Due to its better solubility, the availability of the UV filter in the cosmetic or pharmaceutical composition is increased. Additionally, due to its better solubility, the UV filter can be furnished in a higher concentration in the final formulation or can be furnished in a lower concentration in the final formulation compared to a composition including a UV filter with low solubility and without the additional of an alkanediol.

Due to its better solubility, a uniformly solubilized product, where the UV filter is uniformly dissolved in the product can be obtained. Thus, the concentration of the UV filter is exactly the same through the whole formulation from the very first splash to the very last ml, until the bottle is completely empty. Without the use of a hetero alkanediol mixture as specified herein, the distribution of the UV filter would be quite haphazardly in the formulation and would vary from application to application.

In a preferred variant of the cosmetic or pharmaceutical composition or the homecare product according to the second aspect of the present invention, the linear alkanediol is a 2,3-alkanediol, preferably a 2,3-alkanediol selected from the group consisting of 2,3-pentanediol, 2,3-hexanediol, 2,3-heptanediol, 2,3-octanediol, 2,3-nonanediol, 2,3-decanediol, 2,3-undecanediol, 2,3-dodecanediol, 2,3-tridecanediol, and mixtures thereof.

In a more preferred variant of the cosmetic or pharmaceutical composition or the homecare product according to the second aspect of the present invention, the linear alkanediol is selected from the group consisting of 2,3-pentanediol, 2,3-hexanediol, 2,3-heptanediol, 2,3-octanediol, 2,3-nonanediol, and mixtures thereof.

Surprisingly, the addition of 2,3-heptanediol as well as other 2,3-alkanediols such as 2,3-octanediol, 2,3-nonanediol, 2,3-decanediol, 2,3-undecanediol, 2,3-dodecanediol and 2,3-tridecanediol improves the solubility of a solid UV filter in a cosmetic or pharmaceutical preparation or homecare product, compared to the corresponding 1,2-alkanediols such as 1,2-octanediol, 1,2-nonanediol etc. All solutions of said 2,3-alkanediols with a solid UV filter are clear and stable and the solid UV filter does not precipitate out even during storage, as it is demonstrated by the following examples. In addition, an improvement of the sensory properties (spreading behaviour, fatty/greasy skin feeling and absorption (remaining residue on the skin) can also be observed for said 2,3-alkanediols.

In addition, the 2,3-alkanediols as specified above have the benefit to be liquids, whereby their incorporation in semi-finished products or final products can be facilitated and their availability in said products can be increased. Additionally, in an emulsion the 2,3-alkanediols remain in the water phase.

Hence, a good compromise can be achieved when the active UV filter preparation is combined in such a way as to maintain the solubility effect, while improving processability in formulations. The above specified 2,3-alkanediols solve this balancing problem over the solid 1,2-alkanediol substances.

In a particularly advantageous variant according to the second aspect of the present invention, the linear alkanediol or the first alkanediol or the second alkanediol is a liquid alkanediol.

The term "liquid alkanediol" within the context of the present invention means an alkanediol component which is liquid at room or ambient temperature and under normal pressure, i.e. standard RTP conditions.

In a very preferred variant according to the second aspect of the present invention, the linear alkanediol or the first alkanediol or the second alkanediol is a liquid alkanediol selected from the group consisting of 1,2-pentanediol, 2,3-pentanediol, 1,2-hexanediol, 2,3-hexanediol, 1,2-heptanediol, 2,3-heptanediol, 2,3-octanediol, 2,3-nonanediol, 2,3-decanediol, 2,3-undecanediol, and mixtures thereof.

Of the aforesaid liquid alkanediols, 1,2-pentanediol, 2,3-pentanediol, 1,2-hexanediol, 2,3-hexanediol, 1,2-heptanediol, 2,3-heptanediol, 2,3-octanediol, or mixtures thereof are most preferred.

In a most particularly advantageous variant according to the second aspect of the present invention, the linear alkanediol or the first alkanediol or the second alkanediol are characterized by a maximum water solubility of less than or equal to 10 % by weight. In a preferred variant, the maximum water solubility of the linear alkanediol or the first alkanediol or the second alkanediol is less than or equal to 10 % by weight and more than or equal to 1.2 % by weight. More preferred, the maximum water solubility of the linear alkanediol or the first linear alkanediol or the second linear alkanediol is less than or equal to 5 % by weight and more than or equal to 1.4 % by weight.

Preferably, the alkanediol with said water solubility is selected from the group consisting of 1,2-heptanediol, 2,3-heptanediol, 2,3-octanediol, and mixtures thereof.

The water solubility of a substance is the saturation mass concentration of the substance in water at a given temperature. The determination of the solubility in water relates to essentially pure substances which are stable in water and not volatile. The determination of the water solubility of the alkanediols is further described in the following Example 3.

The term "alkanediol" within the context of the present invention also includes its stereoisomers. Stereoisomers are molecules that have the same molecular formula and differ only in how their atoms are arranged in three-dimensional space. In accordance with said definition, the linear alkanediol of the invention and the first alkanediol having a carbon chain of 5 to 14 carbon atoms or the second linear alkanediol having a carbon chain of 5 to 14 carbon atoms as described above in detail encompass the following stereoisomers:
1,2-alkanediol stereoisomers:
   (2S)-pentane-1,2-diol,
   (2S)-hexane-1,2-diol,
   (2S)-heptane-1,2-diol,
   (2S)-octane-1,2-diol,
   (2S)-nonane-1,2-diol,
   (2S)-decane-1,2-diol,
   (2S)-undecane-1,2-diol
   (2S)-dodecane-1,2-diol,
   (2S)-tridecane-1,2-diol,
   (2S)-tetradecane-1,2-diol,
   (2R)-pentane-1,2-diol,
   (2R)-hexane-1,2-diol,
   (2R)-heptane-1,2-diol,
   (2R)-octane-1,2-diol,
   (2R)-nonane-1,2-diol,
   (2R)-decane-1,2-diol,
   (2R)-undecane-1,2-diol,
   (2R)-dodecane-1,2-diol,
   (2R)-tridecane-1,2-diol,
   (2R)-tetradecane-1,2-diol,
2,3-alkanediol stereoisomers:
   (2S,3S)-pentane-2,3-diol,
   (2S,3S)-hexane-2,3-diol,
   (2S,3S)-heptane-2,3-diol,
   (2S,3S)-octane-2,3-diol,
   (2S,3S)-nonane-2,3-diol,
   (2S,3S)-decane-2,3-diol,
   (2S,3S)-undecane-2,3-diol
   (2S,3S)-dodecane-2,3-diol,
   (2S,3S)-tridecane-2,3-diol,
   (2S,3S)-tetradecane-2,3-diol,
   (2R,3R)-pentane-2,3-diol,
   (2R,3R)-hexane-2,3-diol,
   (2R,3R)-heptane-2,3-diol,
   (2R,3R)-octane-2,3-diol,
   (2R,3R)-nonane-2,3-diol,
   (2R,3R)-decane-2,3-diol,
   (2R,3R)-undecane-2,3-diol,
   (2R,3R)-dodecane-2,3-diol,
   (2R,3R)-tridecane-2,3-diol,
   (2R,3R)-tetradecane-2,3-diol,
   (2S,3R)-pentane-2, 3-d iol,
   (2S,3R)-hexane-2,3-diol,
   (2S,3R)-heptane-2,3-diol,
   (2S,3R)-octane-2,3-diol,
   (2S,3R)-nonane-2,3-diol,
   (2S,3R)-decane-2,3-diol,
   (2S,3R)-undecane-2,3-diol,
   (2S,3R)-dodecane-2,3-diol,
   (2S,3R)-tridecane-2,3-diol,
   (2S,3R)-tetradecane-2,3-diol,
   (2R,3S)-pentane-2,3-diol,
   (2R,3S)-hexane-2,3-diol,
   (2R,3S)-heptane-2,3-diol,
   (2R,3S)-octane-2,3-diol,
   (2R,3S)-nonane-2,3-diol,
   (2R,3S)-decane-2,3-diol,
   (2R,3S)-undecane-2,3-diol,
   (2R,3S)-dodecane-2,3-diol,
   (2R,3S)-tridecane-2,3-diol, and
   (2R,3S)-tetradecane-2,3-diol.
3,4-alkanediol stereoisomers:
   (3R,4R)-hexane-3,4-diol,
   (3R,4R)-heptane-3,4-diol,
   (3R,4R)-octane-3,4-diol,
   (3R,4R)-nonane-3,4-diol,
   (3R,4R)-decane-3,4-diol,
   (3R,4R)-undecane-3,4-diol,
   (3R,4R)-dodecane-3,4-diol,
   (3R,4R)-tridecane-3,4-diol,
   (3R,4R)-tetradecane-3,4-diol,
   (3S,4S)-hexane-3,4-diol,
   (3S,4S)-heptane-3,4-diol,
   (3S,4S)-octane-3,4-diol,
   (3S,4S)-nonane-3,4-diol,
   (3S,4S)-decane-3,4-diol,
   (3S,4S)-undecane-3,4-diol,
   (3S,4S)-dodecane-3,4-diol,
   (3S,4S)-tridecane-3,4-diol,
   (3S,4S)-tetradecane-3,4-diol,
   (3R,4S)-hexane-3,4-diol,
   (3R,4S)-heptane-3,4-diol,
   (3R,4S)-octane-3,4-diol,
   (3R,4S)-nonane-3,4-diol,
   (3R,4S)-decane-3,4-diol,
   (3R,4S)-undecane-3,4-diol,
   (3R,4S)-dodecane-3,4-diol,
   (3R,4S)-tridecane-3,4-diol,
   (3R,4S)-tetradecane-3,4-diol,
   (3S,4R)-hexane-3,4-diol,
   (3S,4R)-heptane-3,4-diol,
   (3S,4R)-octane-3,4-diol,
   (3S,4R)-nonane-3,4-diol,
   (3S,4R)-decane-3,4-diol,
   (3S,4R)-undecane-3,4-diol,
   (3S,4R)-dodecane-3,4-diol,
   (3S,4R)-tridecane-3,4-diol, and
   (3S,4R)-tetradecane-3,4-diol.

In the context of the present text, the terms "1,2-diol" and "2,3-diol" or "3,4-diol" includes both the corresponding S-configured enantiomers and also the R-enantiomers as well as arbitrary mixtures of these S- and R-configured enantiomers, i.e. mixtures of racemates of the respective diols.

The alkanediol mixture of the cosmetic or pharmaceutical composition or homecare product according to the first aspect of the present invention according to the first aspect of the present invention comprises the 1,2-heptanediol and the 2,3-heptanediol in a ratio in a range of 50 : 50 to 99.9 : 0.1, preferred in a ratio in a range of 75 : 25 to 99 : 1, more preferred in a ratio in a range of 80 : 20 to 98 : 2, still more preferred in a ratio in a range of 90 : 10 to 95 : 5.

In the cosmetic or pharmaceutical composition according to the first aspect of the present invention, 1,2-heptanediol and 2,3-heptanediol are comprised in the mixture preferably in a ratio in a range of 98 : 2 to 99.9 : 0.1.

In the homecare products according to the first aspect of the present invention, 1,2-heptanediol and 2,3-heptanediol are comprised in the alkanediol mixture preferably in a ratio in a range of 95 : 5 to 99.9 : 0.1.

1,2-heptanediol and 2,3-heptanediol are comprised in the mixture of the cosmetic or pharmaceutical composition or homecare product according to the first aspect of the present invention preferred in a ratio of ≥ 95 : ≤ 5, more preferred in a ratio of ≥ 96 : ≤ 4; still more preferred in a ratio of ≥ 97 : ≤ 3, and most preferred in a ratio of ≥ 98 : ≤ 2.

Most of all the mixture of the cosmetic or pharmaceutical composition or homecare product according to the first aspect of the present invention comprises 1,2-heptanediol and 2,3-heptanediol in a ratio of ≥ 95 : ≤ 5, including the ratios ≥ 95.5 : ≤ 4.5; ≥ 96 : ≤ 4; ≥ 96.5 : ≤ 3.5; ≥ 97 : ≤ 3; ≥ 97.5 : 2,5 and ≥ 98.0 : ≤ 2.0.

Even more preferred the alkanediol mixture of the cosmetic or pharmaceutical composition or homecare product according to the first aspect of the present invention comprises 1,2-heptanediol and 2,3-heptanediol in a ratio of ≥ 98 : ≤ 2, including the ratios of ≥ 98.1 : ≤ 1.9; ≥ 98.2 : ≤ 1.8; ≥ 98.3 : ≤ 1.7; ≥ 98.4 : ≤ 1.6; ≥ 98.5 : ≤ 1.5; ≤ 98.6 : ≤ 1.4; ≥ 98.7 : ≤ 1.3; ≥ 98.8 : ≤ 1.2; ≥ 98.9 : ≤ 1.1; ≥ 99 : ≤ 1.0; ≥ 99.1 : ≤ 0.9; ≥ 99.2 : ≤ 0.8; ≥ 99.3 : ≤ 0.7; ≥ 99.4 : ≤ 0.6; ≥ 99.5 : ≤ 0.5; ≥ 99.6 : ≤ 0.4; ≥ 99.7 : ≤ 0.3; ≥ 99.8 : ≤ 0.2 and ≥ 99.9 : ≤ 0.1.

The first linear alkanediol and the second linear alkanediol, either as defined in detail above, are present in the alkandiol mixture according to the second aspect of the present invention in a ratio in a range of 50 : 50 to 99.9 : 0.1, preferred in a ratio in a range of 75 : 25 to 99 : 1, more preferred in a ratio in a range of 80 : 20 to 98 : 2, still more preferred in a ratio in a range of 90 : 10 to 95 : 5.

In the cosmetic or pharmaceutical preparation according to the second aspect of the present invention, the first linear alkanediol and the second linear alkanediol are comprised in the mixture preferably in a ratio in a range of 98 : 2 to 99.9 : 0.1.

In the homecare products according to second aspect of the present invention, the first linear alkanediol and the second alkanediol are comprised in the alkanediol mixture preferably in a ratio in a range of 95 : 5 to 99.9 : 0.1.

The first linear alkanediol and the second alkanediol are comprised in the mixture of the cosmetic or pharmaceutical composition or homecare product according to the second aspect of the present invention preferred in a ratio of ≥ 95 : ≤ 5, more preferred in a ratio of ≥ 96 : < 4; still more preferred in a ratio of ≥ 97 : ≤ 3, and most preferred in a ratio of ≥ 98 : ≤ 2.

Most of all the alkanediol mixture of the cosmetic or pharmaceutical composition or homecare product according to the second aspect of the present invention comprises the first linear alkanediol and the second linear alkanediol in a ratio of ≤ 95 : ≤ 5, including the ratios ≥ 95.5 : ≤ 4.5; >- 96 : ≤ 4; ≥ 96.5 : ≤ 3.5; ≥ 97 : ≤ 3; ≥ 97.5 : 2,5 and ≥ 98.0 : ≤ 2.0.

Even more preferred, the alkanediol mixture of the cosmetic or pharmaceutical composition or homecare product according to the second aspect of the present invention comprises the first linear alkanediol and the second alkanediol in a ratio in a range of ≥ 98 : ≤ 2, including the ratios of ≥ 98.1 : ≤ 1.9; ≥ 98.2 : ≤ 1.8; ≥ 98.3 : ≤ 1.7; ≥ 98.4 : ≤ 1.6; ≥ 98.5 : ≤ 1.5; ≥ 98.6 : ≤ 1.4; ≥ 98.7 : ≤ 1.3; ≥ 98.8 : ≤ 1.2; ≥ 98.9 : ≤ 1.1; ≤ 99 : ≤ 1.0; ≥ 99.1 : ≤ 0.9; ≥ 99.2 : ≤ 0.8; ≥ 99.3 : ≤ 0.7; ≥ 99.4 : ≤ 0.6; ≥ 99.5 : ≤ 0.5; ≥ 99.6 : ≤ 0.4; ≥ 99.7 : ≤ 0.3; ≥ 99.8 : ≤ 0.2 and ≥ 99.9 : ≤ 0.1.

Alternatively, said ratio ranges for the first and second alkanediol are switched, such that the second alkanediol is the main component and the first alkanediol is the secondary component.

In an advantageous variant according to the second aspect of the present invention, the mixture comprises as first linear alkanediol an 1,2-alkanediol, such as 1,2-pentanediol, 1,2-hexanediol, 1,2-heptanediol, 1,2-octanediol, 1,2-nonanediol, 1,2-decanediol, 1,2-undecanediol, 1,2-dodecanediol, or 1,2-tridecanediol and as second linear alkanediol the corresponding 2,3-alkanediols, such as 2,3-pentanediol, 2,3-hexanediol, 2,3-heptanediol, 2,3-octanediol, 2,3-nonanediol, 2,3-decanediol, 2,3-undecanediol, 2,3-dodecanediol, or 2,3-tridecanediol in a ratio in a range of 50 : 50 to 99.9 : 0.1, preferably in a ratio in a range of 75 : 25 to 99 : 1, more preferred in a ratio in a range of 90 : 10 to 98 : 2, still more preferred in a ratio in a range of 90 : 10 : 95 : 5.

Likewise, for said specific mixtures, the further ratios or ranges of ratios as defined above in general for the first linear alkanediol : second alkanediol are also applicable.

In a still further variant, according to the second aspect of the present invention, the mixture comprises as first linear alkanediol an 2,3-alkanediol, such as 2,3-pentanediol, 2,3-hexanediol, 2,3-heptanediol, 2,3-octanediol, 2,3-nonanediol, 2,3-decanediol, 2,3-undecanediol, 2,3-dodecanediol, or 2,3-tridecanediol and as second linear alkanediol the corresponding 1,2-alkanediols, such as 1,2-pentanediol, 1,2-hexanediol, 1,2-heptanediol, 1,2- octanediol, 1,2-nonanediol, 1,2-decanediol, 1,2-undecanediol, 1,2-dodecanediol, or 1,2-tridecanediol in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said specific mixtures, the further ratios or ranges of ratios as defined above in general for the first linear alkanediol : second alkanediol are also applicable.

In a more advantageous variant according to the second aspect of the present invention, in the mixtures comprising a combination of a 1,2-alkanediol as first linear alkanediol and the corresponding 2,3-alkanediol as second linear alkanediol, such as
1,2-pentanediol and 2,3-pentanediol, or
1,2-hexanediol and 2,3-hexanediol, or
1,2-heptanediol and 2,3-heptanediol, or
1,2-octanediol and 2,3-octanediol, or
1,2-nonanediol and 2,3-nonanediol, or
1,2-decanediol and 2,2-decanediol, or
1,2-undecanediol and 2,3-undecenaediol, or
1,2-dodecanediol and 2,3-dodecanediol, or
1,2-tridecanediol and 2,3-tridecanediol,
the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in ratio in a range of 90 : 10 to 95 : 5.

Likewise, for said mixtures, the ratios of first linear alkanediol : second alkanediol or ranges of ratios as described above are also applicable.

By these mixing ratios the afore mentioned mixtures show an improved solubility towards solid UV filters in direct comparison with the corresponding individual substances which have merely a poor solubility towards solid UV filters, as it is described and demonstrated in the experimental part. By improving the solubility of the solid UV filter, it was possible to prepare clear and non-turbid solutions or formulations, where the UV filters do not recrystallize or precipitate during storage.

In a more advantageous variant according to the second aspect of the present invention, in the mixtures comprising as first linear alkanediol a 2,3-alkanediol and as second linear alkanediol the corresponding 1,2-alkanediols, such as mixtures including
2,3-pentanediol and 1,2-pentanediol, or
2,3-hexanediol and 1,2-hexanediol, or
2,3-heptanediol and 1,2-heptanediol, or
2,3-octanediol and 1,2-octanediol, or
2,3-nonanediol and 1,2-nonanediol, or
2,3-decanediol and 1,2-decanediol, or
2,3-undecanediol and 1,2-undecenaediol, or
2,3-dodecanediol and 1,2-dodecanediol, or
2,3-tridecanediol and 1,2-tridecanediol,
the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said specific mixtures, the further ratios or ranges of ratios as defined above in general for the first linear alkanediol : second alkanediol are also applicable.

By these mixing ratios the afore mentioned mixtures show an improved solubility towards solid UV filters in direct comparison with the corresponding individual substances which have merely a poor solubility towards solid UV filters, as it is described and demonstrated in the experimental part. By improving the solubility of the solid UV filter, it was possible to prepare clear and non-turbid solutions or formulations, where the UV filters do not recrystallize or precipitate during storage.

A preferred variant according to the second aspect the present invention also encompasses a mixture including as first linear alkanediol 1,2-hexanediol and as second linear alkanediol 2,3-octanediol or a mixture including as first linear alkanediol 1,2-octanediol and as second linear alkanediol 2,3-hexanediol or a mixture including as first linear alkanediol 1,2-octanediol and and as second linear alkanediol 2,3-heptandeiol either in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise for said specific mixtures, the further ratios or ranges of ratios as defined above in general for the first linear alkanediol : second alkanediol are also applicable.

By these mixing ratios the afore mentioned mixtures show an improved solubility towards solid UV filters in direct comparison with the corresponding individual substances which have merely a poor solubility towards solid UV filters, as it is described and demonstrated in the experimental part. By improving the solubility of the solid UV filter, it was possible to prepare clear and non-turbid solutions or formulations, where the UV filters do not recrystallize or precipitate during storage.

In a further beneficial variant according to the second aspect of the present invention, the mixtures comprise a first linear alkanediol and a second linear alkanediol, such as mixtures including
1,2-pentanediol and 2,3-hexanediol; or
1,2-pentanediol and 1,2-heptanediol; or
1,2-pentanediol and 2,3-heptanediol; or
1,2-pentanediol and 2,3-octanediol; or
1,2-pentanediol and 1,2-nonanediol; or
1,2-pentanediol and 2,3-nonanediol,
wherein the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said specific mixtures, the further ratios or ranges of ratios as defined above in general for the first linear alkanediol : second alkanediol are also applicable.

By these mixing ratios the afore mentioned mixtures show an improved solubility towards solid UV filters in direct comparison with the corresponding individual substances which have merely a poor solubility towards solid UV filters, as it is described and demonstrated in the experimental part. By improving the solubility of the solid UV filter, it was possible to prepare clear and non-turbid solutions or formulations, where the UV filters do not recrystallize or precipitate during storage.

A particularly preferred variant according to the second aspect the present invention also encompasses a mixture including as first linear alkanediol 1,2-heptanediol and as second linear alkanediol 1,2-octanediol or a mixture including as first linear alkanediol 1,2-heptanediol and as second linear alkanediol 2,3-octanediol or a mixture including as first linear alkanediol 1,2-heptanediol and as second linear alkanediol 1,2-nonanediol or a mixture including as first linear alkanediol 1,2-heptanediol and as second linear alkanediol 2,3-nonanediol wherein the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said specific mixtures, the further ratios or ranges of ratios as defined above in general for the first linear alkanediol : second alkanediol are also applicable.

By these mixing ratios the afore mentioned mixtures show an improved solubility towards solid UV filters in direct comparison with the corresponding individual substances which have merely a poor solubility towards solid UV filters, as it is described and demonstrated in the experimental part. By improving the solubility of the solid UV filter, it was possible to prepare clear and non-turbid solutions or formulations, where the UV filters do not recrystallize or precipitate during storage.

The alkanediols are obtained either by synthesis from petrochemical or other fossil fuel sources by known methods such as olefin bishydroxylation, hydrolysis from epoxide or various chemical transformations or from bioderived feedstock by fermentation or from bio-based natural and renewable feedstock such as biomass by catalytic synthesis as it is described in US 2019/0241491 A1 and US 2020/0189995 A1. The alkanediols used according to the present invention comprise either petrochemically derived and biobased natural and renewable feedstock derived alkanediols. Preferably, the alkanediols are from bio-based sources and are thus bio-alkanediols.

The compound (b) of the cosmetic or pharmaceutical composition according to the first aspect or the second aspect of the present invention relates to a solid UV filter component, which is used in many cosmetic compositions, in particular skincare, haircare or body care, or for pharmaceutical compositions.

An UV filter is a compound or a mixture of compounds that block or absorb ultraviolet (UV) light. Since excessive UV radiation can cause sunburn, photoaging, and skin cancer, care products such as sunscreen usually include a classification for the specific wavelengths they filter. UV classifications include UVA (320 to 400 nm), UVB (290 to 320 nm) and UVC (200 to 280 nm). UV-absorbing compounds are used not only in sunscreen, but also in other personal care products, such as lipstick, shampoo, hair spray, body wash, toilet soap, and insect repellent. Chemical filters protect against UV radiation by absorbing, reflecting, or scattering. Reflection and scattering are accomplished by inorganic physical UV filters, such as titanium dioxide (TiO₂) and zinc oxide (ZnO). Absorption, mainly of UVB, is done by organic UV filters, which are known as chemical UV filters.

Many different organic compounds can serve as UV filters. They fall into several structural classes:
(1) Benzophenones
   a. Benzophenone-3 (BP3)
   b. Benzophenone-4 (BP4)
(2) Salicylates
   a. Homosalate (HMS)
   b. 2-ethylhexyl salicylate (EHS)
(3) p-Aminobenzoic acid and derivatives
   a. Ethylhexyl dimethyl PABA (OD-PABA)
   b. 4-p-aminobenzoic acid (PABA)
(4) Benzimidazole derivatives
   a. Phenylbenzimidazole sulfonic acid (PMDSA)
   b. Disodium phenyl dibenzimidazole tetrasulfonate (bisdisulizole disodium)
(5) Triazines
   a. Ethylhexyltriazone (OT)
   b. Diethylhexyl butamido triazone (DBT)
   c. Bis-ethylhexyloxyphenol methoxyphenyl triazine (EMT)
(6) Benzotriazoles
   a. Drometrizole trisiloxane (DRT)
   b. Methylene bis-benzotriazolyl tetramethylbutylphenol (MBP, biscotrizole)
(7) Dibenzoylmethane derivatives
   a. 4-*tert*-Butyl-4'-methoxydibenzoylmethane (BM-DBM, avobenzone)
(8) Cinnamates
   a. Ethylhexyl methoxycinnamate (OMC)
   b. Isoamyl *p*-methoxycinnamate (IMC, amiloxate)
(9) Camphor derivatives
   a. Terephtalydene dicamphor sulfonic acid (PDSA)
   b. 3-benzylidene camphor (3BC)
   c. Benzylidene camphor sulfonic acid (BCSA)
   d. 4-methylbenzylidene camphor (4-MBC)
   e. Polyacrylamidomethyl benzylidene camphor (PBC)
   f. Camphor benzalkonium methosulfate (CBM)

The solid UV filter component according to the first and second aspect of the present invention belong to different structural classes as specified above.

The term "solid UV filter component" within the context of the present invention means a UV filter component which is solid at room or ambient temperature and under normal pressure, i.e. standard RTP conditions.

The solid UV filter component in the cosmetic or pharmaceutical composition according to the first and second aspect of the present invention is selected from the group consisting of:
- 1-(4-tert-Butylphenyl)-3-(4-methoxyphenyl)-propane-1,3-dione (INCI: Butyl Methoxydibenzoylmethane; Avobenzone),
- Sodium salt of 2,2'-bis(1,4-phenylene)-1H-benzimidazole-4,6-disulfonic acid (INCI: Disodium Phenyl Dibenzimidazole Tetrasulfonate),
- 2-Hydroxy-4-methoxybenzophenone (INCI: Benzophenone-3),
- 2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol),
- Isopentyl-4-methoxycinnamate (INCI: Isoamyl p-Methoxycinnamate; Amiloxate),
- 2-Phenylbenzimidazole-5-sulfonic acid and its potassium, sodium and triethanolamine salts (INCI: Phenylbenzimidazole Sulfonic Acid),
- 3-(4-Methylbenzylidene)-d1 camphor (INCI: 4-Methylbenzylidene Camphor),
- 1,3,5-Triazine-2,4,6-tris[1,1'-biphenyl]-4-yl (INCI: Tris-biphenyl Triazine, incl. Nanomaterial),
- 2-Hydroxy-4-methoxybenzophenone- 5-sulfonic acid and its sodium salt (INCI: Benzophenone-4/Benzophenone-5),
- Zinc Oxide (INCI: Zinc Oxide),
- Benzoic acid, 2-[-4-(diethylamino)-2-hydroxybenzoyl]-hexylester (INCI: Diethylamino Hydroxybenzoyl Hexyl Benzoate),
- 2,4,6-Trianilino-(p-carbo-2'-ethylhexyl-1'-oxy)-1,3,5-triazine (INCI: Ethylhexyl Triazone),
- alpha-(2-Oxoborn-3-ylidene)-toluene-4-sulphonic acid and its salts (INCI: Benzylidene Camphor Sulfonic Acid),
- Polymer of N-{(2 and 4)-[(2-oxoborn-3-ylidene)-methyl]-benzyl}-acrylamide (INCI: Polyacrylamido Methyl Benzylidene Camphor),
- 3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid),
- Ethoxylated Ethyl-4-aminobenzoate (INCI: PEG-25 PABA),
- Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone),
- Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane),
- 3-Benzylidene camphor (INCI: 3-Benzylidene Camphor),
- Phenylene-bis-diphenyltriazine (Triasorb B),
- Neo Heliopan^{®} Flat (INCI: Homosalate, Octocrylene, Bis-Ethylhexyloxyphenol Methoxyphenyl Triazine, Butyl Methoxydibenzoylmethane, Ethylhexyl Salicylate),
- Titanium dioxide (INCI: Titanium Dioxide), and
mixtures of two or more of the aforesaid solid UV filters.

The aforesaid UV filters can be used either as a single component or in mixture with two or more further different UV filter(s) as specified above.

In a preferred variant, the solid UV filter of the cosmetic or pharmaceutical composition of the present invention is a solid UV filter selected from the group consisting of:
- 1-(4-tert-Butylphenyl)-3-(4-methoxyphenyl)-propane-1,3-dione (INCI: Butyl Methoxydibenzoylmethane; Avobenzon),
- 2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol),
- 3-(4-Methylbenzylidene)-d1 camphor (INCI: 4-Methylbenzylidene Camphor),
- 1,3,5-Triazine-2,4,6-tris[1,1'-biphenyl]-4-yl (INCI: Tris-biphenyl Triazine, incl. Nanomaterial),
- Benzoic acid, 2-[-4-(diethylamino)-2-hydroxybenzoyl]-hexylester (INCI: Diethylamino Hydroxybenzoyl Hexyl Benzoate),
- 2,4,6-Trianilino-(p-carbo-2'-ethylhexyl-1'-oxy)-1,3,5-triazine (INCI: Ethylhexyl Triazone),
- Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone),
- Phenylene-bis-diphenyltriazine (Triasorb B),
- Neo Heliopan^{®} Flat, and
mixtures of two or more of the aforesaid solid UV filters.

In a particularly preferred variant, the solid UV filter of the cosmetic or pharmaceutical composition of the present invention is a solid UV filter selected from the group consisting of:
- 2,2'-(6-(4-Methoxyphenyl)-1,3,5-triazine-2,4-diyl)-bis(5-((2-ethylhexyl)-oxy)-phenol) (INCI: Bis-Ethylhexyloxyphenol Methoxyphenyl Triazine; Bemotrizinol),
- 1,3,5-Triazine-2,4,6-tris[1,1'-biphenyl]-4-yl (INCI: Tris-biphenyl Triazine, incl. Nanomaterial),
- 2,4,6-Trianilino-(p-carbo-2'-ethylhexyl-1'-oxy)-1,3,5-triazine (INCI: Ethylhexyl Triazone),
- Benzoic acid, 4,4-((6-((4-(((1,1-dimethylethyl)-amino)-carbonyl)-phenyl)-amino)-1,3,5-triazine-2,4- diyl)-diimino)-bis,bis-(2-ethylhexyl)-ester (INCI: Diethylhexyl Butamido Triazone),
- Phenylene-bis-diphenyltriazine (Triasorb B),
- Neo Heliopan^{®} Flat, and
mixtures of two or more of the aforesaid solid UV filters.

In a particular advantageously variant, the cosmetic or pharmaceutical composition according to the first aspect or second aspect of the present invention comprises one of the following combinations of components (a) and (b):
▪ 1,2-pentanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or 1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or 2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or 1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or 1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-decanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-undecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl.
   Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-dodecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI:
   Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 2,3-pentanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or Phenylene-bis-diphenyltriazine (Triasorb B), and/or
   Neo Heliopan^{®} Flat, and/or
   Titanium dioxide (INCI: Titanium Dioxide); or
▪ 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 2,3-decanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or 3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 2,3-undecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 2,3-dodecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 2,3-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-heptanediol in combination with 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-heptanediol in combination with one or more of 1,2-pentanediol, 1,2-hexanediol, 1,2-octanediol, 1,2-nonanediol, 1,2-decanediol, 1,2-undecanediol, 1,2-dodecanediol or 1,2-tridecanediol, plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-heptanediol in combination with one or more of 2,3-pentanediol, 2,3-hexanediol, 2,3-heptanediol, 2,3-octanediol, 2,3-nonanediol, 2,3-decanediol, 2,3-undecanediol, 2,3-dodecanediol, or 2,3-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-pentanediol in combination with 2,3-pentanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-hexanediol in combination with 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI:
   Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-octanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI:
   Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-nonanediol in combination with 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-decanediol in combination with 2,3-decanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-undecanediol in combination with 2,3-undecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-dodecanediol in combination with 2,3-dodecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI:
   Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-tridecanediol in combination with 2,3-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-hexanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-octanediol in combination with 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-ylmethanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-octanediol in combination with 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl.
   Nanomaterial), and/or
   2-Hydroxy-4-methoxybenzophenone- 5-sulfonic acid and its sodium salt (INCI:
   Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-pentanediol in combination with 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-pentanediol in combination with 1,2-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-pentanediol in combination with 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-pentanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-pentanediol in combination with 1,2-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-pentanediol in combination with 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-heptanediol in combination with 1,2-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-heptanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-heptanediol in combination with 1,2-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide); or
▪ 1,2-heptanediol in combination with 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   Sodium salt of 2,2'-bis(1,4-phenylene)-1H-benzimidazole-4,6-disulfonic acid (INCI: Disodium Phenyl Dibenzimidazole Tetrasulfonate), and/or
   2-Hydroxy-4-methoxybenzophenone (INCI: Benzophenone-3), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   Isopentyl-4-methoxycinnamate (INCI: Isoamyl p-Methoxycinnamate; Amiloxate), and/or
   2-Phenylbenzimidazole-5-sulfonic acid and its potassium, sodium and triethanolamine salts (INCI: Phenylbenzimidazole Sulfonic Acid), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   2-Hydroxy-4-methoxybenzophenone- 5-sulfonic acid and its sodium salt (INCI: Benzophenone-4/Benzophenone-5), and/or
   Zinc Oxide (INCI: Zinc Oxide), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   alpha-(2-Oxoborn-3-ylidene)-toluene-4-sulphonic acid and its salts (INCI: Benzylidene Camphor Sulfonic Acid), and/or
   Polymer of N-{(2 and 4)-[(2-oxoborn-3-ylidene)-methyl]-benzyl}-acrylamide (INCI: Polyacrylamido Methyl Benzylidene Camphor), and/or
   3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid), and/or
   Ethoxylated Ethyl-4-aminobenzoate (INCI: PEG-25 PABA), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane), and/or
   3-Benzylidene camphor (INCI: 3-Benzylidene Camphor), and/or
   Phenylene-bis-diphenyltriazine (Triasorb B), and/or
   Neo Heliopan^{®} Flat, and/or
   Titanium dioxide (INCI: Titanium Dioxide).

More preferred combinations of component (a) and (b) in the cosmetic or pharmaceutical composition according to the first aspect or second aspect of the present invention are:
▪ 1,2-pentanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-decanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-undecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-dodecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-pentanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-decanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-undecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-dodecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone); and/or
   Neo Heliopan^{®} Flat, and/or
   Phenylene-bis-diphenyltriazine (Triasorb B); or
▪ 1,2-heptanediol in combination with 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-heptanediol in combination with one or more of 1,2-pentanediol, 1,2-hexanediol, 1,2-octanediol, 1,2-nonanediol, 1,2-decanediol, 1,2-undecanediol, 1,2-dodecanediol or 1,2-tridecanediol, plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-heptanediol in combination with one or more of 2,3-pentanediol, 2,3-hexanediol, 2,3-heptanediol, 2,3-octanediol, 2,3-nonanediol, 2,3-decanediol, 2,3-undecanediol, 2,3-dodecanediol, or 2,3-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-hexanediol in combination with 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-octanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-nonanediol in combination with 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-decanediol in combination with 2,3-decanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-undecanediol in combination with 2,3-undecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-dodecanediol in combination with 2,3-dodecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-tridecanediol in combination with 2,3-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-hexanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-octanediol in combination with 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-octanediol in combination with 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-pentanediol in combination with 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-pentanediol in combination with 1,2-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-pentanediol in combination with 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-pentanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-pentanediol in combination with 1,2-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-pentanediol in combination with 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-heptanediol in combination with 1,2-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-heptanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-heptanediol in combination with 1,2-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-heptanediol in combination with 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B).

Most preferred combinations of component (a) and (b) in the cosmetic or pharmaceutical composition according to the first aspect or second aspect of the present invention are:
▪ 1,2-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-heptanediol in combination with 2,3-heptanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-pentanediol in combination with 2,3-pentanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-hexanediol in combination with 2,3-hexanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-octanediol in combination with 2,3-octanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-nonanediol in combination with 2,3-nonanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-decanediol in combination with 2,3-decanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-undecanediol in combination with 2,3-undecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-dodecanediol in combination with 2,3-dodecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone), and/or
   Neo Heliopan^{®} Flat, and/or
   Phenylene-bis-diphenyltriazine (Triasorb B); or
▪ 1,2-tridecanediol in combination with 2,3-tridecanediol plus one or more of the following solid UV filters:
   1-(4-tert-Butylphenyl)-3-(4-methoxyphenyl)-propane-1,3-dione (INCI: Butyl Methoxydibenzoylmethane; Avobenzon), and/or
   2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol), and/or
   3-(4-Methylbenzylidene)-d1 camphor (INCI: 4-Methylbenzylidene Camphor), and/or
   1,3,5-Triazine-2,4,6-tris[1,1'-biphenyl]-4-yl (INCI: Tris-biphenyl Triazine, incl. Nanomaterial), and/or
   Benzoic acid, 2-[-4-(diethylamino)-2-hydroxybenzoyl]-hexylester (INCI: Diethylamino Hydroxybenzoyl Hexyl Benzoate), and/or
   2,4,6-Trianilino-(p-carbo-2'-ethylhexyl-1'-oxy)-1,3,5-triazine (INCI: Ethylhexyl Triazone), and/or
   Benzoic acid, 4,4-((δ-((4-(((1,1-dimethylethyl)-amino)-carbonyl)-phenyl)-amino)-1,3,5-triazine-2,4-diyl)-diimino)-bis, bis-(2-ethylhexyl)-ester (INCI: Diethylhexyl Butamido Triazone), and/or
   Phenylene-bis-diphenyltriazine (Triasorb B).

Of the above combinations, the combinations with 1,2-heptanediol, 2,3-heptanediol or a 2,3-alkanediol, preferably 2,3-heptanediole and 2,3-octanediole, or an alkanediol mixture comprising a first linear alkanediol and a second linear alkanediol result in distinguished solubility properties, i.e. the solid UV filter(s) can be good solubilized in the ready-to-use composition, in particular in an emulsion, and does/do not crystallize out or precipitate out of the composition neither shortly after preparation nor during storage. This effect is demonstrated by the following examples.

The above-defined specific combinations can be combined with one or more further components (c) as described later on.

In order to optimize the SPF, i.e. to obtain a high SPF in a range of 5 to 50, preferably 60, and to cover a broad UVA and UVB range, the solid UV filter(s) in the cosmetic or pharmaceutical composition as defined herein, is/are advantageously combined with at least one further primary sun protection factor and/or with at least one further secondary sun protection factor. The combination of effective sun protection factors of different categories such as UV-A filter, UV-B, broadband filter, inorganic pigments provides reliable protection against the different UV rays.

Primary sun protection factors: In addition to the above disclosed solid UV filters, the cosmetic or pharmaceutical composition according to the present invention preferably includes one or more further primary sun protection factors. Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The cosmetic or pharmaceutical composition according to the invention advantageously contains at least one UV-A filter and/or at least one UV-B filter and/or a broadband filter and/or at least one inorganic pigment. Compositions according to the invention preferably contain at least one UV-B filter or a broadband filter, more particularly preferably at least one UV-A filter and at least one UV-B filter. Preferred cosmetic compositions, preferably topical compositions according to the present invention comprise one, two, three or more sun protection factors selected from the group consisting of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

In addition, a combination with active ingredients which penetrate into the skin and protect the skin cells from inside against sunlight-induced damage and reduce the level of cutaneous matrix metalloproteases is advantageously. Preferred respective ingredients are so called arylhydrocarbon receptor antagonists. Preferred is 2-benzylidene-5,6-dimethoxy-3,3-dimethylindan-1-one.

The UV filters cited below which can be used within the context of the present invention are preferred but naturally are not limiting.

UV filters which are preferably used are selected from the group consisting of:
- Neo Heliopan^{®} Flat INCI: Homosalate, Octocrylene, Bis-Ethylhexyloxyphenol Methoxyphenyl Triazine, Butyl Methoxydibenzoylmethane, Ethylhexyl Salicylate,
- p-aminobenzoic acid,
- p-aminobenzoic acid ethyl ester (25 mol) ethoxylated (INCI name: PEG-25 PABA),
- p-dimethylaminobenzoic acid-2-ethylhexyl ester,
- p-aminobenzoic acid ethyl ester (2 mol) N-propoxylated,
- p-aminobenzoic acid glycerol ester,
- salicylic acid homomenthyl ester (homosalates) (Neo Heliopan^{®}HMS),
- salicylic acid-2-ethylhexyl ester (Neo Heliopan^{®}OS),
- triethanolamine salicylate,
- 4-isopropyl benzyl salicylate,
- anthranilic acid menthyl ester (Neo Heliopan^{®}MA),
- diisopropyl cinnamic acid ethyl ester,
- p-methoxycinnamic acid-2-ethylhexyl ester (Neo Heliopan^{®}AV),
- diisopropyl cinnamic acid methyl ester,
- p-methoxycinnamic acid isoamyl ester (Neo Heliopan^{®}E 1000),
- p-methoxycinnamic acid diethanolamine salt,
- p-methoxycinnamic acid isopropyl ester,
- 2-phenylbenzimidazole sulfonic acid and salts (Neo Heliopan^{®}Hydro),
- 3-(4'-trimethylammonium) benzylidene bornan-2-one methyl sulfate,
- beta-imidazole-4(5)-acrylic acid (urocanic acid),
- 3-(4'-sulfo)benzylidene bornan-2-one and salts,
- 3-(4'-methyl benzylidene)-D,L-camphor (Neo Heliopan^{®}MBC),
- 3-benzylidene-D,L-camphor,
- N-[(2 and 4)-[2-(oxoborn-3-ylidene) methyl]benzyl] acrylamide polymer,
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb^{®}HEB),
- benzylidene malonate polysiloxane (Parsol^{®}SLX),
- glyceryl ethylhexanoate dimethoxycinnamate,
- dipropylene glycol salicylate,
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul^{®}T150),
and mixutres thereof

In a preferred variant the cosmetic or pharmaceutical composition according to the present invention comprises a combination with one or more broadband filters which are selected from the group consisting of:
- 2-ethylhexyl-2-cyano-3,3-diphenyl acrylate,
- ethyl-2-cyano-3,3'-diphenyl acrylate,
- dihydroxy-4-methoxybenzophenone,
- 2,4-dihydroxybenzophenone,
- tetrahydroxybenzophenone,
- 2,2'-dihydroxy-4,4'-dimethoxybenzophenone,
- 2-hydroxy-4-n-octoxybenzophenone,
- 2-hydroxy-4-methoxy-4'-methylbenzophenone,
- sodium hydroxymethoxybenzophenone sulfonate,
- disodium-2,2'-dihydroxy-4,4'-dimethoxy-5,5'-disulfobenzophenone,
- phenol, 2-(2H-benzotriazol-2-yl)-4-methyl-6-(2-methyl-3(1,3,3,3-tetramethyl-1-(trimethylsilyl)oxy)disiloxyanyl) propyl),
- 2,2'-methylene-bis-(6-(2H-benzotriazol-2-yl)-4-1,1,3,3-tetramethylbutyl)phenol) (Methylene-Bis-Benzotriazolyl Tetramethylbutylphenol),
- 2,4-bis-[4-(2-ethylhexyloxy)-2-hydroxyphenyl]-1,3,5-triazine,
- 2,4-bis-[{(4-(2-ethylhexyloxy)-2-hydroxy}phenyl]-6-(4-methoxyphenyl)-1,3,5-triazine (INCI: Aniso Triazin),
- 2,4-bis-[{(4-(3-sulfonato)-2-hydroxypropyloxy)-2-hydroxy}phenyl]-6-(4-methoxyphenyl)-1,3,5-triazine sodium salt,
- 2,4-bis-[{(3-(2-propyloxy)-2-hydroxypropyloxy)-2-hydroxy}phenyl]-6-(4-methoxyphenyl)-1,3,5-triazine,
- 2,4-bis-[{4-(2-ethylhexyloxy)-2-hydroxy}phenyl]-6-[4-(2-methoxyethylcarbonyl) phenylamino]-1,3,5-triazine,
- 2,4-bis-[{4-(3-(2-propyloxy)-2-hydroxypropyloxy)-2-hydroxy}phenyl]-6-[4-(2-ethylcarboxyl) phenylamino]-1,3,5-triazine,
- 2,4-bis-[{4-(2-ethylhexyloxy)-2-hydroxy}phenyl]-6-(1-methylpyrrol-2-yl)-1,3,5-triazine,
- 2,4-bis-[{4-tris-(trimethylsiloxysilylpropyloxy)-2-hydroxy}phenyl]-6-(4-methoxyphenyl)-1,3,5-triazine,
- 2,4-bis-[{4-(2"-methylpropenyloxy)-2-hydroxy}phenyl]-6-(4-methoxyphenyl)-1,3,5-triazine,
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine,
- dioctylbutylamidotriazone (INCI: Dioctylbutamidotriazone),
- 2,4-bis-[5-1 (dimethylpropyl)benzoxazol-2-yl-(4-phenyl)-imino]-6-(2-ethylhexyl)-imino-1,3,5-triazine,
- 4,4',4"-(1,3,5-triazine-2,4,6-triyltriimino)tris-benzoic acid-tris(2-ethyl-hexylester) (also: 2,4,6-tris[anilino(p-carbo-2'-ethyl-1'-hexyloxy)]-1,3,5-triazine (INCI: Octyl Triazone),
- 2,4,6-tribiphenyl-4-yl-1,3,5-triazine,
- 2-Ethylhexyl 4-methoxycinnamate (Ethylhexyl Methoxycinnamate),
- Benzoic acid, 2-hydroxy-, 3,3,5-trimethylcyclohexyl ester (Homosalate),
- 2-Ethylhexyl salicylate (Ethylhexyl Salicylate),
- 2-Ethylhexyl 4-(dimethylamino)benzoate (Ethylhexyl Dimethyl PABA),
- N,N,N-Trimethyl-4-(2-oxoborn-3-ylidenemethyl) anilinium methyl sulfate (Camphor Benzalkonium Methosulfate),
- Dimethicodiethylbenzalmalonate (Polysilicone-15),
and mixtures thereof.

In a preferred variant the cosmetic or pharmaceutical composition according to the present invention comprises a combination with one or more UV-A filters which are selected from the group consisting of:
- 4-isopropyl dibenzoyl methane,
- terephthalylidene dibornane sulfonic acid and salts,
- 4-t-butyl-4'-methoxydibenzoyl methane (avobenzone),
- phenylene bis-benzimidazyl tetrasulfonic acid disodium salt,
- 2,2'-(1,4-phenylene)-bis-(1H-benzimidazole-4,6-disulfonic acid), monosodium salt,
- 2-(4-diethylamino-2-hydroxybenzoyl) benzoic acid hexyl ester,
- indanylidene compounds,
and mixtures thereof.

In a more preferred variant, the cosmetic or pharmaceutical composition according to the present invention comprises a combination with one or more UV filters which are selected from the group consisting of:
- p-aminobenzoic acid,
- 3-(4'-trimethylammonium) benzylidene bornan-2-one methyl sulfate.
- salicylic acid homomenthyl ester (Neo Heliopan^{®}HMS),
- 2-phenylbenzimidazole sulfonic acid (Neo Heliopan^{®}Hydro),
- terephthalylidene dibornane sulfonic acid and salts (Mexoryl^{®}SX),
- 4-tert-butyl-4'-methoxydibenzoyl methane (Neo Heliopan^{®}357),
- 3-(4'-sulfo)benzylidene bornan-2-one and salts,
- 2-ethylhexyl-2-cyano-3,3-diphenyl acrylate (Neo Heliopan^{®}303),
- N-[(2 and 4)-[2-(oxoborn-3-ylidene) methyl]benzyl] acrylamide polymer,
- p-methoxycinnamic acid-2-ethylhexyl ester (Neo Heliopan^{®}AV),
- p-aminobenzoic acid ethyl ester (25 mol) ethoxylated (INCI name: PEG-25 PABA),
- p-methoxycinnamic acid isoamyl ester (Neo Heliopan^{®}E1000),
- 2,4,6-trianilino-(p-carbo-2'-ethylhexyl-1'-oxy)-1,3,5-triazine (Uvinul^{®}T150),
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL),
- 4,4'-[(6-[4-(1,1-dimethyl)aminocarbonyl) phenylamino]-1,3,5-triazine-2,4-diyl)di-imino]-bis-(benzoic acid-2-ethylhexyl ester) (Uvasorb HEB),
- 3-(4'-methyl benzylidene)-D,L-camphor (Neo Heliopan^{®}MBC),
- 3-benzylidene camphor,
- salicylic acid-2-ethylhexyl ester (Neo Heliopan^{®}OS),
- 4-dimethylaminobenzoic acid-2-ethylhexyl ester (Padimate O),
- hydroxy-4-methoxybenzophenone-5-sulfonic acid and Na salt,
- 2,2'-methylene bis-(6-(2H-benzotriazol-2-yl)-4-1,1,3,3-tetramethylbutyl) phenol) (Tinosorb^{®}M),
- phenylene bis-benzimidazyl tetrasulfonic acid disodium salt (Neo Heliopan^{®}AP),
- 2,4-bis-[{(4-(2-ethylhexyloxy)-2-hydroxy}phenyl]-6-(4-methoxyphenyl)-1,3,5-triazine (Tinosorb^{®}S),
- benzylidene malonate polysiloxane (Parsol^{®}SLX),
- menthyl anthranilate (Neo Heliopan^{®}MA),
- 2-(4-diethylamino-2-hydroxybenzoyl) benzoic acid hexyl ester (Uvinul^{®} A Plus),
- indanylidene compounds,
and mixtures thereof.

In a further preferred variant the cosmetic or pharmaceutical composition according to the invention contains a total amount of sunscreen agents, i.e. in particular UV filters and/or inorganic pigments (UV filtering pigments) so that the composition according to the invention has a sun protection factor SPF of 5 to 50, preferably 5 to 60. Such compositions according to the invention are particularly suitable for protecting the skin and hair.

Secondary sun protection factors: Besides the groups of primary sun protection factors mentioned above, secondary sun protection factors of the antioxidant type may also be advantageously used in the cosmetic or pharmaceutical composition according to the present invention. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts. The total quantity of inorganic pigments, in particular hydrophobic inorganic micro-pigments in the finished cosmetic preparation according to the present invention is advantageously from 0.1 to 30 % by weight, preferably 0.5 to 10.0% by weight, in each case based on the total weight of the preparation.

Also preferred are particulate UV filters or inorganic pigments, which can optionally be hydrophobed, can be used, such as the oxides of iron (Fe₂O₃), zirconium (ZrO₂), silicon (SiO₂), manganese (e.g. MnO), aluminium (Al₂O₃), cerium (e.g. Ce₂O₃), and/or mixtures thereof.

The cosmetic or pharmaceutical, in particular dermatological, composition as defined herein, are preferably based on a carrier, which comprises at least one oil phase or at least one oil component. However, preparations solely based on water are also possible. Accordingly, oils, oil-in-water and water-in-oil emulsions, etc. are suitable.

The oil phase or the oil component in the cosmetic or pharmaceutical composition according to the present invention which may be suitable are for example plant oils, hydrocarbons, fatty alcohols, fatty acid esters, or mixtures of two or more of the aforesaid oil components.

The oil phase or oil component in the cosmetic or pharmaceutical composition is preferably a plant oil and even more preferably a liquid plant oil. It can also advantageously be a mixture of two or more plant oils components, especially liquid plant oil mixtures.

Plant oils or vegetable oils are oils extracted from seeds, or less often, from other parts of fruits. Like animal fats, plant oils are mixtures of triglycerides. Soybean oil, rapeseed oil and cocoa butter are examples of plant oils from seeds. Olive oil, palm oil and rice bran oil are examples of oils from other parts of fruits. In common usage, plant oil or vegetable oil may refer exclusively to vegetable fats which are liquid at room temperature or at 35 to 37 °C skin temperature. Vegetable oils are usually edible.

The term "plant oils" also includes unsaturated plant oils. Unsaturated oils or vegetable oils can be transformed through partial or complete "hydrogenation" into oils of higher melting point. The hydrogenation process involves "sparging" the oil at high temperature and pressure with hydrogen in the presence of a catalyst, typically a powdered nickel compound. As each carbon-carbon double-bond is chemically reduced to a single bond, two hydrogen atoms each form single bonds with the two carbon atoms. The elimination of double bonds by adding hydrogen atoms is called saturation; as the degree of saturation increases, the oil progresses toward being fully hydrogenated. An oil may be hydrogenated to increase resistance to rancidity (oxidation) or to change its physical characteristics. As the degree of saturation increases, the oil's viscosity and melting point increase.

In a preferred variant, the plant oil is selected from the group consisting of Persea Gratissima (Avocado Oil), Abies Alba Seed Oil, Acacia Victoriae Seed Oil, Actinidia Chinensis (Kiwi) Seed Oil, Amaranthus Hypochondriacus Seed Oil, Arachis Hypogaea (Peanut) Oil, Astrocaryum Murumuru Seed Butter, Astrocaryum Tucuma Seed Butter, Astrocaryum Tucuma Seed Oil, Astrocaryum Vulgare Fruit Oil, Astrocaryum Vulgare Kernel Oil, Avena Sativa (Oat) Kernel Oil, Brassica Alba Seed Oil, Brassica Campestris (Rapeseed) Seed Oil, Butyrospermum Parkii (Shea) Butter, Butyrospermum Parkii (Shea) Oil, Calendula Officinalis Seed Oil, Calophyllum Inophyllum Seed Oil, Calophyllum Tacamahaca Seed Oil, Camellia Oleifera Seed Oil, Camellia Reticulata Seed Oil, Camellia Sinensis Seed Oil, Cannabis Sativa Seed Oil, Cannabis Sativa Seed/Stem Oil, Canola Oil, Carthamus Tinctorius (Safflower) Seed Oil, Chlorella Vulgaris Oil, Citrullus Lanatus (Watermelon) Seed Oil, Citrus Aurantifolia (Lime) Seed Oil, Citrus Aurantium Dulcis (Orange) Seed Oil, Citrus Grandis (Grapefruit) Seed Oil, Cocos Nucifera (Coconut) Oil, Cocos Nucifera (Coconut) Seed Butter, Coffea Arabica (Coffee) Seed Oil, Chlorella Oil (biotech), Corylus Americana (Hazelnut) Seed Oil, Corylus Avellana (Hazelnut) Seed Oil, Cucumis Melo (Melon) Seed Oil, Cucumis Sativus (Cucumber) Seed Oil, Cucurbita Pepo (Pumpkin) Seed Oil, Elaeis Guineensis (Palm) Oil, Elaeis (Palm) Fruit Oil, Glycine Soja (Soybean) Oil, Gossypium Herbaceum (Cotton) Seed Oil, Gossypium Hirsutum (Cotton) Seed Oil, Helianthus Annuus (Sunflower) Seed Oil, Macadamia Integrifolia Seed Oil, Macadamia Ternifolia Seed Oil, Mangifera Indica (Mango) Seed Butter, Mangifera Indica (Mango) Seed Oil, Melissa Officinalis Seed Oil, Microalgae Oil, Moringa Oleifera Seed Oil, Moringa Peregrina Seed Oil, Oenothera Biennis (Evening Primrose) Oil, Olea Europaea (Olive) Fruit Oil, Olus Oil, Orbignya Oleifera Seed Oil, Orbignya Speciosa Kernel Oil, Oryza Sativa (Rice) Bran/Germ Oil, Oryza Sativa (Rice) Bran Oil, Oryza Sativa (Rice) Germ Oil, Oryza Sativa (Rice) Lipids, Oryza Sativa (Rice) Seed Oil, Papaver Somniferum Seed Oil, Passiflora Edulis Seed Oil, Persea Gratissima (Avocado) Butter, Persea Gratissima (Avocado) Oil, Prunus Amygdalus Dulcis (Sweet Almond) Oil, Prunus Armeniaca (Apricot) Kernel Oil, Prunus Persica (Peach) Kernel Oil, Punica Granatum Seed Oil, Pyrus Malus (Apple) Seed Oil, Ricinoleic/Caproic/Caprylic/Capric Triglyceride(s), Ricinus Communis (Castor) Seed Oil, Rosa Canina Fruit Oil, Rosa Moschata Seed Oil, Rubus Idaeus (Raspberry) Seed Oil, Sesamum Indicum (Sesame) Seed Butter, Soybean Glycerides, Theobroma Cacao (Cocoa) Seed Butter, Theobroma Grandiflorum Seed Butter, Triticum Vulgare (Wheat) Bran Lipids, Triticum Vulgare (Wheat) Germ Oil, Vitis Vinifera (Grape) Seed Oil, Zea Mays (Corn) Germ Oil, and Zea Mays (Corn) Oil.

In a preferred variant, the cosmetic or pharmaceutical composition according to the first aspect or the second aspect of the present invention comprises a plant oil, selected from the group consisting of Persea Gratissima (Avocado Oil), Argania spinosa Kernel Oil (Argan oil), Butyrospermum Parkii (Shea) Butter, Cocos Nucifera (Coconut) Oil, Glycine Soja (Soybean) Oil, Helianthus Annuus (Sunflower) Seed Oil, Olea Europaea (Olive) Fruit Oil, Prunus Amygdalus Dulcis (Sweet Almond) Oil, Ricinus Communis (Castor) Seed Oil, Simmondsia Chinensis (Jojoba) Seed oil, Theobroma Cacao (Cocoa) Seed Butter, Vitis Vinifera (Grape) Seed Oil, Caprylic Capric Triglycerides, and mixtures of two or more of the aforesaid plant oils. The plant oil can be used either as a single component or in mixture with one or more further different plant oil(s) as specified above.

The above specified plant oils are the most common natural lipid components used as basic substances for the manufacture of cosmetics or pharmaceutical formulations. However, the drawback of said plant oils is that they exhibit unpleasant sensory properties upon topical application such as low spreading, an oily, greasy feeling, imparting unwanted gloss to the skin and rendering the composition sticky. Additionally, they are often not easily absorbed or leave an off feeling of the absorbed product on the skin.

Hydrocarbons (mineral oils) are in general organic compounds consisting entirely of hydrogen and carbon. As defined by IUPAC nomenclature or organic chemistry, the classifications for hydrocarbons are:
1. Saturated hydrocarbons are the simplest of the hydrocarbon species. They are composed entirely of single bonds and are saturated with hydrogen. The formula for acyclic saturated hydrocarbons (i.e., alkanes) is C*ₙ*H_{2*n*+2}.The most general form of saturated hydrocarbons is C*ₙ*H_{2*n*+2(1-*r*)}, where r is the number of rings. Those with exactly one ring are the cycloalkanes. Saturated hydrocarbons are the basis of petroleum fuels and are found as either linear or branched species.
2. Unsaturated hydrocarbons have one or more double or triple bonds between carbon atoms. Those with double bond are called alkenes. Those with one double bond have the formula C*ₙ*H*₂ₙ* (assuming non-cyclic structures). Those containing triple bonds are called alkynes. Those with one triple bond have the formula C*ₙ*H_{2*n-*2}*.*
3. Aromatic hydrocarbons, also known as arenes, are hydrocarbons that have at least one aromatic ring.

Hydrocarbons can be inter alia liquids (e.g. hexane and benzene), waxes or low melting solids (e.g. paraffin wax and naphthalene). The term 'aliphatic' refers to non-aromatic hydrocarbons. Saturated aliphatic hydrocarbons are sometimes referred to as "paraffins". Mineral oils and waxes are mixtures of predominantly saturated hydrocarbons consisting of straight-chain, branched and ring structures with carbon chain lengths greater than C14. Mineral oils and waxes are chemical substances prepared from naturally occurring crude petroleum oil. They mainly consist of mineral oil saturated hydrocarbons (MOSH) and mineral oil aromatic hydrocarbons (MOAH). Hydrocarbons have been used for many decades in skin and lip care cosmetic products due to their excellent skin tolerance as well as their high protecting and cleansing performance and broad viscosity options. In contrast to vegetable oils, mineral oils are non-allergenic since they are highly stable and not susceptible to oxidation or rancidity.

In a preferred variant, the hydrocarbon is selected from the group consisting of undecane, tridecane, mineral oil, petrolatum, squalane, isohexadecane, C7 - C8 isoparaffin, C8 - C9 isoparaffin, C9 - C11 isoparaffin, C9 - C12 isoparaffin, C9 - C13 isoparaffin, C9 - C14 isoparaffin, C9 - C16 isoparaffin, C10 - C11 isoparaffin, C10 - C12 isoparaffin, C10 - C13 isoparaffin, C11 - C12 isoparaffin, C11 - C13 isoparaffin, C11 - C14 isoparaffin, C12 - C14 isoparaffin, C12 - C15 isoparaffin, C12 - C20 isoparaffin, C13 - C14 isoparaffin, C13 - C16 isoparaffin, C14 - C16 isoparaffin, C15 - C19 isoparaffin, and C18 - C70 isoparaffin. The hydrocarbon can be used either as a single component or in mixture with one or more further different hydrocarbon(s) as specified above.

In a more preferred variant, the hydrocarbon is selected from the group consisting of hydrocarbons, petrolatum, squalane, isohexadecane, C13 - C14 isoparaffin, and mixtures of two or more of the aforesaid hydrocarbons.

A fatty alcohol (or long-chain alcohol) is usually a high-molecular-weight, straight-chain primary alcohol, but can also range from as few as 4 to 6 carbons to as many as 22 to 26, derived from natural fats and oils. The precise chain length varies with the source. Some commercially important fatty alcohols are lauryl, stearyl and oleyl alcohols. They are colourless oily liquids (for smaller carbon numbers) or waxy solids, although impure samples may appear yellow. Fatty alcohols usually have an even number of carbon atoms and a single alcohol group (-OH) attached to the terminal carbon. Some are unsaturated and some are branched. Most fatty alcohols in nature are found as waxes which are esters with fatty acids and fatty alcohols. The traditional sources of fatty alcohols have largely been various vegetable oils and these remain a large-scale feedstock. The alcohols are obtained from the triglycerides (fatty acid triesters), which form the bulk of the oil. The process involves the transesterification of the triglycerides to give methyl esters which are then hydrogenated to give the fatty alcohols. Fatty alcohols are also prepared from petrochemical sources. In the Ziegler process, ethylene is oligomerized using triethylaluminium followed by air oxidation. Alternatively, ethylene can be oligomerized to give mixtures of alkenes, which are subjected to hydroformylation, this process affording odd-numbered aldehyde, which is subsequently hydrogenated. Fatty alcohols are mainly used in the production of detergents and surfactants. They are components also of cosmetic solvents. They find use as co-emulsifiers, emollients and thickeners in cosmetics.

In a preferred variant, the fatty alcohol is selected from the group consisting of phenyl propanol, dimethyl phenylbutanol, hexyldecanol, octyldodecanol, octyldecanol, tridecylalcohol, isostearyl alcohol, phenylisohexanol, phenylpropanol, trimethylbenzenepropanol, isoamylalcohol, isostearyl alcohol, and isotridecyl alcohol. In a more preferred variant, the fatty alcohol is selected from the group consisting of hexyldecanol, octyldodecanol, phenylpropanol, isoamylalcohol, and mixtures of two or more of the aforesaid fatty alcohols. The fatty alcohol can be used either as a single component or in mixture with one or more further different fatty alcohol(s) as specified above.

A fatty acid ester is a type of ester that results from the combination of a fatty acid with an alcohol. When the alcohol component is glycerol, the fatty acid esters produced can be monoglycerides, diglycerides or triglycerides. Fatty acid esters have a conditioning effect of softening the skin to create a smoothing sensation. They are also added to cosmetics to dissolve high-polarity active ingredients and UV absorbers. Esters of straight-chain fatty acids and lower alcohols are effective for dissolving slightly soluble ingredients for oils with a light touch during application. Isostearic acids and other liquid oils with branched fatty acids and unsaturated fatty acids are commonly used as emollients. Higher fatty acid esters and esters of higher alcohols with relatively high melting points are added to skin creams to adjust the application touch.

In a preferred variant, the fatty acid ester is selected from the group consisting of C12 - C15 Alkyl Benzoate, Capric/Lauric/Myristic/Oleic Triglyceride, Caprylic/Capric Triglyceride, Caprylic/Capric/Lauric Triglyceride, Caprylic/Capric/Linoleic Triglyceride, Caprylic/Capric/Myristic/Stearic Triglyceride, Caprylic/Capric/Palmitic/Stearic Triglyceride, Caprylic/Capric/Stearic Triglyceride, Caprylic/Capric/Succinic Triglyceride, Caprylyl Caprylate, Cetearyl Ethylhexanoate, Cetearyl Isononanoate, Cetearyl Nonanoate, Coco-Caprylate, Decyl Cocoate, Decyl Oleate, Dicaprylyl Carbonate, Diethyl Succinate, Diethylhexyl 2,6-Naphthalate, Diethylhexyl Carbonate, Dibutyl Adipate, Diisopropyl Adipate, Dipropylheptyl Carbonate, Ethyl Laurate, Ethylhexyl Isononanoate, Ethylhexyl Palmitate, Ethylhexyl Stearate, Glyceryl Caprylate Caprate, Glyceryl Caprylate, Glyceryl Laurate, Glyceryl Triacetyl Hydroxystearate, Glyceryl Triacetyl Ricinoleate, Hexyl Laurate, Isoamyl acetate, Isoamyl Cocoate, Isopropyl Palmitate, Isosorbide Dicaprylate, Isopropyl Myristate, Myristyl Myristate, Oleic/Linoleic Triglyceride, Oleic/Palmitic Triglyceride, Oleic/Palmitic/Lauric/Myristic/Linoleic Triglyceride, Propanediol Caprylate, Ricinoleic/Caproic/Caprylic/ Capric Triglyceride, Oleostearine, Oleyl Erucate, Palmitic/Stearic Triglyceride, Propanediol Dicaprylate Caprate, Propylheptyl Caprylate, Stearyl Heptanoate/Stearyl Caprylate, Triheptanoin, Trihydroxystearin, Triisononanoin, Triisopalmitin, Triisostearin, Trilaurin, Trilinolein, Trilinolenin, Trimyristin, Triolein, Tripalmitin, Tripalmitolein, Tripelargonin, Triricinolein, and Tristearin.

In a more preferred variant, the fatty acid ester is selected from the group consisting of C12 - C15 Alkyl Benzoate, Caprylic/Capric Triglyceride, Caprylyl Caprylate, Cetearyl Ethylhexanoate, Cetearyl Isononanoate, Cetearyl Nonanoate, Coco-Caprylate, Decyl Cocoate, Decyl Oleate, Dicaprylyl Carbonate, Diethylhexyl 2,6-Naphthalate, Dibutyl Adipate, Diisopropyl Adipate, Ethyl Laurate, Ethylhexyl Isononanoate, Ethylhexyl Palmitate, Ethylhexyl Stearate, Glyceryl Caprylate, Glyceryl Laurate, Hexyl Laurate, Isoamyl Cocoate, Isopropyl Palmitate, Isopropyl Myristate, Myristyl Myristate, Propanediol Caprylate, Oleyl Erucate, Palmitic/Stearic Triglyceride, Propanediol Dicaprylate Caprate, Trihydroxystearin, Triisostearin, Triisononanoin, Tristearin, and mixtures of two or more of the aforesaid fatty acid esters. The fatty acid ester can be used either as a single component or in mixture with one or more further different fatty acid ester(s) as specified above.

In addition to the oil phase or oil component as defined above, the cosmetic or pharmaceutical composition according to the present invention preferably includes one or more oil bodies. Suitable oil bodies, which form constituents of the O/W emulsions, are, for example, Guerbet alcohols based on fatty alcohols having 6 to 18, preferably 8 to 10, carbon atoms, esters of linear C5 - C22 fatty acids with linear or branched C5 - C22 fatty alcohols or esters of branched C1 - C13 carboxylic acids with linear or branched C6 - C22 fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C6 - C22 fatty acids with branched alcohols, in particular 2-ethylhexanol, esters of C18 - C38 alkylhydroxy carboxylic acids with linear or branched C6 - C22 fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C6 - C10 fatty acids, liquid mono-/di-/triglyceride mixtures based on C6 - C18 fatty acids, esters of C6 - C22 fatty alcohols and/or Guerbet alcohols with aromatic carboxylic acids, in particular benzoic acid, esters of C2 - C12 dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched CC - C22 fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

Within the context of the present invention, it is also possible and in some cases advantageous to combine the cosmetic or pharmaceutical, in particular dermatological, composition according to the first aspect or second aspect of the present invention with other customary active substances, adjuvants or additives.

The cosmetically or pharmaceutically active agents and/or adjuvants and/or additives can in some instances provide one or more than one benefit or operate via more than one mode of action.

Thus, optionally, other conventional cosmetically and/or pharmaceutically active substances, adjuvants or additives, as further described below, may be added as component (c), i.e. in order to obtain a ready-for-use composition or formulation.

The cosmetic or pharmaceutical composition according to the present invention can advantageously be combined with other cosmetically or pharmaceutically active agents and/or adjuvants and/or additives or auxiliaries, such as are customarily used in such compositions, such as for example abrasives, anti-acne agents, agents against ageing of the skin, anti-cellulitis agents, anti-dandruff agents, anti-inflammatory agents, anti-microbial agents, irritation-preventing agents, irritation-inhibiting agents, antioxidants, astringents, odor absorbers, perspiration-inhibiting agents, antiseptic agents, anti-statics, binders, buffers, carrier materials, chelating agents, cell stimulants, cleansing agents, depilatory agents, surface-active substances, deodorizing agents, antiperspirants, softeners, emulsifiers, enzymes, enzyme inhibitors, essential oils, fibers, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, dyes, friction-reducing agents, lubricants, moisturizing creams, ointments, opacifying agents, plasticizing agents, covering agents, polish, preservatives, gloss agents, green and synthetic polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, surfactants, UV-absorbing agents, UV filters, primary sun protection factors, secondary sun protection factors, detergents, fabric conditioning agents, suspending agents, skin-tanning agents, actives modulating skin or hair pigmentation, matrix-metalloproteinase inhibitors, skin moisturizing agents, glycosaminoglycan stimulators, TRPV1 antagonists, desquamating agents, anti-cellulite agents or fat enhancing agents, hair growth activators or inhibitors, thickeners, rheology additives, vitamins, oils, waxes, pearlizing waxes, fats, phospholipids, saturated fatty acids, mono- or polyunsaturated fatty acids, α-hydroxy acids, polyhydroxyfatty acids, liquefiers, dyestuffs, colour-protecting agents, pigments, anti-corrosives, fragrances or perfume oils, aromas, flavouring substances, odoriferous substances, polyols, electrolytes, organic solvents, and mixtures of two or more of the aforesaid substances, as further described below.

Of the above cosmetically or pharmaceutically active agents and/or adjuvants and/or additives or auxiliaries agents against ageing of the skin, anti-microbial agents, antioxidants, chelating agents, emulsifiers, preservatives, green and synthetic polymers, skin-cooling agents, rheology additives, oils, fragrances or perfume oils, and polyols are particularly preferred in the preparation of cosmetic and pharmaceutical composition.

Since dermatological conditions or diseases are often associated with dry skin, scratched skin, skin lesions or even inflammation, the cosmetic or pharmaceutical composition according to the present invention advantageously contains preferably anti-inflammatories, antibacterial or antimycotic substances, substances having a reddening-alleviating or itch-alleviating action, lenitive substances, moisturisers and/or cooling agents, osmolytes, keratolytic substances, nurturing substances, anti-inflammatory, antibacterial or antimycotic substances, substances having a reddening-alleviating or itch-alleviating action, lenitive substances, anti-dandruff substances, or other active compounds such as solvents, fragrances antioxidants, preservatives, (metal) chelating agents, penetration enhancers, or mixtures of two or more of afore specified agents, as further described below.

**Anti-ageing actives:** The cosmetic or pharmaceutical composition according to the present invention preferably contains one or more anti-ageing actives. In the context of the invention, anti-ageing or biogenic agents are, for example antioxidants, matrix-metalloproteinase inhibitors (MMPI), skin moisturizing agents, glycosaminglycan stimulators, anti-inflammatory agents, TRPV1 antagonists and plant extracts.

**Antioxidants:** A preferred cosmetic or pharmaceutical composition according to the present invention comprises one or more antioxidants. Suitable antioxidants encompass amino acids (preferably glycine, histidine, tyrosine, tryptophan) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone. If vitamin E and/or derivatives thereof are used as the antioxidant(s), it is advantageous to choose their concentrations from the range from about 0.001 to about 10 % by weight, based on the total weight of the composition. If vitamin A or vitamin A derivatives or carotenes or derivatives thereof are used as the antioxidant(s), it is advantageous to choose their concentrations from the range from about 0.001 to about 10 % by weight based on the total weight of the composition.

**Matrix-Metalloproteinase inhibitors (MMPI):** A preferred cosmetic or pharmaceutical composition according to the present invention comprises one or more matrix-metalloproteinase inhibitors, especially those inhibiting matrix-metalloproteinases enzymatically cleaving collagen, selected from the group consisting of ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf, as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

**Skin-moisturizing agents:** A preferred cosmetic or pharmaceutical composition according to the present invention comprises one or more skin-moisturizing agents. Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of glycerol, 1,2-propylene glycol, 1,2-butylene glycol, 1,3-butylene glycol, 1,2-pentanediol, 1,2-hexanediol, 1,2-octanediol and 1,2-decanediol.

**Glycosaminoglycan stimulators:** A preferred cosmetic or pharmaceutical composition according to the present invention comprises one or more substances stimulating the synthesis of glycosaminoglycans which are selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

**TRPV1 antagonists:** A preferred cosmetic or pharmaceutical composition according to the present invention comprises one or more TRPV1 antagonists. Suitable compounds which reduce the hypersensitivity of skin nerves based on their action as TRPV1 antagonists, encompass e.g. trans-4-tert-butyl cyclohexanol, or indirect modulators of TRPV1 by an activation of the µ-receptor, e.g. acetyl tetrapeptide-15, are preferred.

**Plant extracts:** A preferred cosmetic or pharmaceutical composition according to the present invention comprises one or more plant extracts. Plant extracts, special highly active plant extract fractions and also highly pure active substances isolated from plant extracts can also be used in the cosmetic or pharmaceutical composition according to the present invention. Extracts, fractions and active substances from camomile, *aloe vera, Commiphora* species, Rubia species, willows, willow-herb, ginger, marigold, arnica, Glycyrrhiza species, Echinacea species, Rubus species and pure substances such as *inter alia* bisabolol, apigenin, apigenin-7-glucoside, gingerols such as [6]-gingerol, paradols such as [6]-paradol, boswellic acid, phytosterols, glycyrrhizine, glabridin or licochalcone A are particularly preferred.

**Anti-inflammatory agents:** The cosmetic or pharmaceutical composition according to the present invention preferably also contains anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. More particularly:
(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone,
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone,
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea, or single active compounds thereof,
(iv) histamine receptor antagonists, serine protease inhibitors (e.g. of Soy extracts), TRPV1 antagonists (e.g. 4-t-Butylcyclohexanol), NK1 antagonists (e.g. Aprepitant, Hydroxyphenyl Propamidobenzoic Acid), cannabinoid receptor agonists (e.g. Palmitoyl Ethanolamine) and TRPV3 antagonists.

Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives are preferred anti-itch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenan-thramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3->1,4-β-glucan from oats.

When bisabolol is used in the context of the present invention it can be of natural or synthetic origin, and is preferably "alpha-bisabolol". Preferably, the bisabolol used is synthetically prepared or natural (-)-alpha-bisabolol and/or synthetic mixed-isomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide (CO₂), hexane, methylene chloride, chloroform or other solvents or solvent mixtures of comparable polarity. The extracts are characterized by the presence of active skin irritation-reducing amounts of constituents such as e.g. gingerols, shogaols, gingerdiols, dehydrogingerdiones and/or paradols.

**Physiological cooling agents:** The cosmetic or pharmaceutical composition according to the present invention can be particularly advantageously combined with one or more physiological cooling agent(s). The use of cooling agents can alleviate itching. Preferred individual cooling agents for use within the framework of the present invention are listed below. The person skilled in the art can add many other cooling agents to this list; the cooling agents listed can also be used in combination with one another: which are preferably selected here from the following list: menthol and menthol derivatives (for example L-menthol, D-menthol, racemic menthol, isomenthol, neoisomenthol, neomenthol) menthylethers (for example (I-menthoxy)-1,2-propanediol, (I-menthoxy)-2-methyl-1,2-propanediol, I-menthyl-methylether), menthone glyceryl acetal, menthone glyceryl ketal or mixtures of both, menthylesters (for example menthylformiate, menthylacetate, menthylisobutyrate, menthyhydroxyisobutyrat, menthyllactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{α}-(menthanecarbonyl)glycinethylester [WS5], menthanecarboxylic acid-N-(4-cyanophenyl)amide or menthanecarboxylic acid-N-(4-cyanomethylphenyl)amide, menthanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (I-(-)-isopulegol, I-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(1-isopropyl)cyclohexane-carboxamide [WS12], oxamates and [(1R,2S,5R)-2-isopropyl-5-methyl-cyclohexyl] 2-(ethylamino)-2-oxo-acetate (X Cool). Cooling agents which are preferred due to their particular synergistic effect are I-menthol, d-menthol, racemic menthol, menthone glycerol acetal (trade name: Frescolat^{®} MGA), menthyl lactate (preferably I-menthyl lactate, in particular I-menthyl I-lactate (trade name: Frescolat^{®} ML)), substituted menthyl-3-carboxamides (such as menthyl-3-carboxylic acid N-ethyl amide), 2-isopropyl-N-2,3-trimethyl butanamide, substituted cyclohexane carboxamides, 3-menthoxypropane-1,2-diol, 2-hydroxyethyl menthyl carbonate, 2-hydroxypropyl menthyl carbonate and isopulegol. Particularly preferred cooling agents are I-menthol, racemic menthol, menthone glycerol acetal (trade name: Frescolat^{®} MGA), menthyl lactate (preferably I-menthyl lactate, in particular I-menthyl I-lactate (trade name: Frescolat^{®} ML)), 3-menthoxypropane-1,2-diol, 2-hydroxyethyl menthyl carbonate and 2-hydroxypropyl menthyl carbonate. Very particularly preferred cooling agents are I-menthol, menthone glycerol acetal (trade name: Frescolat^{®} MGA) and menthyl lactate (preferably I-menthyl lactate, in particular I-menthyl I-lactate (trade name: Frescolat^{®} ML).

**Moisturising and/or moisture-retaining substances:** Itching occurs with particular intensity when the skin is dry. The use of skin-moisturising and/or moisture-retaining substances can significantly alleviate itching. The cosmetic or pharmaceutical composition according to the present invention can therefore advantageously also contain one or more of the following moisturising and/or moisture-retaining substances: sodium lactate, urea, urea derivatives, alcohols, glycerol, diols such as propylene glycol, hexylene glycol, collagen, elastin or hyaluronic acid, diacyl adipates, petrolatum, urocanic acid, lecithin, panthenol, phytantriol, lycopene, (pseudo-)ceramides, glycosphingolipids, cholesterol, phytosterols, chitosan, chondroitin sulphate, lanolin, lanolin esters, amino acids, alpha-hydroxy acids (such as citric acid, lactic acid, malic acid) and their derivatives, mono-, di- and oligosaccharides such as glucose, galactose, fructose, mannose, fructose and lactose, polysugars such as R-glucans, in particular 1,3-1,4-β-glucan from oats, alpha-hydroxy fatty acids, triterpene acids such as betulinic acid or ursolic acid, and algae extracts.

**Lenitive substances:** The cosmetic or pharmaceutical composition according to the present invention can also contain advantageously one or more lenitive substances, wherein any lenitive substances can be used which are suitable or customary in cosmetic or pharmaceutical applications such as alpha-bisabolol, azulene, guaiazulene, 18-beta-glycyrrhetinic acid, allantoin, Aloe vera juice or gel, extracts of Hamamelis virginiana (witch hazel), Echinacea species, Centella asiatica, chamomile, Arnica monatana, Glycyrrhiza species, algae, seaweed and Calendula officinalis, and vegetable oils such as sweet almond oil, baobab oil, olive oil and panthenol, Laureth-9, Trideceth-9 and 4-t-butylcyclohexanol.

**Antibacterial or antimycotic active substances:** Antibacterial or antimycotic active substances can also particularly advantageously be used in the cosmetic or pharmaceutical composition according to the present invention, wherein any antibacterial or antimycotic active substances can be used which are suitable or customary in cosmetic or pharmaceutical, in particular dermatological applications. In addition to the large group of conventional antibiotics, other products which are advantageous here include those relevant to cosmetics such as in particular triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate or combinations of said substances, which are used *inter alia* against underarm odour, foot odour or dandruff.

**Anti-microbial agents:** The cosmetic or pharmaceutical composition according to the present invention preferably contains one or more anti-microbioal agents. Suitable anti-microbial agents are, in principle, all substances effective against Gram-positive bacteria, such as, for example, 4- hydroxybenzoic acid and its salts and esters, N-(4-chlorophenyl)-N'-(3,4-dichlorophenyl)urea, 2,4,4'-trichloro-2'-hydroxy-diphenyl ether (triclosan), 4-chloro-3,5-dimethylphenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

**Desquamating agents:** The cosmetic or pharmaceutical composition according to the present invention preferably contains one or more desquamating agents. The expression "desquamating agent" is understood to mean any compound capable of acting:
- either directly on desquamation by promoting exfoliation, such as β-hydroxy acids, in particular salicylic acid and its derivatives (including 5-n-octanoylsalicylic acid); α-hydroxy acids, such as glycolic, citric, lactic, tartaric, malic or mandelic acids; urea; gentisic acid; oligofucoses; cinnamic acid; extract of Sophora japonica; resveratrol and some derivatives of jasmonic acid;
- or on the enzymes involved in the desquamation or the degradation of the corneodesmosomes, glycosidases, stratum corneum chymotryptic enzyme (SCCE) or other proteases (trypsin, chymotrypsin-like). There may be mentioned agents chelating inorganic salts: EDTA; N-acyl-N,N',N'-ethylenediaminetriacetic acid; aminosulphonic compounds and in particular (N-2-hydroxyethylpiperazine-N-2-ethane)sulphonic acid (HEPES); derivatives of 2-oxothiazolidine-4-carboxylic acid (procysteine); derivatives of alpha-amino acids of the glycine type (as described in EP-0 852 949, and sodium methylglycine diacetate marketed by BASF under the trade name TRILON M); honey; sugar derivatives such as O-octanoyl-6-D-maltose and N-acetylglucosamine; chestnut extracts such as those marketed by the company SILAB under the name Recoverine^{®}, prickly pear extracts such as those marketed under the name Exfolactive^{®} by the company SILAB, or Phytosphingosine SLC^{®} (phytosphingosine grafted with a salicylic acid) marketed by the company Degussa.

Desquamating agents suitable for the invention may be chosen in particular from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids such as retinol and its esters, retinal, retinoic acid and its derivatives, chestnut or prickly pear extracts, in particular marketed by SILAB; reducing compounds such as cysteine or cysteine precursors. Desquamating agents which can be used are also nicotinic acid and its esters and nicotinamide, also called vitamin B3 or vitamin PP, and ascorbic acid and its precursors.

**Anti-dandruff substances:** In addition, the cosmetic or pharmaceutical composition according to the present invention can also advantageously be used in combination with one or more anti-dandruff substances, including triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate, Propanediol Monocaprylate or combinations of said substances, which are used inter alia against dandruff.

Further suitable anti-dandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®} (4-acetyl-1-{4-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

**(Metal) chelating agents:** A combination with one or more (metal) chelating agents can also be advantageous used in the cosmetic or pharmaceutical composition according to the present invention, wherein any metal chelating agents can be used which are suitable or customary in cosmetic or pharmaceutical applications. Preferred (metal) chelating agents include α-hydroxy fatty acids, phytic acid, lactoferrin, α-hydroxy acids, such as *inter alia* gluconic acid, glyceric acid, glycolic acid, isocitric acid, citric acid, lactic acid, malic acid, mandelic acid, tartaric acid, as well as humic acids, bile acids, bile extracts, bilirubin, biliverdin or EDTA, EGTA and their derivatives. The use of one or more chelating agent(s) improves the stability of the composition according to the present invention.

**Emulsifiers:** In addition, the cosmetic or pharmaceutical composition according to the present invention can also advantageously contain one or more emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

Partial glycerides: Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

Sorbitan esters: Suitable sorbitan esters are sorbitan monoisostearate, sorbitan sesqui-isostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesqui-hydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Polyglycerol esters: Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Anionic emulsifiers: Typical anionic emulsifiers are aliphatic C12 to C 22 fatty acids, such as palmitic acid, stearic acid or behenic acid for example, and C12 to C22 dicarboxylic acids, such as azelaic acid or sebacic acid for example.

Amphoteric emulsifiers: Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of Coc*amidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-coco-alkylaminopropionate, cocoacylaminoethyl aminopropionate and C_{12/18} acyl sarcosine.

**Preservatives:** For preservative purposes, the cosmetic or pharmaceutical composition according to the present invention preferably includes one or more preservatives which are suitable or customary in cosmetic or pharmaceutical composition. Suitable and advantageously preservatives are, for example, phenoxyethanol, formaldehyde solution, parabens, pentanediol or sorbic acid.

**Green and synthetic polymers:** The cosmetic or pharmaceutical composition according to the present invention preferably includes one or more green or synthetic polymers. Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohydroxypropyl diethylenetriamine (Cartare-tine^{®}, Sandoz), copolymers of acrylic acid with dimethyl diallyl ammonium chloride (Merquat^{®}550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®}A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300. Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones. In a preferred variant, the cosmetic or pharmaceutical composition according to the present invention preferably includes an uncrosslinked or polyol-crosslinked polyacrylic acid as additionally polymer component.

**Thickening agents and/or rheology additives:** The cosmetic or pharmaceutical composition according to the present invention preferably includes one or more thickening agents and/or rheology additives. Suitable thickeners are polymeric thickeners, such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

**Perfume oils and/or fragrances:** The cosmetic or pharmaceutical composition according to the present invention preferably includes one or more perfume oils and/or fragrances. Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, beta-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

**Anti-cellulite agent:** The cosmetic or pharmaceutical composition according to the present invention preferably includes one or more anti-cellulite agents. Anti-cellulite agents and lipolytic agents are preferably selected from the group consisting of beta-adrenergic receptor agonists such as synephrine and its derivatives, and cyclohexyl carbamates. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

**Fat enhancing agents:** The cosmetic or pharmaceutical composition according to the present invention preferably includes one or more fat enhancing agents and/or adipogenic agents as well as agents enhancing or boosting the activity of fat enhancing agents. A fat enhancing agent is for example hydroxymethoxyphenyl propylmethylmethoxybenzofuran (trade name: Sym3D^{®}).

**Superfatting agents and/or consistency factors:** The cosmetic or pharmaceutical composition according to the present invention preferably includes one or more superfatting agents and/or consistency factors. Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers. The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

**Pearlizing waxes:** The cosmetic or pharmaceutical composition according to the present invention preferably includes one or more pearlizing waxes. Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups, and mixtures thereof.

**Silicones:** In order to impart a silky, spreadable, and luxurious texture and to make skin look and feel smoother, and additionally to improve processability (antifoaming) the cosmetic or pharmaceutical composition according to the present invention preferably includes one or more silicones or silicone deratives. Suitable silicones can be chosen from the group consisting of Acefylline Methylsilanol Mannuronate, Acetylmethionyl Methylsilanol Elastinate Acrylates/Behenyl, Acrylate/Dimethicone Methacrylate Copolymer, Acrylates/Behenyl Methacrylate/Dimethicone Methacrylate Copolymer, Acrylates/Bis-Hydroxypropyl Dimethicone Crosspolymer, Acrylates/Dimethicone Copolymer, Acrylates/Dimethicone Methacrylate/Ethylhexyl Acrylate Copolymer, Acrylates/Dimethiconol Acrylate Copolymer, Acrylates/Ethylhexyl Acrylate/Dimethicone Methacrylate Copolymer, Acrylates/Octylacrylamide/Diphenyl Amodimethicone Copolymer, Acrylates/Polytrimethylsiloxymethacrylate Copolymer, Acrylates/Propyl Trimethicone Methacrylate Copolymer, Acrylates/Stearyl Acrylate/Dimethicone Methacrylate Copolymer, Acrylates/Tridecyl Acrylate/Triethoxysilylpropyl Methacrylate/Dimethicone Methacrylate Copolymer, Acrylates/Trifluoropropylmethacrylate/Polytrimethyl Siloxymethacrylate Copolymer, Amino Bispropyl Dimethicone, Aminoethylaminopropyl Dimethicone, Aminopropyl Dimethicone, Aminopropyl Phenyl Trimethicone, Aminopropyl Triethoxysilane, Ammonium Dimethicone PEG-7 Sulfate, Amodimethicone, Amodimethicone Hydroxystearate, Amodimethicone/Silsesquioxane Copolymer, Ascorbyl Carboxydecyl Trisiloxane, Ascorbyl Methylsilanol Pectinate, Behenoxy Dimethicone, Behentrimonium Dimethicone PEG-8 Phthalate, Behenyl Dimethicone, Bisamino PEG/PPG-41/3 Aminoethyl PG-Propyl Dimethicone, Bis-Aminopropyl/Ethoxy Aminopropyl Dimethicone, Bis(Butylbenzoate) Diaminotriazine Aminopropyltrisiloxane, Bis-Butyldimethicone Polyglyceryl-3, Bis-Butyloxyamodimethicone/PEG-60 Copolymer, Bis(C13-15 Alkoxy) Hydroxybutamidoamodimethicone, Bis(C13-15 Alkoxy) PG- Amodimethicone, Bis-(C1-8 Alkyl Lauroyl Lysine Decylcarboxamide) Dimethicone, Bis-Cetyl Cetyl Dimethicone, Bis-Cetyl/PEG-8 Cetyl PEG-8 Dimethicone, Bis-Diphenylethyl Disiloxane, Bis-Ethyl Ethyl Methicone, Bis-Gluconamidoethylaminopropyl Dimethicone, Bis-Hydrogen Dimethicone, Bis-Hydroxyethoxypropyl Dimethicone Bis-Hydroxylauryl, Dimethicone/IPDI Copolymer, Bis-Hydroxy/Methoxy Amodimethicone, Bis-Hydroxypropyl Dimethicone Behenate, Bis-Hydroxypropyl Dimethicone/SMDI Copolymer, Bis-Isobutyl PEG-14/Amodimethicone Copolymer, Bis-Isobutyl PEG- 15/Amodimethicone Copolymer, Bis-Isobutyl PEG/PPG-20/35/Amodimethicone Copolymer, Bis- Isobutyl PEG/PPG-10/7/Dimethicone Copolymer, Bis-Isobutyl PEG-24/PPG-7/Dimethicone Copolymer, Bis-PEG-1 Dimethicone, Bis-PEG-4 Dimethicone, Bis-PEG-8 Dimethicone, Bis-PEG-12 Dimethicone,Bis-PEG-20 Dimethicone, Bis-PEG-12 Dimethicone Beeswax, Bis-PEG-12 Dimethicone Candelillate, Bis-PEG-15 Dimethicone/IPDI Copolymer, Bis-PEG-15 Methyl Ether Dimethicone, Bis- PEG-18 Methyl Ether Dimethyl Silane, Bis-PEG/PPG-14/14 Dimethicone, Bis-PEG/PPG-15/5 Dimethicone, Bis-PEG/PPG-18/6 Dimethicone, Bis-PEG/PPG-20/20 Dimethicone, Bis-PEG/PPG- 16/16 PEG/PPG-16/16 Dimethicone, Bis-PEG/PPG-20/5 PEG/PPG-20/5 Dimethicone, Bisphenylhexamethicone, Bis-Phenylpropyl Dimethicone, Bispolyethylene Dimethicone, Bis-(Polyglyceryl-3 Oxyphenylpropyl) Dimethicone, Bis-(Polyglyceryl-7 Oxyphenylpropyl) Dimethicone, Bis-PPG-15 Dimethicone/IPDI Copolymer, Bis(PPG-7 Undeceneth-21) Dimethicone, Bis-Stearyl Dimethicone, Bis-Trimethoxysilylethyl Tetramethyldisiloxyethyl Dimethicone, Bis-Vinyldimethicone, Bis-Vinyl Dimethicone/Dimethicone Copolymer, Borage Seed Oil PEG-7 Dimethicone Esters, Butyl Acrylate/C6-14 Perfluoroalkylethyl Acrylate/Mercaptopropyl Dimethicone Copolymer, Butyl Acrylate/Hydroxypropyl Dimethicone Acrylate Copolymer, Butyl Dimethicone Acrylate/Cyclohexylmethacrylate/Ethylhexyl Acrylate Copolymer, Butyldimethicone Methacrylate/Methyl Methacrylate Crosspolymer, t-Butyl Dimethyl Silyl Grape Seed Extract, Butyl Polydimethylsiloxyl Ethylene/Propylene/Vinylnorbornene Copolymer, C6-8 Alkyl C3-6 Alkyl Glucoside Dimethicone, C20-24 Alkyl Dimethicone,C24-28 Alkyl Dimethicone, C26-28 Alkyl Dimethicone, C30-45 Alkyl Dimethicone, C30-60 Alkyl Dimethicone, C32 Alkyl Dimethicone, C30-45 Alkyl Dimethicone/Polycyclohexene Oxide Crosspolymer, C26-28 Alkyldimethylsilyl Polypropylsilsesquioxane, C30-45 Alkyldimethylsilyl Polypropylsilsesquioxane, C20-24 Alkyl Methicone, C24-28 Alkyl Methicone, C26-28 Alkyl Methicone, C30-45 Alkyl Methicone, C20-28 Alkyl Perfluorodecylethoxy Dimethicone, C26-54 Alkyl Tetradecyl Dimethicone, Capryl Dimethicone, Caprylyl Dimethicone Ethoxy Glucoside, Caprylyl Methicone, Caprylyl Trimethicone, Carboxydecyl Trisiloxane, Castor Oil Bis-Hydroxypropyl Dimethicone Esters Cerotyl Dimethicone, Cetearyl Dimethicone Crosspolymer, Cetearyl Dimethicone/Vinyl Dimethicone Crosspolymer, Cetearyl Methicone, Cetrimonium Carboxydecyl PEG-8 Dimethicone, Cetrimonium Dimethicone PEG-7 Phthalate, Cetyl Behenyl Dimethicone, Cetyl Dimethicone, Cetyl Dimethicone/Bis-Vinyldimethicone Crosspolymer, Cetyl Hexacosyl Dimethicone, Cetyloxy Dimethicone, Cetyl PEG-8 Dimethicone, Cetyl PEG/PPG-15/15 Butyl Ether Dimethicone, Cetyl PEG/PPG-7/3 Dimethicone, Cetyl PEG/PPG-10/1 Dimethicone, Cetyl Triethylmonium Dimethicone PEG-8 Phthalate, Cetyl Triethylmonium Dimethicone PEG-8 Succinate, Copper Acetyl Tyrosinate Methylsilanol, Copper PCA Methylsilanol, C4-14 Perfluoroalkylethoxy Dimethicone, Cycloethoxymethicone, Cycloheptasiloxane, Cyclohexasiloxane, Cyclomethicone, Cyclopentasiloxane, Cyclophenylmethicone, Cyclotetrasiloxane,mCyclovinylmethicone, Cystine Bis-PG-Propyl Silanetriol, DEA PG-Propyl PEG/PPG-18/21 Dimethicone, Diisostearoyl Trimethylolpropane Siloxy Silicate, Dilauroyl Trimethylolpropane Siloxy Silicate, Dilinoleamidopropyl Dimethylamine Dimethicone PEG-7 Phosphate, Dimethicone, Dimethicone Crosspolymer, Dimethicone Crosspolymer-3, Dimethicone/Divinyldimethicone/Silsesquioxane Crosspolymer, Dimethicone Ethoxy Glucoside, Dimethicone Hydroxypropyl Trimonium Chloride, Dimethicone/Mercaptopropyl Methicone Copolymer, Dimethicone PEG-15 Acetate Dimethicone PEG-8 Adipate, Dimethicone PEG-7 Avocadoate, Dimethicone PEG-8 Avocadoate, Dimethicone PEG-8 Beeswax, Dimethicone PEG-8 Benzoate, Dimethicone PEG-8 Borageate, Dimethicone PEG-7 Cocoate, Dimethicone/PEG-10 Crosspolymer, Dimethicone/PEG-10/15 Crosspolymer, Dimethicone/PEG-15 Crosspolymer, Dimethicone PEG-7 Isostearate, Dimethicone PEG-8 Isostearate, Dimethicone PEG-7 Lactate, Dimethicone PEG-8 Lanolate, Dimethicone PEG-8 Laurate, Dimethicone PEG-8 Meadowfoamate, Dimethicone PEG-7 Octyldodecyl Citrate, Dimethicone PEG-7 Olivate, Dimethicone PEG-8 Olivate, Dimethicone PEG-7 Phosphate, Dimethicone PEG-8 Phosphate, Dimethicone PEG-10 Phosphate, Dimethicone PEG-7 Phthalate, Dimethicone PEG-8 Phthalate, Dimethicone PEG-8 Polyacrylate, Dimethicone PEG/PPG- 20/23 Benzoate, Dimethicone PEG/PPG-7/4 Phosphate, Dimethicone PEG/PPG-12/4 Phosphate, Dimethicone PEG-7 Succinate, Dimethicone PEG-8 Succinate, Dimethicone PEG-7 Sulfate, Dimethicone PEG-7 Undecylenate, Dimethicone PG-Diethylmonium Chloride, Dimethicone/Phenyl Vinyl Dimethicone Crosspolymer, Dimethicone/Polyglycerin-3 Crosspolymer, Dimethicone/PPG-20 Crosspolymer, Dimethicone Propylethylenediamine Behenate, Dimethicone Propyl PG-Betaine, Dimethicone/Silsesquioxane Copolymer, Dimethicone Silylate, Dimethicone/Dimethicone Crosspolymer, Dimethicone/Vinyltrimethylsiloxysilicate Crosspolymer, Dimethiconol, Dimethiconol Arginine, Dimethiconol Beeswax, Dimethiconol Behenate, Dimethiconol Borageate, Dimethiconol Candelillate, Dimethiconol Carnaubate, Dimethiconol Cysteine, Dimethiconol Dhupa Butterate, Dimethiconol Fluoroalcohol Dilinoleic Acid, Dimethiconol Hydroxystearate, Dimethiconol Illipe Butterate, Dimethiconol/IPDI Copolymer, Dimethiconol Isostearate, Dimethiconol Kokum Butterate, Dimethiconol Lactate, Dimethiconol Meadowfoamate, Dimethiconol Methionine, Dimethiconol/Methylsilanol/Silicate Crosspolymer, Dimethiconol Mohwa Butterate, Dimethiconol Panthenol, Dimethiconol Sal Butterate, Dimethiconol/Silica Crosspolymer, Dimethiconol/Silsesquioxane Copolymer, Dimethiconol Stearate, Dimethiconol/Stearyl, Methicone/Phenyl Trimethicone Copolymer, Dimethoxysilyl Ethylenediaminopropyl Dimethicone, Dimethylaminopropylamido PCA Dimethicone, Dimethyl Oxobenzo Dioxasilane, Dimethylsilanol Hyaluronate, Dioleyl Tocopheryl Methylsilanol, Diphenyl Amodimethicone, Diphenyl Dimethicone, Diphenyl Dimethicone Crosspolymer Diphenyl Dimethicone?/inyl Diphenyl Dimethicone/Silsesquioxane Crosspolymer, Diphenylethyl Benzyloxy Dilsiloxane, Diphenylisopropyl Dimethicone, Diphenylsiloxy Phenyl/Propyl Trimethicone, Diphenylsiloxy Phenyl Trimethicone Disiloxane, Disodium Amodimethicone Disuccinamide, Disodium PEG-12 Dimethicone Sulfosuccinate, Disodium PEG-8 Lauryl Dimethicone Sulfosuccinate, Divinyldimethicone/Dimethicone Copolymer, Divinyldimethicone/Dimethicone Crosspolymer, Drometrizole Trisiloxane, Ethylhexyl Acrylate/VP/Dimethicone Methacrylate Copolymer, Ethyl Methicone, Ethyl Trisiloxane, Fluoro C2-8 Alkyldimethicone, Gluconamidopropyl Aminopropyl Dimethicone, 4-(2-Beta-Glucopyranosiloxy) Propoxy-2-Hydroxybenzophenone, Glyceryl Undecyl Dimethicone, Glycidoxy Dimethicone, Hexadecyl Methicone, Hexyl Dimethicone, Hexyl Methicone, Hexyltrimethoxysilane, Hydrogen Dimethicone, Hydrogen Dimethicone/Octyl Silsesquioxane Copolymer, Hydrolyzed Collagen PG-Propyl Dimethiconol, Hydrolyzed Collagen PG-Propyl Methylsilanediol, Hydrolyzed Collagen PG-Propyl Silanetriol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Silk PG-Propyl Methylsilanediol Crosspolymer, Hydrolyzed Soy Protein/Dimethicone PEG-7 Acetate, Hydrolyzed Soy Protein PG-Propyl Methylsilanediol, Hydrolyzed Vegetable Protein PG-Propyl Silanetriol, Hydrolyzed Wheat Protein/Cystine Bis-PG-Propyl Silanetriol Copolymer, Hydrolyzed Wheat Protein/Dimethicone PEG-7 Acetate, Hydrolyzed Wheat Protein/Dimethicone PEG-7 Phosphate Copolymer, Hydrolyzed Wheat Protein PG-Propyl Methylsilanediol, Hydrolyzed Wheat Protein PG-Propyl Silanetriol, Hydroxyethyl Acetomonium PG-Dimethicone, Hydroxypropyldimethicone, Hydroxypropyl Dimethicone Behenate, Hydroxypropyl Dimethicone Isostearate, Hydroxypropyl Dimethicone Stearate, Isobutylmethacrylate/Bis-Hydroxypropyl Dimethicone Acrylate Copolymer, Isobutylmethacrylate/Trifluoroethylmethacrylate/Bis-Hydroxypropyl Dimethicone Acrylate Copolymer, Isopentyl Trimethoxycinnamate Trisiloxane, Isopolyglyceryl-3 Dimethicone, Isopolyglyceryl-3 Dimethiconol, Isopropyl Titanium Triisostearate/Triethoxysilylethyl, Polydimethylsiloxyethyl Dimethicone Crosspolymer, Isostearyl Carboxydecyl PEG-8 Dimethicone, Lactoyl Methylsilanol Elastinate, Lauryl Dimethicone, Lauryl Dimethicone PEG-15 Crosspolymer, Lauryl Dimethicone PEG- 10 Phosphate, Lauryl Dimethicone/Polyglycerin-3 Crosspolymer, Lauryl Methicone, Lauryl PEG-8 Dimethicone, Lauryl PEG-10 Methyl Ether Dimethicone, Lauryl PEG-9 Polydimethylsiloxyethyl Dimethicone, Lauryl PEG/PPG-18/18 Methicone, Lauryl Phenylisopropyl Methicone, Lauryl Phenylpropyl Methicone, Lauryl Polydimethylsiloxyethyl Dimethicone/Bis-Vinyldimethicone Crosspolymer, Lauryl Polyglyceryl-3 Polydimethylsiloxyethyl Dimethicone, Lauryl Trimethicone, Linoleamidopropyl PG-Dimonium Chloride Phosphate Dimethicone, Methacryloyl Propyltrimethoxysilane, Methicone, Methoxy Amodimethicone/Silsesquioxane Copolymer, Methoxycinnamidopropyl Polysilsesquioxane, Methoxycinnamoylpropyl Silsesquioxane Silicate, Methoxy PEG-13 Ethyl Polysilsesquioxane, Methoxy PEG/PPG-7/3 Aminopropyl Dimethicone, Methoxy PEG/PPG-25/4 Dimethicone, Methoxy PEG-10 Propyltrimethoxysilane, Methyleugenyl PEG- 8 Dimethicone, Methylpolysiloxane Emulsion, Methylsilanol Acetylmethionate, Methylsilanol Acetyltyrosine, Methylsilanol Ascorbate, Methylsilanol Carboxymethyl Theophylline, Methylsilanol Carboxymethyl Theophylline Alginate, Methylsilanol Elastinate, Methylsilanol Glycyrrhizinate, Methylsilanol Hydroxyproline, Methylsilanol Hydroxyproline Aspartate, Methylsilanol Mannuronate, Methylsilanol PCA, Methylsilanol PEG-7 Glyceryl Cocoate, Methylsilanol/Silicate Crosspolymer, Methylsilanol Spirulinate, Methylsilanol Tri-PEG-8 Glyceryl Cocoate, Methyl Trimethicone, Methyltrimethoxysilane, Myristylamidopropyl Dimethylamine Dimethicone PEG-7 Phosphate, Myristyl Methicone, Myristyl Trisiloxane, Nylon-611/Dimethicone Copolymer, PCA Dimethicone, PEG-7 Amodimethicone, PEG-8 Amodimethicone, PEG-8 Cetyl Dimethicone, PEG-3 Dimethicone, PEG-6 Dimethicone, PEG-7 Dimethicone, PEG-8 Dimethicone, PEG-9 Dimethicone, PEG-10 Dimethicone, PEG-12 Dimethicone, PEG-14 Dimethicone, PEG-17 Dimethicone, PEG-10 Dimethicone Crosspolymer, PEG-12 Dimethicone Crosspolymer, PEG-8 Dimethicone Dimer Dilinoleate, PEG-8 Dimethicone/Dimer Dilinoleic Acid Copolymer, PEG-10 Dimethicone/Vinyl Dimethicone Crosspolymer, PEG-8 Distearmonium Chloride PG-Dimethicone, PEG-10/Lauryl Dimethicone Crosspolymer, PEG- 15/Lauryl Dimethicone Crosspolymer, PEG-15/Lauryl Polydimethylsiloxyethyl Dimethicone Crosspolymer, PEG-8 Methicone, PEG-6 Methicone Acetate, PEG-6 Methyl Ether Dimethicone, PEG- 7 Methyl Ether Dimethicone, PEG-8 Methyl Ether Dimethicone, PEG-9 Methyl Ether Dimethicone, PEG-10 Methyl Ether Dimethicone, PEG-11 Methyl Ether Dimethicone, PEG-32 Methyl Ether Dimethicone, PEG-8 Methyl Ether Triethoxysilane, PEG-10 Nonafluorohexyl Dimethicone Copolymer, PEG-4 PEG-12 Dimethicone, PEG-8 PG-Coco-Glucoside Dimethicone, PEG-9 Polydimethylsiloxyethyl Dimethicone, PEG/PPG-20/22 Butyl Ether Dimethicone, PEG/PPG-22/22 Butyl Ether Dimethicone, PEG/PPG-23/23 Butyl Ether Dimethicone, PEG/PPG-24/18 Butyl Ether Dimethicone, PEG/PPG-27/9 Butyl Ether Dimethicone, PEG/PPG-3/10 Dimethicone, PEG/PPG-4/12 Dimethicone, PEG/PPG-6/4 Dimethicone, PEG/PPG-6/11 Dimethicone, PEG/PPG-8/14 Dimethicone, PEG/PPG-8/26 Dimethicone, PEG/PPG-10/2 Dimethicone, PEG/PPG-12/16 Dimethicone, PEG/PPG- 12/18 Dimethicone, PEG/PPG-14/4 Dimethicone, PEG/PPG-15/5 Dimethicone, PEG/PPG-15/15 Dimethicone, PEG/PPG-16/2 Dimethicone, PEG/PPG-16/8 Dimethicone, PEG/PPG-17/18 Dimethicone, PEG/PPG-18/6 Dimethicone, PEG/PPG-18/12 Dimethicone, PEG/PPG-18/18 Dimethicone, PEG/PPG-19/19 Dimethicone, PEG/PPG-20/6 Dimethicone, PEG/PPG-20/15 Dimethicone, PEG/PPG-20/20 Dimethicone, PEG/PPG-20/23 Dimethicone, PEG/PPG-20/29 Dimethicone, PEG/PPG-22/23 Dimethicone, PEG/PPG-22/24 Dimethicone, PEG/PPG-23/6 Dimethicone, PEG/PPG-25/25 Dimethicone, PEG/PPG-27/27 Dimethicone, PEG/PPG-30/10 Dimethicone, PEG/PPG-25/25 Dimethicone/Acrylates Copolymer, PEG/PPG-20/22 Methyl Ether Dimethicone, PEG/PPG-24/24 Methyl Ether Glycidoxy Dimethicone, PEG/PPG-10/3 Oleyl Ether Dimethicone, PEG/PPG-5/3 Trisiloxane, PEG-4 Trifluoropropyl Dimethicone Copolymer, PEG-8 Trifluoropropyl Dimethicone Copolymer, PEG-10 Trifluoropropyl Dimethicone Copolymer, PEG-8 Trisiloxane, Perfluorocaprylyl riethoxysilylethyl Methicone, Perfluorononyl Dimethicone, Perfluorononyl Dimethicone/Methicone/Amodimethicone Crosspolymer, Perfluorononylethyl Carboxydecyl Behenyl Dimethicone, Perfluorononylethyl Carboxydecyl Hexacosyl Dimethicone, Perfluorononylethyl Carboxydecyl Lauryl/Behenyl Dimethicone, Perfluorononylethyl Carboxydecyl Lauryl Dimethicone, Perfluorononylethyl Carboxydecyl PEG-8 Dimethicone, Perfluorononylethyl Carboxydecyl PEG-10 Dimethicone, Perfluorononylethyl Dimethicone/Methicone Copolymer, Perfluorononylethyl PEG-8 Dimethicone, Perfluorononylethyl Stearyl Dimethicone, Perfluorooctylethyl/Diphenyl Dimethicone Copolymer, Perfluorooctylethyl Triethoxysilane, Perfluorooctylethyl Trimethoxysilane, Perfluorooctylethyl Trisiloxane, Perfluorooctyl Triethoxysilane, PG-Amodimethicone, Phenethyl Dimethicone, Phenethyl Disiloxane, Phenyl Dimethicone, Phenylisopropyl Dimethicone, Phenyl Methicone, Phenyl Methiconol, Phenylpropyldimethylsiloxysilicate, Phenylpropyl Ethyl Methicone, Phenyl Propyl Trimethicone, Phenyl Propyl Trimethicone/Diphenylmethicone, Phenyl Trimethicone, Platinum Divinyldisiloxane, Polyacrylate-6, Polydiethylsiloxane, Polydimethylsiloxyethyl Dimethicone/Bis-Vinyldimethicone Crosspolymer, Polydimethylsiloxyethyl Dimethicone/Methicone Copolymer, Polydimethylsiloxy PEG/PPG-24/19 Butyl Ether Silsesquioxane, Polydimethylsiloxy PPG- 13 Butyl Ether Silsesquioxane, Polyglyceryl-3 Disiloxane Dimethicone, Polyglyceryl-3/Lauryl Polydimethylsiloxyethyl Dimethicone Crosspolymer, Polyglyceryl-3 Polydimethylsiloxyethyl Dimethicone, Poly(Glycol Adipate)/Bis-Hydroxyethoxypropyl Dimethicone Copolymer, Polymethylsilsesquioxane, Polymethylsilsesquioxane/Trimethylsiloxysilicate, Polyphenylsilsesquioxane, Polypropylsilsesquioxane, Polysilicone-1, Polysilicone-2, Polysilicone-3, Polysilicone-4, Polysilicone-5, Polysilicone-6, Polysilicone-7, Polysilicone-8, Polysilicone-9, Polysilicone-10, Polysilicone-11, Polysilicone-12, Polysilicone-13, Polysilicone-14, Polysilicone-15, Polysilicone-16, Polysilicone-17, Polysilicone-18, Polysilicone-19, Polysilicone-20, Polysilicone-21, Polysilicone-18 Cetyl Phosphate, Polysilicone-1 Crosspolymer, Polysilicone-18 Stearate, Polyurethane-10, Potassium Dimethicone PEG-7 Panthenyl Phosphate, Potassium Dimethicone PEG- 7 Phosphate, PPG-12 Butyl Ether Dimethicone, PPG-2 Dimethicone, PPG-12 Dimethicone, PPG-27 Dimethicone, PPG-4 Oleth-10 Dimethicone, Propoxytetramethyl Piperidinyl Dimethicone, Propyl Trimethicone, Quaternium-80, Retinoxytrimethylsilane, Silanediol Salicylate, Silanetriol, Silanetriol Arginate, Silanetriol Glutamate, Silanetriol Lysinate, Silanetriol Melaninate, Silanetriol Trehalose Ether, Silica, Silica Dimethicone Silylate, Silica Dimethyl Silylate, Silica Silylate, Silicon Carbide, Silicone Quaternium-1, Silicone Quaternium-2, Silicone Quaternium-2 Panthenol Succinate, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, SiliconeQuaternium-16, Silicone Quaternium-16/Glycidoxy Dimethicone Crosspolymer, Silicone Quaternium-17, Silicone Quaternium- 18, Silicone Quaternium-19, Silicone Quaternium-20, Silicone Quaternium-21, Silicone Quaternium- 22, Silicone Quaternium-24, Silicone Quaternium-25, Siloxanetriol Alginate, Siloxanetriol Phytate, Simethicone, Sodium Carboxydecyl PEG-8 Dimethicone, Sodium Dimethicone PEG-7 Acetyl Methyltaurate, Sodium Hyaluronate Dimethylsilanol, Sodium Lactate Methylsilanol, Sodium Mannuronate Methylsilanol, Sodium PCA Methylsilanol, Sodium PG-Propyldimethicone Thiosulfate Copolymer, Sodium PG-Propyl Thiosulfate Dimethicone, Sodium Propoxyhydroxypropyl Thiosulfate Silica, Sorbityl Silanediol, Soy Triethoxysilylpropyldimonium Chloride, Stearalkonium Dimethicone PEG-8 Phthalate, Stearamidopropyl Dimethicone, Steardimonium Hydroxypropyl Panthenyl PEG-7 Dimethicone Phosphate Chloride, Steardimonium Hydroxypropyl PEG-7 Dimethicone Phosphate Chloride, Stearoxy Dimethicone, Stearoxymethicone/Dimethicone Copolymer, Stearoxytrimethylsilane, Stearyl Aminopropyl Methicone, Stearyl Dimethicone, Stearyl/Lauryl Methacrylate Crosspolymer, Stearyl Methicone, Stearyl Triethoxysilanek, Stearyl Trimethicone, Styrene/Acrylates/Dimethicone Acrylate Crosspolymer, Styrene/Acrylates/Dimethicone Copolymer, TEA-Dimethicone PEG-7 Phosphate, Tetrabutoxypropyl Trisiloxane, Tetramethyl Hexaphenyl Tetrasiloxane, Tetramethyl Tetraphenyl Trisiloxane, Tocopheryloxypropyl Trisiloxane, Trideceth-9 PG-Amodimethicone, Triethoxycaprylylsilane, Triethoxysilylethyl Dimethicone/Methicone Copolymer, Triethoxysilylethyl Polydimethylsiloxyethyl Dimethicone, Triethoxysilylethyl Polydimethylsiloxyethyl Hexyl Dimethicone, Triethoxysilylpropylcarbamoyl Ethoxypropyl Butyl Dimethicone, Trifluoromethyl C1-4 Alkyl Dimethicone, Trifluoropropyl Cyclopentasiloxane, Trifluoropropyl Cyclotetrasiloxane, Trifluoropropyl Dimethicone, Trifluoropropyl Dimethicone/PEG-10 Crosspolymer, Trifluoropropyl Dimethicone/Trifluoropropyl Divinyldimethicone Crosspolymer, Trifluoropropyl Dimethicone/Vinyl Trifluoropropyl, Dimethicone/Silsesquioxane Crosspolymer, Trifluoropropyl Dimethiconol, Trifluoropropyldimethyl/trimethylsiloxysilicate, Trifluoropropyl Methicone, Trimethoxycaprylylsilane, Trimethoxysilyl Dimethicone, Trimethyl Pentaphenyl Trisiloxane, Trimethylsiloxyamodimethicone, Trimethylsiloxyphenyl Dimethicone, Trimethylsiloxysilicate, Trimethylsiloxysilicate/Dimethicone Crosspolymer, Trimethylsiloxysilicate/Dimethiconol Crosspolymer, Trimethylsiloxysilylcarbamoyl Pullulan, Trimethylsilyl Hydrolyzed Conchiolin Protein PG-Propyl Methylsilanediol Crosspolymer, Trimethylsilyl Hydrolyzed Silk PG-Propyl Methylsilanediol Crosspolymer, Trimethylsilyl Hydrolyzed Wheat Protein PG-Propyl Methylsilanediol Crosspolymer, Trimethylsilyl Pullulan, Trimethylsilyl Trimethylsiloxy Glycolate, Trimethylsilyl Trimethylsiloxy Lactate, Trimethylsilyl Trimethylsiloxy Salicylate, Triphenyl Trimethicone, Trisiloxane, Tris-Tributoxysiloxymethylsilane, Undecylcrylene Dimethicone, Vinyl Dimethicone, Vinyl Dimethicone/Lauryl Dimethicone Crosspolymer, Vinyl Dimethicone/Methicone Silsesquioxane Crosspolymer, Vinyldimethyl/Trimethylsiloxysilicate Stearyl Dimethicone Crosspolymer, VP/Dimethiconylacrylate/Polycarbamyl/Polyglycol Ester, Zinc Carboxydecyl Trisiloxane, Zinc Dimethicone PEG-8 Succinate, and mixtures thereof.

More preferably, the silicones to be contained in the cosmetic or pharmaceutical composition according to the invention are Dimethicone, Cyclomethicone, Cyclopentasiloxane, Cyclotetrasiloxane, Phenyl Trimethicone, and Cyclohexasiloxane.

**Waxes and/or stabilizers:** Besides natural oils used, one or more waxes may also be present in the cosmetic or pharmaceutical composition according to the present invention, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes. Metal salts of fatty acids such as, for example, magnesium, aluminium and/or zinc stearate or ricinoleate may be used as stabilizers.

**Actives modulating skin and/or hair pigmentation:** The cosmetic or pharmaceutical composition according to the present invention may include active ingredients for skin and/or hair lightening. Preferred active ingredients for skin and/or hair lightening are selected from the group consisting of kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates, sulfur-containing molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin and hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract.

Preferred skin lighteners are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with sclareolide according to the present invention. In addition, said preferred skin lighteners are readily available.

Advantageous skin and hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophyl-line and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-Ile- Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(II) bicarbonate complexes ("pseudocat-alases"), tetrasubstituted cyclohexene derivatives, isoprenoids, melanin derivatives such as Melasyn-100 and MelanZe, diacyl glycerols, aliphatic or cyclic diols, psoralens, prostaglandins and ana-logues thereof, activators of adenylate cyclase and compounds which activate the transfer of melanosomes to keratinocytes such as serine proteases or agonists of the PAR-2 receptor, extracts of plants and plant parts of the chrysanthemum species, san-guisorba species, walnut extracts, urucum extracts, rhubarb extracts, microalgae extracts, in particular Isochrysis galbana, trehalose, erythru-lose and dihydroxyacetone. Flavonoids which bring about skin and hair tinting or brown-ing (e.g. quercetin, rhamnetin, kaempferol, fisetin, genistein, daidzein, chrysin and api-genin, epicatechin, diosmin and diosmetin, morin, quercitrin, naringenin, hesperidin, phloridzin and phloretin) can also be used.

**Hair growth activators or inhibitors:** Cosmetic or pharmaceutical compositions according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormones, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/nd hydrolysates from rice or wheat.

Alternatively, the cosmetic or pharmaceutical composition according to the present invention may include one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

**Enzyme inhibitors:** The cosmetic or pharmaceutical composition according to the present invention may comprise one or more enzyme inhibitors. Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

**Odour absorbers and/or antiperspirant active agents:** The cosmetic or pharmaceutical composition according to the present invention may include one or more odour absorbers and/or antiperspirant active agents (antiperspirants). Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β- damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

**Film formers:** The cosmetic or pharmaceutical composition according to the present invention may include one or more film formers. Standard film formers are preferably chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

**Carriers and hydrotropes:** The cosmetic or pharmaceutical composition according to the present invention may comprise a carrier or a mixture of different carriers. Preferred cosmetics carrier materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water, and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

The cosmetic or pharmaceutical composition as defined herein may optionally include powders. The optional powders provide formulas that are smoother and softer on the skin. Representative powders include talc, mica, magnesium carbonate, calcium carbonate, magnesium silicate, aluminium magnesium silicate, silica, titanium dioxide, zinc oxide, red iron oxide, yellow iron oxide, black iron oxide, polyethylene powder, methacrylate powder, polystyrene powder, silk powder, Preferred solid powder materials, which may be a component of the cosmetic or pharmaceutical composition according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin, inulin, and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine; or
- dialcoholamines, such as diethanolamine or 2-aminopropane-1,3-diol.

**Dyes:** The cosmetic or pharmaceutical composition according to the present invention may comprise one or more dyes. Suitable dyes are any of the substances suitable and approved for cosmetic purposes. Examples include cochineal red A (Cl. 16255), patent blue V (Cl. 42051), indigotin (Cl. 73015), chlorophyllin (Cl. 75810), quinoline yellow (Cl. 47005), titanium dioxide (CI. 77891), indanthrene blue RS (Cl. 69800) and madder lake (CI. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

In addition to the above-described substances, further ingredients commonly used in the cosmetic or pharmaceutical industry, which are suitable or customary in the compositions of the present invention, can be used.

The 1,2-heptanediol or the 2,3-heptanediol or the mixture comprising 1,2-heptanediol and 2,3-heptanediol according to the first aspect of the present invention is present in the cosmetic or pharmaceutical composition or the homecare product in an amount of 0.001 to 15.0 % by weight, based on the total weight of the composition or homecare product. In a preferred variant, the cosmetic or pharmaceutical composition or homecare product comprises the 1,2-heptanediol or the 2,3-heptanediol or the mixture comprising 1,2-heptanediol and 2,3-heptanediol in an amount of 0.01 to 10.0 % by weight, based on the total weight of the composition or homecare product. In a more preferred variant, the 1,2-heptanediol or the 2,3-heptanediol or the mixture comprising 1,2-heptanediol and 2,3-heptanediol is advantageously used in the cosmetic or pharmaceutical composition in an amount of at 0.1 to 5.0 % by weight, based on the total weight of the composition or homecare product. In a still more preferred variant, the 1,2-heptanediol or the 2,3-heptanediol or the mixture comprising 1,2-heptanediol and 2,3-heptanediol is advantageously used in the cosmetic or pharmaceutical composition in an amount of at 0.3 to 3.0 % by weight, based on the total weight of the composition or homecare product. In a most preferred variant, the 1,2-heptanediol or 2,3-heptanediol or the mixture comprising 1,2-heptanediol and 2,3-heptanediol is advantageously used in the cosmetic or pharmaceutical composition or homecare product in an amount of 0.5 to 1.0 % by weight, based on the total weight of the composition or homecare product.

For the heptanediol alkane mixture, the above amounts relate to the total content of the 1,2-heptanediol and the 2,3-heptanediol in the mixture, i.e. the amount is the sum of the content of 1,2-heptanediol and 2,3-heptanediol in the mixture.

The at least one linear alkanediol or the mixture comprising at least one first linear alkanediol and one or more second linear alkanediol according to the second aspect of the present invention is present in the cosmetic or pharmaceutical composition or homecare product in an amount of 0.001 to 15.0 % by weight, based on the total weight of the composition or homecare product. In a preferred variant, the cosmetic or pharmaceutical composition or homecare product comprises the at least one linear alkanediol or the mixture comprising at least one first linear alkanediol and one or more second linear alkanediol in an amount of 0.01 to 10.0 % by weight, based on the total weight of the composition or homecare product. In a more preferred variant, the at least one linear alkanediol or the mixture comprising at least one first linear alkanediol and one or more second linear alkanediol is advantageously used in the cosmetic or pharmaceutical composition or homecare product in an amount of at 0.1 to 5.0 % by weight, based on the total weight of the composition or homecare product. **In** a still more preferred variant, the at least one linear alkanediol or the mixture comprising at least one first linear alkanediol and one or more second linear alkanediol is advantageously used in the cosmetic or pharmaceutical composition or homecare product in an amount of at 0.3 to 3.0 % by weight, based on the total weight of the composition or homecare product. In a most preferred variant, the at least one linear alkanediol or the mixture comprising at least one first linear alkanediol and one or more second linear alkanediol is advantageously used in the cosmetic or pharmaceutical composition or homecare product in an amount of at 0.5 to 1.0 % by weight, based on the total weight of the composition or homecare product.

For the alkane mixture, the above amounts relate to the total content of the first linear alkanediols and the second linear alkanediols in the mixture, i.e. the amount is the sum of the content of the first linear alkanediols and the second linear alkanediols in the mixture.

In a particular preferred variant according to the first aspect of the present invention, the cosmetic or pharmaceutical composition or the homecare product comprises the 2,3-heptanediol or the 2,3-heptanediol of the mixture comprising 1,2-heptanediol and 2,3-heptanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, and most preferred in an amount of 0.5 to 1.0 % by weight, based on the total weight of the composition or homecare product.

Even more preferred, the cosmetic or pharmaceutical composition or the homecare product comprises the 2,3-heptanediol or the 2,3-heptanediol of the mixture comprising 1,2-heptanediol and 2,3-heptanediol in an amount of 0.001 to 0.5 % weight, preferably in an amount of 0.005 to 0.1 % by weight and most preferably in an amount of 0.01 to 0.075 % by weight, based on the total weight of the composition or homecare product.

In a particular preferred variant according to the second aspect of the present invention, the cosmetic or pharmaceutical composition or the homecare product comprises the 2,3-alkanediol as linear alkanediol or of the mixture comprising at least one first linear alkanediol and one or more second linear alkanediol in an amount of 0.001 to 15.0 % by weight, preferably in an amount of 0.01 to 10.0 % by weight, more preferred in an amount of 0.1 to 5.0 % by weight, still more preferred in an amount of 0.3 to 3.0 % by weight, and most preferred in an amount of 0.5 to 1.0 % by weight, based on the total weight of the composition or homecare product.

Even more preferred, the cosmetic or pharmaceutical composition or the homecare product comprises the 2,3-alkanediol as linear alkanediol or of the mixture comprising at least one first linear alkanediol and one or more second linear alkanediol in an amount of 0.001 to 0.5 % weight of the composition or homecare product, preferably in an amount of 0.005 to 0.1 % by weight and most preferably in an amount of 0.01 to 0.075 % by weight, based on the total weight of the composition or homecare product.

The solid UV filter component and optionally further UV filter components as defined herein is/are present in the cosmetic or pharmaceutical composition or homecare product according to the present invention, in an amount of 0.1 to 50.0 % by weight, based on the total weight of the composition or homecare product. In a preferred variant, the cosmetic or pharmaceutical composition or homecare product comprises the solid UV filter component and optionally further UV filter components as defined herein in an amount of 0.5 to 45.0 % by weight, based on the total weight of the composition or homecare product. In a more preferred variant, the solid UV filter component and optionally further UV filter components as defined herein is/are advantageously used in the cosmetic or pharmaceutical composition or homecare product in an amount of 1.0 to 40 % by weight, based on the total weight of the composition or homecare product. Thus, the above amounts relate either to the content of the solid UV filter component if the solid UV filter component is used solely, or to the total content of all UV filters, if the solid UV filter component is used in combination with further UV filter components as defined herein, i.e. the amount relate to the sum of the content of all UV filters present in the composition or homecare product.

As is demonstrated in the following examples, the presence of 1,2-heptanediol considerably improves the solubility of a solid UV filter in a cosmetic or pharmaceutical composition compared to 1,2-pentanediol and 1,2-hexanediol. Surprisingly, the combination of a solid UV filter as defined herein with 1,2-heptanediol results in a non-turbid, clear and stable solution. After storage, the solution or emulsion with 1,2-heptanediol is stable and showed either no recrystallization or precipitation of the solid UV filter component or showed less, i.e. the lowest, recrystallization or precipitation of the solid UV filter, compared to 1,2-pentanediol, 1,2-hexanediol or 1,2-octanediol.

The same effect could be observed for 2,3-alkanedioles, in particular 2,3-heptanediol and 2,3-octanediol. The use of said compounds also considerably improves the solubility of a solid UV filter in a cosmetic or pharmaceutical composition compared to 1,2-pentanediol and 1,2-hexanediol, as it is demonstrated by the following examples. Surprisingly, the combination of a solid UV filter as defined herein with 2,3-heptanediol or 2,3-octanediol results in a non-turbid, clear and stable solution. After storage, the solution or emulsion with 2,3-heptanediol or 2,3-octanediol is stable and showed either no recrystallization or precipitation of the solid UV filter component or showed less, i.e. the lowest, recrystallization or precipitation of the solid UV filter, compared to 1,2-pentanediol, 1,2-hexanediol or 1,2-octanediol.

Surprisingly, the same effect could be observed for a mixture including a 1,2-alkanediol and a 2,3-alkanediol. The use of a blend including 1,2-heptanediol and 2,3-heptanediol (1 : 1) remarkably improves the solubility of a solid UV filter and result in a clear, non-turbid solution without recrystallization of the solid UV filter. The use of blends containing mixtures of 1,2-heptanediol and 2,3-heptanediol in the ratio 9 : 1 and in the ratio 7 : 3 showed only slight recrystallization. However, the comparative solution samples containing only 1,2-heptanediol or 2,3-heptanediol showed a poorer solubility against the solid UV filter. Thus, a mixture including 1,2-heptanediol and 2,3-heptanediol leads to a clearly improved solubility in comparison to 1,2-heptanediol and 2,3-heptanediol either alone. An improved solubility against a solid UV filter could also be observed for a blend of 1,2-octanediol and 2,3-octanediol. The use of a blend containing a mixture of 1,2-octanediol and 2,3-octanediol in a ratio of 70 : 30 is not completely clear but shows only a low grade of recrystallisation compared to the solution samples containing only 1,2-octanediol or 2,3-octanediol alone.

Without the use of an alkanediol or an alkanediol mixture as specified herein, the solubility of a solid UV filter in a cosmetic or pharmaceutical preparation or homecare product would be poor, so that storage of such formulations would be problematic or even impossible.

Due to its better solubility, the availability of the solid UV filter in the cosmetic or pharmaceutical composition and, thus, on skin upon application of the cosmetic or pharmaceutical composition, is increased. Additionally, due to its better solubility, the solid UV filter component can be furnished in a higher concentration in the final formulation or can be furnished in a lower concentration in the final formulation to have the same sun protection effect compared to a composition including a UV filter with low solubility and without the additional of an alkanediol.

Due to its better solubility, a uniformly solubilized product, where the UV filter is uniformly dispersed in the product as very small droplets can be obtained. Thus, the concentration of the UV filter is exactly the same through the whole formulation from the very first splash to the very last ml till the bottle is completely empty. Without the use of an alkanediol as specified herein, the distribution of the UV filter would be quite haphazardly in the formulation and would vary from application to application.

The use of 1,2-heptanediol or 2,3-heptanediol or a mixture comprising 1,2-heptanediol and 2,3-heptanediol or a linear alkanediol or a mixture comprising a first linear alkanediol and a second linear alkanediol provides a stable formulation without turbidity or recrystallization of precipitation of the solid UV filter component, which can even maintained during storage.

In addition to the solubility improvement for solid UV filters, with the use of 1,2-heptanediol or 2,3-heptanediol or a mixture of 1,2-heptanediol and 2,3-heptanediol or the use of 1,2-octanediol or 2,3-octanediol or a mixture of 1,2-octanediol and 2,3-octanediol also the sensory properties, i.e. skin sensation, of the cosmetic or pharmaceutical composition can be improved. As it is obvious from Table 13, the spreading behavior of the oil components as defined above and commonly used for the preparation of cosmetic or pharmaceutical compositions, can be significantly improved. Additionally, for all samples an oily or fatty/greasy skin feeling, imparting unwanted gloss to the skin and rendering the composition sticky, can be considerably improved by the use of one of the aforementioned alkanediols. Furthermore, the test shows, that the oil components of the composition are better absorbed and do not leave an off-feeling on the skin when applied in combination with one of the aforementioned alkanediol.

For some oily component samples, especially solid UV filters, even two or three of the abovementioned criteria, i.e. spreading and/or fatty/greasy skin feeling and/or absorption can be improved, resulting in excellent sensory properties of the applied cosmetic or pharmaceutical composition comprising an oil component according to the present invention.

In particular, oily components which have a poor spreadability by nature, which initial spreading, i.e. without the addition of an alkanediol components, was scored with < 2, show a remarkably improvement in spreading both the tested single alkanediol compounds and the tested alkanediol mixture. 1,2-heptanediol and a mixture of 1,2-heptanediol and 2,3-heptanediol perform best.

The cosmetic or pharmaceutical, in particular dermatological, composition according to the first aspect or second aspect of the present invention is intended for topical applications. The term "topical" is understood to mean external applications on a mammal's skin or mucosa, which are in particular for the treatment, protection, care and cleansing of the skin, scalp, eyelashes, eyebrows, nails, mucous membranes and hair. The mammal is preferably a human.

For topical application, the cosmetic or pharmaceutical composition is either a rinse off or a leave on preparation.

The cosmetic or pharmaceutical, in particular dermatological, composition according to the first aspect and second aspect of the present invention can be present in different forms, e.g. in the form of a dispersion, in the form of a water free formulation, or in the form of an aqueous, aqueous/alcoholic, in particular aqueous/ethanolic, or an aqueous/glycolic based solution.

In a preferred variant, the cosmetic or pharmaceutical composition according to the first and second aspect of the invention is a dispersion. The term "dispersion" in the context of the present invention means, that the cosmetic or pharmaceutical composition is a disperse two-phase system consisting of colloidal particles (disperse phase) and a medium in which they are suspended (disperse medium). Both phases are not miscible with each other, only with an emulsifier. Such dispersions, for example emulsions, comprise the at least one oil component (without the solid UV filter and optionally further UV filters) preferably in an amount of ≥ 1 % by weight, more preferably in an amount of ≥ 3 % by weight.

If the cosmetic or pharmaceutical composition according to the present invention is a dispersion, preferably an emulsion, the oil component (without the solid UV filter and optionally further UV filters) is present in the cosmetic or pharmaceutical composition in an amount of 0.01 to 50.0 % by weight, based on the total weight of the composition. In a preferred variant, the cosmetic or pharmaceutical composition comprises the oil component in an amount of 1.0 to 40.0 % by weight, based on the total weight of the composition. In a particular preferred variant, the oil component is advantageously used in the cosmetic or pharmaceutical composition in an amount of at 3.0 to 30 % by weight, based on the total weight of the composition.

Preferably, the cosmetic or pharmaceutical composition according to the first aspect or second aspect of the present invention takes various forms such as an emulsion, in particular a O/W emulsion, a W/O emulsion, a multiple emulsion, a hydrodispersion gel, a balm, a multiple emulsion of the water-in-oil type (W/O/WO) or of the oil-in-water type (O/W/O), PIT emulsion, Pickering emulsion, a micro-emulsion, a liquid, a lotion, a suspension, a milk, an ointment, a paste, a gel, a cream based, an oil based or a liposome-based formulation.

In a further variant, the cosmetic or pharmaceutical, preferably dermatological, composition according to the first and second aspect of the invention is a water free formulation, i.e. an oil formulation.

If the cosmetic or pharmaceutical composition according to the present invention is a water free formulation, i.e. an oil formulation, the oil component is present in the cosmetic or pharmaceutical composition in an amount of ≥ 60 % by weight, preferably in an amount of ≥ 75 % by weight, more preferably in an amount of ≥ 90 % by weight, based on the total weight of the composition.

Such water free formulations include e.g. oils, skin butters, powders, lip stick, antiperspirant/deo stick, and decorative cosmetics, etc.

Alternatively, the cosmetic or pharmaceutical composition as disclosed herein is an aqueous or aqueous/alcoholic, preferably aqueous/ethanolic, or aqueous/glycolic solution. Said solution is a homogeneous one phase system of water and additional components. The aqueous/alcoholic or aqueous/glycolic based solution comprises an aliphatic alcohol or a glycol in an amount of 0.1 to ≤ 50 % by weight, preferably in an amount of 0.5 to 30 % by weight, more preferably in an amount of 2 to 20 % by weight, based on the total weight of the solution. The aliphatic alcohol is preferably selected from the group consisting of ethanol, isopropanol, n-propanol. The glycol is preferably selected from the group consisting of glycerin, propyleneglycol, butyleneglycol or dipropylene glycol.

Preferably, the overall water content in the final solutions can be ≥ 60 % by weight, more preferably ≥ 70 % by weight, still more preferably ≥ 80 % by weight, most preferably ≥ 90 % by weight. New applications such as those including solutions for wet wipes have high water content.

Such aqueous or aqueous/alcoholic or aqueous/glycolic based solutions include for example deo/antiperspirant preparations, after shave, cleansing preparations, or anti-acne preparations. In a preferred variant, the inventive composition can be used for wet wipe applications; they may then most preferably contain ≥ 95 % by weight water, or even ≥ 98 % by weight water.

In a most preferred variant, the cosmetic or pharmaceutical composition according to the present invention is in the form of an emulsion as defined herein, advantageously in the form of an oil-in-water (O/W) emulsion comprising an oily phase dispersed in an aqueous phase in the presence of an O/W emulsifier.

In another preferred variant, the cosmetic or pharmaceutical composition according to the present invention is in the form of an aqueous/alcoholic, preferably aqueous/ethanolic, or aqueous/glycolic based solution. Typically, this could be glycerin in water compositions.

The above formulations or compositions are prepared according to usual and known methods.

In a further aspect, the composition according to the first and second aspect of the present invention is a preparation for cosmetic and/or non-therapeutic use for personal care, skin protection, skin care, scalp protection, scalp care, hair care, nail care, in particular for the prevention and treatment of skin conditions, intolerant or sensitive skin, skin irritation, skin reddening, wheals, pruritis (itching), skin aging, wrinkle formation, loss of skin volume, loss of skin elasticity, piment spots, pigment abnormalities, skin dryness, flaking, greasiness, hypopigmentation and/or hyperpigmentation of the skin; or a preparation for animal care.

Examples of personal care are preferably anti-ageing preparations, skin care emulsions, body oils, body lotions, cleansing lotions, face or body balms, after shave balms, after sun balms, deo emulsions, cationic emulsions, body gels, treatment creams, skin protection ointments, moisturizing gels, face and/or body moisturizers, light protective preparations (sunscreens), micellar water, hair sprays, color protection hair care products, skin lightning products, anti-dark-spot reparation, etc.

Alternatively, the composition according to the present invention is a preparation of medical use.

The pharmaceutical, in particular dermatological, composition according to the present invention is a preparation for the prevention and treatment of a condition of the skin or mucosa.

Preferably, the pharmaceutical composition according to the first and second aspect of the present invention is used in the prevention and/or treatment of of dysfunctions of human hair, skin and/or nails, in particular dermatological or keratological diseases, wherein the dermatological or keratological diseases are selected from the group consisting of atopic dermatitis (neurodermitis), psoriasis, acneiform exanthema, sebostasis, xerosis, eczema, hyper seborrhea and hypo seborrhea, dermatitis, rosacea, erythema, wheals, pruritus (itching), inflammation, irritation, fibrosis, *lichen planus, pityriasis rosea, pityriasis versicolor,* autoimmune bullous diseases, urticaria, angioedema, allergic skin reactions, wound healing, tissue regeneration and in the treatment of inflammatory diseases.

In order to be used, the cosmetic or pharmaceutical, in particular dermatological composition according to the first aspect or second aspect of the present invention is applied to the skin, hair, scalp and/or nails in an adequate amount in such manner as is customary with cosmetics and dermatological products.

The homecare products according to the first and second aspect of the invention are predominantly detergent formulations, usually liquids, powders, sprays, granules or tablets, used to remove dirt, including dust, stains, bad smells and clutter on surfaces or other types of housecleaning or laundry detergent that is added for cleaning laundry or liquid soap. Typical homecare products include all-purpose cleaners, dishwashing detergents, hard floor and surface cleaners, glass cleaners, carpet cleaners, oven cleaners, laundry detergents, fabric softeners, laundry scent, scent lotions, car shampoo, rim block gel, car shampoo, furniture polish, all aforementioned goods also in encapsulated form or air fresheners.

Surprisingly, the use of 1,2-heptanediol or a 2,3-alkanediol such as 2,3-heptanediol or 2,3-octanediol improves the solubility of a solid UV filter in a cosmetic or pharmaceutical formulation. The solid UV filter does not crystallize out or precipitate out not even storage and results in a stable cosmetic or pharmaceutical formulation.

Thus, the present invention relates in a further aspect to the use of 1,2-heptanediol or 2,3-heptanediol or a mixture including 1,2-heptanediol and 2,3-heptanediol or of at least one linear alkanediol or of a mixture comprising at least one first linear alkanediol and one or more second linear alkanediol for improving the solubility of a solid UV filter as defined herein, and/or for improving the availability of the UV filter in a cosmetic or pharmaceutical composition, and, thus, on skin upon application of the cosmetic or pharmaceutical composition. With less recrystallization or without recrystallization of the solid UV filter, also the shelf life of a cosmetic or pharmaceutical composition can be improved.

The present invention also relates to the objects according to the following 30 clauses.
1. A cosmetic or pharmaceutical composition or homecare product, comprising or consisting of
   (a1) 1,2-heptanediol;
   (b1) at least one solid UV filter; and
   (c1) optionally at least one active substance for a cosmetic or pharmaceutical composition or a homecare product and/or additive;
      or
   (a2) 2,3-heptanediol;
   (b2) at least one solid UV filter; and
   (c2) optionally at least one active substance for a cosmetic or pharmaceutical composition or a homecare product and/or additive;
      or
   (a3) a mixture comprising 1,2-heptanediol and 2,3-heptanediol;
   (b3) at least one solid UV filter; and
   (c3) optionally at least one active substance for a cosmetic or pharmaceutical composition or a homecare product and/or additive.
2. A cosmetic or pharmaceutical composition or homecare product, comprising or consisting of
   (a) at least one linear alkanediol having a carbon chain of 5 to 14 carbon atoms or a mixture comprising at least one first linear alkanediol having a carbon chain of 5 to 14 carbon atoms and one or more second linear alkanediol having a carbon chain of 5 to 14 carbon atoms which is different from the first linear alkanediol;
   (b) at least one solid UV filter; and
   (c) optionally at least one active substance for a cosmetic or pharmaceutical composition or a homecare product and/or additive.
3. Cosmetic or pharmaceutical composition or homecare product according to clause 2, wherein the linear alkanediol or the first linear alkanediol is selected from the group consisting of pentanediol, hexanediol, heptanediol, octanediol, nonanediol, decanediol, undecanediol, dodecanediol, and tridecanediol, in particular pentanediol, hexanediol, heptanediol, octanediol, nonanediol and decanediol.
4. Cosmetic or pharmaceutical composition or homecare product according to clause 2 or clause 3, wherein the linear alkanediol or the first linear alkanediol is selected from the group consisting of pentanediol, heptanediol, and nonanediol, in particular heptanediol.
5. Cosmetic or pharmaceutical composition or homecare product according to any one of clauses 2 to 4, wherein the second linear alkanediol is selected from the group consisting of pentanediol, hexanediol, heptanediol, octanediol, nonanediol, decanediol, undecanediol, dodecanediol, and tridecanediol.
6. Cosmetic or pharmaceutical composition according to any one of clauses 2 to 5, wherein the linear alkanediol, the first linear alkanediol or the second linear alkanediol is a (x,x+1) constitutional isomer, wherein x stands for the number of the carbon atom in the alkanediol chain, to which the OH groups of the alkanediol are chemically bonded, in particular a 1,2-alkanediol, a 2,3-alkanediol, a 3,4-alkanediol, or mixtures thereof.
7. Cosmetic or pharmaceutical composition or homecare product according to any one of clauses 2 to 6, wherein the linear alkanediol or the first linear alkanediol, is selected from the group consisting of:
   1,2-pentanediol, 2,3-pentanediol, 1,2-hexanediol, 2,3-hexanediol,
   1,2-heptanediol, 2,3-heptanediol, 1,2-octanediol, 2,3-octanediol,
   1,2-nonanediol, 2,3-nonanediol, 1,2-decanediol, 2,3-decanediol,
   1,2-undecanediol, 2,3-undecanediol, 1,2-dodecanediol, 2,3-dodecanediol,
   1,2-tridecanediol, 2,3-tridecanediol, and mixtures thereof,
   preferably wherein the first linear alkanediol is selected form the group consisting of 1,2-pentanediol, 2,3-pentanediol, 1,2-hexanediol, 2,3-hexanediol, 1,2-heptanediol, 2,3-heptanediol, 2,3-octanediol, 2,3-nonanediol and mixtures thereof; and/or
   wherein the second linear alkanediol is selected from the group consisting of:
      1,2-pentanediol, 2,3-pentanediol, 1,2-hexanediol, 2,3-hexanediol,
      1,2-heptanediol, 2,3-heptanediol, 1,2-octanediol, 2,3-octanediol,
      1,2-nonanediol, 2,3-nonanediol, 1,2-decanediol, 2,3-decanediol,
      1,2-undecanediol, 2,3-undecanediol, 1,2-dodecanediol, 2,3-dodecanediol, 1,2-tridecanediol, 2,3-tridecanediol, and mixtures thereof,
      preferably wherein the second linear alkanediol is selected from the group consisting of 1,2-1,2-pentanediol, 2,3-pentanediol, 1,2-hexanediol, 2,3-hexanediol, 1,2-heptanediol, 2,3-heptanediol, 2,3-octanediol, 2,3-nonanediol and mixtures thereof.
8. Cosmetic or pharmaceutical composition or homecare product according to clause 7, wherein the mixture comprising at least one first linear alkanediol and one or more second linear alkanediol is selected from the group consisting of:
   a mixture comprising 1,2-hexanediol and 2,3-pentanediol,
   a mixture comprising 1,2-hexanediol and 2,3-hexanediol;
   a mixture comprising 1,2-heptanediol and 2,3-heptanediol;
   a mixture comprising 1,2-octanediol and 2,3-octanediol;
   a mixture comprising 1,2-nonanediol and 2,3-nonanediol;
   a mixture comprising 1,2-decanediol and 2,3-decanediol;
   a mixture comprising 1,2-undecanediol and 2,3-undecanediol;
   a mixture comprising 1,2-dodecanediol and 2,3-dodecanediol; and
   a mixture comprising 1,2-tridecanediol and 2,3-tridecanediol.
9. Cosmetic or pharmaceutical composition or homecare product according to clause 7, wherein the mixture comprising at least one first linear alkanediol and one or more second linear alkanediol is selected from the group consisting of:
   a mixture comprising 1,2-hexanediol and 2,3-octanediol;
   a mixture comprising 1,2-octanediol and 2,3-hexanediol; and
   a mixture comprising 1,2-octanediol and 1,2-heptanediol.
10. Cosmetic or pharmaceutical composition or homecare product according to clause 7, wherein the mixture comprising at least one first linear alkanediol and one or more second linear alkanediol is selected from the group consisting of:
   a mixture comprising 1,2-pentanediol and 2,3-hexanediol;
   a mixture comprising 1,2-pentanediol and 1,2-heptanediol;
   a mixture comprising 1,2-pentanediol and 2,3-heptanediol;
   a mixture comprising 1,2-pentanediol and 2,3-octanediol;
   a mixture comprising 1,2-pentanediol and 1,2-nonanediol; and
   a mixture comprising 1,2-pentanediol and 2,3-nonanediol.
11. Cosmetic or pharmaceutical composition or homecare product according to clause 7, wherein the mixture comprising at least one first linear alkanediol and one or more second linear alkanediol is selected from the group consisting of:
   a mixture comprising 1,2-heptanediol and 1,2-octanediol;
   a mixture comprising 1,2-heptanediol and 2,3-octanediol;
   a mixture comprising 1,2-heptanediol and 1,2-nonanediol; and
   a mixture comprising 1,2-heptanediol and 2,3-nonanediol.
12. Cosmetic or pharmaceutical composition or homecare product according to clause 7, wherein the linear alkanediol is selected from the group consisting of 2,3-pentanediol, 2,3-hexanediol, 2,3-heptanediol, 2,3-octanediol, 2,3-nonanediol, 2,3-decanediol, 2,3-undecanediol, 2,3-dodecanediol, 2,3-tridecanediol and mixtures thereof, preferably wherein the linear alkanediol is selected from the group consisting of 2,3-pentanediol, 2,3-hexanediol, 2,3-heptanediol, 2,3-octanediol, 2,3-nonanediola and mixtures thereof.
13. Cosmetic or pharmaceutical composition or homecare product according to any one of clauses 2 to 12, wherein the linear alkanediol or the first linear alkanediol or the second linear alkanediol is an alkanediol having a maximum water solubility of less than or equal to 10 % by weight, in particular a maximum water solubility of less than or equal to 5 % by weight and more than or equal to 1.4 % by weight, preferably an alkanediol selected from the group consisting of 1,2-heptanediol, 2,3-heptanediol, 2,3-octanediol, and mixtures thereof.
14. Cosmetic or pharmaceutical composition or homecare product according to any one of clauses 1 to 13,
   - wherein the mixture comprising 1,2-heptanediol and 2,3-heptanediol comprises the 1,2-heptanediol and the 2,3-heptanediol in a ratio in a range of 50 : 50 to 99.9 : 0.1, preferred in a ratio in a range of 75 : 25 to 99 : 1, more preferred in a ratio in a range of 80 : 20 to 98 : 2, still more preferred in a ratio in a range of 90 : 10 to 95 : 5; or
   - wherein the mixture comprising at least one first linear alkanediol and one or more second linear alkanediol comprises the first linear alkanediol and the second alkanediol in a ratio in a range of 50 : 50 to 99.9 : 0.1, preferred in a ratio in a range of 75 : 25 to 99 : 1, more preferred in a ratio in a range of 80 : 20 to 98 : 2,still more preferred in a ratio in a range of 90 : 10 to 95 : 5.
15. Cosmetic or pharmaceutical composition or homecare product according to any one of clauses 1 to 14, wherein the at least one solid UV filter is selected from the group consisting of:
   - 1-(4-tert-Butylphenyl)-3-(4-methoxyphenyl)-propane-1,3-dione (INCI: Butyl Methoxydibenzoylmethane; Avobenzon),
   - Sodium salt of 2,2'-bis(1,4-phenylene)-1H-benzimidazole-4,6-disulfonic acid (INCI: Disodium Phenyl Dibenzimidazole Tetrasulfonate),
   - 2-Hydroxy-4-methoxybenzophenone (INCI: Benzophenone-3),
   - 2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol),
   - Isopentyl-4-methoxycinnamate (INCI: Isoamyl p-Methoxycinnamate; Amiloxate),
   - 2-Phenylbenzimidazole-5-sulfonic acid and its potassium, sodium and triethanolamine salts (INCI: Phenylbenzimidazole Sulfonic Acid),
   - 3-(4-Methylbenzylidene)-d1 camphor (INCI: 4-Methylbenzylidene Camphor),
   - 1,3,5-Triazine-2,4,6-tris[1,1'-biphenyl]-4-yl (INCI: Tris-biphenyl Triazine, incl. Nanomaterial),
   - 2-Hydroxy-4-methoxybenzophenone- 5-sulfonic acid and its sodium salt (INCI: Benzophenone-4/Benzophenone-5), and/or
   - Zinc Oxide (INCI: Zinc Oxide),
   - Benzoic acid, 2-[-4-(diethylamino)-2-hydroxybenzoyl]-hexylester (INCI: Diethylamino Hydroxybenzoyl Hexyl Benzoate),
   - 2,4,6-Trianilino-(p-carbo-2'-ethylhexyl-1'-oxy)-1,3,5-triazine (INCI: Ethylhexyl Triazone),
   - alpha-(2-Oxoborn-3-ylidene)-toluene-4-sulphonic acid and its salts (INCI: Benzylidene Camphor Sulfonic Acid),
   - Polymer of N-{(2 and 4)-[(2-oxoborn-3-ylidene)-methyl]-benzyl}-acrylamide (INCI: Polyacrylamido Methyl Benzylidene Camphor),
   - 3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid),
   - Ethoxylated Ethyl-4-aminobenzoate (INCI: PEG-25 PABA),
   - Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone),
   - Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane),
   - 3-Benzylidene camphor (INCI: 3-Benzylidene Camphor), and/or
   - Phenylene-bis-diphenyltriazine (Triasorb B),
   - Neo Heliopan^{®} Flat, and/or
   - Titanium dioxide (INCI: Titanium Dioxide),
   and mixtures of two or more of the aforesaid UV filters.
16. Cosmetic or pharmaceutical composition or homecare product according to any one of clauses 1 to 15, wherein the at least one active substance for a cosmetic or pharmaceutical composition or homecare product and/or additive is selected from the group consisting of agents against ageing of the skin, anti-microbial agents, antioxidants, chelating agents, emulsifiers, preservatives, green and synthetic polymers, skin-cooling agents, rheology additives, oils, fragrances or perfume oils, polyols, and mixtures of two or more of the aforementioned substances.
17. Cosmetic or pharmaceutical composition or homecare product according to any one of clauses 1 to 16, further comprising at least one additional UVA filter, and/or further comprising at least one additional UVB filter, and/or further comprising at least one additional broadband filter, and/or further comprising at least one pigment, or mixtures thereof.
18. Cosmetic or pharmaceutical composition or homecare product according to any one of clauses 1 to 17, further comprising at least one oil component selected from the group consisting of plant oils, hydrocarbons, fatty alcohols, fatty acid esters, and mixtures of two or more of the aforesaid liquid oil components.
19. Cosmetic or pharmaceutical composition or homecare product according to any one of clauses 1 to 18, comprising the 1,2-heptanediol or the 2,3-heptanediol or the mixture comprising 1,2-heptanediol and 2,3-heptanediol or the at least one linear alkanediol or the mixture comprising at least one first linear alkanediol and one or more second linear alkanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, most preferred in an amount of 0.5 to 1.0 % by weight, based on the total weight of the composition or homecare product.
20. Cosmetic or pharmaceutical composition or homecare product according to any one of clauses 1 to 19, comprising
   - the 2,3-heptanediol or the 2,3-heptanediol of the mixture comprising 1,2-heptanediol and 2,3-heptanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, most preferred 0.5 to 1.0 % by weight, based on the total weight of the composition or homecare product; preferably in an amount of 0.001 to 0.5 % by weight, more preferably in an amount of 0.005 to 0.1 % by weight and most preferably in an amount of 0.01 to 0.075 % by weight, based on the total weight of the composition or homecare product; or
   - the 2,3-alkanediol as linear alkanediol or of the mixture comprising at least one first linear alkanediol and one or more second linear alkanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, most preferred in an amount of 0.5 to 1.0 % by weight, based on the total weight of the composition or homecare product;
   preferably in an amount of 0.001 to 0.1 % by weight, more preferably in an amount of 0.005 to 0.1 % by weight and most preferably in an amount of 0.01 to 0.075 % by weight, based on the total weight of the composition or homecare product.
21. Cosmetic or pharmaceutical composition according to any one of clauses 1 to 20, comprising the at least one solid UV filter and optionally further UV filters in an amount of 0.1 to 50.0 % by weight, particularly in an amount of 0.5 to 45.0 % by weight, most particularly in an amount of 1.0 to 40 % by weight, based on the total weight of the composition or homecare product.
22. Cosmetic or pharmaceutical composition according to any one of clauses 1 to 21 in the form of
   - a dispersion comprising the at least one oil component in an amount of ≥ 1% by weight, in particular in an amount of ≥ 3 % by weight, in particular wherein the cosmetic or pharmaceutical composition is in the form of an emulsion, in particular a O/W emulsion, a W/O emulsion, a multiple emulsion, a hydrodispersion gel, a balm, a multiple emulsion of the water-in-oil type (W/O/W) or of the oil-in-water type (O/W/O), PIT emulsion, Pickering emulsion a micro-emulsion, a liquid, a lotion, a suspension, a milk, an ointment, a paste, a gel, a cream based, an oil based or a liposome-based formulation;
   - a water free formulation; or
   - an aqueous or aqueous/alcoholic, in particular aqueous/ethanolic, or an aqueous/glycolic solution, comprising the alcohol or glycol in an amount of 0.1 % to ≤ 50 % by weight, based on the total weight of the solution.
23. Cosmetic or pharmaceutical composition according to any one of clauses 1 to 22 as a cosmetic, for personal care, in particular for skin, hair, scalp or nail care, as a pharmaceutical, or for animal care.
24. Use of 1,2-heptanediol or 2,3-heptanediol or a mixture comprising 1,2-heptanediol and 2,3-heptanediol or at least one linear alkanediol or a mixture comprising at least one first linear alkanediol and one or more second linear alkanediol as defined in any one of clauses 2 to 14 for improving the solubility of a solid UV filter, preferably a UV filter according to clause 15, in a cosmetic or pharmaceutical composition or homecare product; and/or for improving the availability of a solid UV filter, preferably a UV filter according to clause 15, in a cosmetic or pharmaceutical composition on skin upon application of the cosmetic of pharmaceutical composition.
25. Use according to clause 24, wherein the linear alkanediol or the first linear alkanediol is selected from the group consisting of
   1,2-pentanediol, 2,3-pentanediol, 1,2-hexanediol, 2,3-hexanediol,
   1,2-heptanediol, 2,3-heptanediol, 1,2-octanediol, 2,3-octanediol,
   1,2-nonanediol, 2,3-nonanediol, 1,2-decanediol, 2,3-decanediol,
   1,2-undecanediol, 2,3-undecanediol, 1,2-dodecanediol, 2,3-dodecanediol, 1,2-tridecanediol, 2,3-tridecanediol, and mixtures thereof,
   preferably wherein the first linear alkanediol is selected from the group consisting of 1,2-pentanediol, 2,3-pentanediol, 1,2-hexanediol, 2,3-hexanediol, 1,2-heptanediol, 2,3-heptanediol, 2,3-octanediol, 2,3-nonanediol and mixtures thereof;
      and/or
   wherein the second linear alkanediol is selected from the group consisting of
   1,2-pentanediol, 2,3-pentanediol, 1,2-hexanediol, 2,3-hexanediol,
   1,2-heptanediol, 2,3-heptanediol, 1,2-octanediol, 2,3-octanediol,
   1,2-nonanediol, 2,3-nonanediol, 1,2-decanediol, 2,3-decanediol,
   1,2-undecanediol, 2,3-undecanediol, 1,2-dodecanediol, 2,3-dodecanediol,
   1,2-tridecanediol, 2,3-tridecanediol, and mixtures thereof,
   preferably wherein the second linear alkanediol is selected from the group consisting of 1,2-pentanediol, 2,3-pentanediol, 1,2-hexanediol, 2,3-hexanediol, 1,2-heptanediol, 2,3-heptanediol, 2,3-octanediol, 2,3-nonanediol and mixtures thereof.
26. Use according to clause 24, wherein the mixture comprising at least one first linear alkanediol and one or more second linear alkanediol is selected from the group consisting of
   a mixture comprising 1,2-pentanediol and 2,3-pentanediol;
   a mixture comprising 1,2-hexanediol and 2,3-hexanediol;
   a mixture comprising 1,2-heptanediol and 2,3-heptanediol;
   a mixture comprising 1,2-octanediol and 2,3-octanediol;
   a mixture comprising 1,2-nonanediol and 2,3-nonanediol;
   a mixture comprising 1,2-decanediol and 2,3-decanediol;
   a mixture comprising 1,2-undecanediol and 2,3-undecanediol;
   a mixture comprising 1,2-dodecanediol and 2,3-dodecanediol; and
   a mixture comprising 1,2- tridecanediol and 2,3-tridecanediol.
27. Use according to clause 24, wherein the mixture of comprising least one first linear alkanediol and one or more second linear alkanediol is selected from the group consisting of
   a mixture comprising 1,2-hexanediol and 2,3-octanediol;
   a mixture comprising 1,2-octanediol and 2,3-hexanediol; and
   a mixture comprising 1,2-octanediol and 2,3-heptanediol.
28. Use according to clause 24, wherein the mixture comprising at least one first linear alkanediol and one or more second linear alkanediol is selected from the group consisting of
   a mixture comprising 1,2-pentanediol and 2,3-hexanediol;
   a mixture comprising 1,2-pentanediol and 1,2-heptanediol;
   a mixture comprising 1,2-pentanediol and 2,3-heptanediol;
   a mixture comprising 1,2-pentanediol and 2,3-octanediol;
   a mixture comprising 1,2-pentanediol and 1,2-nonanediol; and
   a mixture comprising 1,2-pentanediol and 2,3-nonanediol.
29. Use according to clause 24, wherein the mixture comprising at least one first linear alkanediol and one or more second linear alkanediol is selected from the group consisting of
   a mixture comprising 1,2-heptanediol and 1,2-octanediol;
   a mixture comprising 1,2-heptanediol and 2,3-octanediol;
   a mixture comprising 1,2-heptanediol and 1,2-nonanediol; and
   a mixture comprising 1,2-heptanediol and 2,3-nonanediol.
30. Use according to clause 24, wherein the linear alkanediol is selected from the group consisting of 2,3-pentanediol, 2,3-hexanediol, 2,3-heptanediol, 2,3-octanediol, 2,3-nonanediol, 2,3-decanediol, 2,3-undecanediol, 2,3-dodecanediol, 2,3-tridecanediol and mixtures thereof, preferably wherein the linear alkanediol is selected from the group consisting of 2,3-pentanediol, 2,3-hexanediol, 2,3-heptanediol, 2,3-octanediol, 2,3-nonanediol and mixtures thereof.

The present invention shall now be described in detail with reference to the following examples, which are merely illustrative of the present invention, such that the content of the present invention is not limited by or to the following examples.

### Examples

### Example 1: Solubility of solid UV filters in 1,2-heptanediol, 1,2-pentanediol, 1,2-hexanediol or 1,2-octanediol

The following tests were performed to show the influence of different 1,2-alkanediols on solubility of solid UV filters (specified by their INCl; International Nomenclature of Cosmetic Ingredients) in neat alkanediol solutions:
(a) Bis-Ethylhexyloxyphenol Methoxyphenyl Triazine (Bemotrizinol)
(b) Butyl Methoxydibenzoylmethane (Avobenzon)
(c) 4-Methylbenzylidene Camphor
(d) Ethylhexyl Triazone
(e) Diethylamino Hydroxbenzoyl Hexyl Benzoate

The visual appearance of the sample solutions was evaluated according to the following scale:

| Description | Coding |
|---|---|
| Slight Recrystallization | R |
| Recrystallization | R+ |
| Turbid | T |
| Strong recrystallization | R++ |
| Clear | C |

### Example 1.1: Solubility of Bis-Ethylhexyloxyphenol Methoxyphenyl Triazine in 1,2-pentanediol, 1,2-hexanediol, 1,2-heptanediol, 2,3-heptanediol or 1,2-octanediol

**Table 1:**

| **Ingredients (w/w %)** | **Sample 3** | **Sample 4** | **Sample 5** | **Sample 6** | **Sample 7** |
|---|---|---|---|---|---|
| Bis-Ethylhexyloxy-phenol Methoxyphenyl Triazine | 1.0 | 1.0 | 1.0 | 1.0 | 1.0 |
| 1,2-pentanediol | 99.0 | - | - | - | - |
| 1,2-hexanediol | - | 99.0 | - | - | - |
| 1,2-heptanediol | - | - | 99.0 | - | - |
| 2,3-heptanediol | - | - | - | 99.0 | - |
| 1,2-octanediol | - | - | - | - | 99.0 |
| **Sum:** | 100.0 | | | | |
| Appearance after 24h storage at 5 °C | R+ | R+; T | C | C | R++ |

Production method: Bis-Ethylhexyloxyphenol Methoxyphenyl Triazine was dissolved in the corresponding 1,2-alkanediol by heating up to 80 °C. The solutions were stirred by using a magnetic stirrer until they achieve ambient temperature.

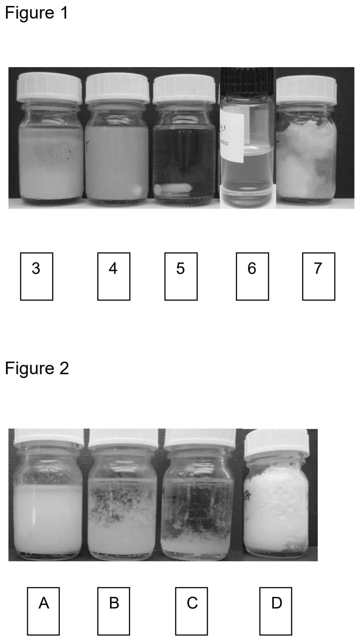

The images of the solutions 3, 4, 5, 6 and 7 are shown in Figure 1.

After two weeks storage at ambient temperature, it could be observed, that with sample 5 (containing 1,2-heptanediol) and sample 6 (containing 2,3-heptanediol) a clear solution was obtained, whereas samples 3 (1,2-pentanediol), 4 (1,2-hexanediol) and 7 (1,2-octanediol) crystallized out and were turbid.

### Example 1.2: Solubility of Butyl Methoxydibenzoylmethane in 1,2-pentanediol, 1,2-hexanediol, 1,2-heptanediol or 1,2-octanediol

**Table 2:**

| **Ingredients (w/w %)** | **Sample A** | **Sample B** | **Sample C** | **Sample D** |
|---|---|---|---|---|
| 1,2-pentanediol | 97.5 | - | - | - |
| 1,2-hexanediol | - | 97.5 | - | - |
| 1,2-heptanediol | - | - | 97.5 | - |
| 1,2-octanediol | - | - | - | 97.5 |
| Butyl Methoxydibenzoylmethane | 2.5 | 2.5 | 2.5 | 2.5 |
| **Sum:** | 100.0 | | | |
| Appearance | R++ | R+ | R | R++ |

Production method: Butyl Methoxydibenzoylmethane was dissolved in the corresponding 1,2-alkanediol by heating up to 80 °C. The solutions were stirred by using a magnetic stirrer until they achieve ambient temperature.

The images of the solutions A, B, C and D are shown in Figure 2.

After two weeks storage at ambient temperature, it could be observed that solution C (containing 1,2-heptanediol) showed the lowest recrystallization versus solutions A (containing 1,2-pentanediol), B (containing 1,2-hexanediol) and blend D (containing 1,2-octanediol). Additionally, the filtration residue was determined. Solutions A, B and C were filtrated and the amount of recrystallized Butyl Methoxydibenzoylmethane was gravimetric determined. Blend D could not be filtrated due to its solid state.

**Table 3:**

| Solution | Amount residue [g] |
|---|---|
| A | 0.664 |
| B | 0.634 |
| C | 0.385 |

Solution C (containing 1,2-heptanediol) showed the lowest amount of recrystallization (0.385g) in comparison to solutions A (0.664g) and solution B (0.634g).

### Example 1.3: Solubility of 4-Methylbenzylidene Camphor in 1,2-pentanediol, 1,2-hexanediol, 1,2-heptanediol or 1,2-octanediol

**Table 4:**

| **Ingredients (w/w %)** | **Sample E** | **Sample F** | **Sample G** | **Sample H** |
|---|---|---|---|---|
| 1,2-pentanediol | 85.0 | - | - | - |
| 1,2-hexanediol | - | 85.0 | - | - |
| 1,2-heptanediol | - | - | 85.0 | - |
| 1,2-octanediol | - | - | - | 85.0 |
| 4-Methylbenzylidene Camphor | 15.0 | 15.0 | 15.0 | 15.0 |
| **Sum:** | 100.0 | | | |
| Appearance | R++ | R+ | R | R++ |

Production method: 4-Methylbenzylidene Camphor was dissolved in the corresponding 1,2-alkanediol by heating up to 80 °C. The solutions were stirred by using a magnetic stirrer until they achieve ambient temperature.

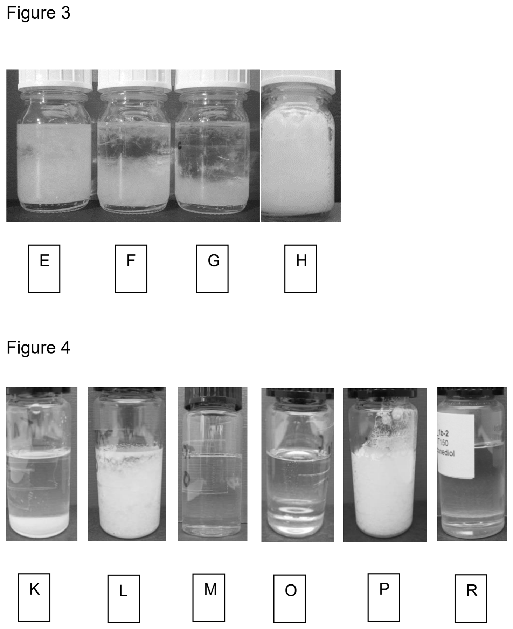

The images of the solutions E, F, G and H are shown in Figure 3.

After two weeks storage at ambient temperature it could be observed that solution G (containing 1,2-heptanediol) showed the lowest recrystallization versus solutions E (containing 1,2-pentanediol), F (containing 1,2-hexanediol) and blend H (containing 1,2-octanediol). Additionally, the filtration residue was determined. Solutions E, F and G were filtrated and the amount of recrystallized 4-Methylbenzylidene Camphor was gravimetric determined. Blend H could not be filtrated due to its solid state.

**Table 5:**

| Solution | Amount residue (g) |
|---|---|
| E | 2.036 |
| F | 1.499 |
| G | 1.202 |

Solution G (containing 1,2-heptanediol) showed the lowest amount of recrystallization (1,202 g) in comparison to solutions E (2,036 g) and solution F (1,499 g).

### Example 1.4: Solubility of Ethylhexyl Triazone in 1,2-pentanediol, 1,2-hexanediol, 1,2-heptanediol, 2,3-heptanediol or 1,2-octanediol

**Table 6:**

| **Ingredients** | **Sample K** | **Sample L** | **Sample M** | **Sample O** | **Sample P** | **Sample R** |
|---|---|---|---|---|---|---|
| 1,2-pentanediol | 92.5 | - | - | - | - | - |
| 1,2-hexanediol | - | 92.5 | - | - | - | - |
| 1,2-heptanediol | - | - | 92.5 | - | - | - |
| 2,3-heptanediol | - | - | - | 92.5 | - | - |
| 1,2-octanediol | - | - | - | - | 92.5 | - |
| 2,3-octanediol | - | - | - | - | - | 92.5 |
| Ethylhexyl Triazone | 7.5 | 7.5 | 7.5 | 7.5 | 7.5 | 7.5 |
| **Sum:** | 100.0 | | | | | |
| Appearance | R | R++ | C | C | R++ | C |

Production method: Ethylhexyl triazone was dissolved in the corresponding 1,2-alkanediol by heating up to 80 °C. The solutions were stirred by using a magnetic stirrer until they achieve ambient temperature.

The images of the solutions K, L, M, O, P and R are shown in Figure 4.

After two weeks storage at ambient temperature it could be observed that solution M (containing 1,2-heptanediol), solution O (containing 2,3-heptanediol) and solution R (containing 2,3-octanediol) showed no recrystallization in comparison to solution K (containing 1,2-pentanediol) and solution L (containing 1,2-hexanediol) which are visible recrystallized. Blend P (containing 1,2-octanediol) was totally recrystallized.

### Example 1.5: Solubility of Diethylamino Hydroxbenzoyl Hexyl Benzoate in 1,2-heptanediol, 1,2-pentanediol, 1,2-hexanediol or 1,2-octanediol

**Table 7:**

| **Ingredients (w/w %)** | **Sample U** | **Sample W** | **Sample X** | **Sample Z** |
|---|---|---|---|---|
| 1,2-pentanediol | 90.0 | - | - | - |
| 1,2-hexanediol | - | 90.0 | - | - |
| 1,2-heptanediol | - | - | 90.0 | - |
| 1,2-octanediol | - | - | - | 90.0 |
| Diethylamino Hydroxybenzoyl Hexyl Benzoate | 10.0 | 10.0 | 10.0 | 10.0 |
| **Sum:** | 100.0 | | | |
| Appearance | R+ | R | C | R++ |

Production method: Diethylamino Hydroxbenzoyl Hexyl Benzoate was dissolved in the corresponding 1,2-alkanediol by heating up to 80 °C. The solutions were stirred by using a magnetic stirrer until they achieve ambient temperature.

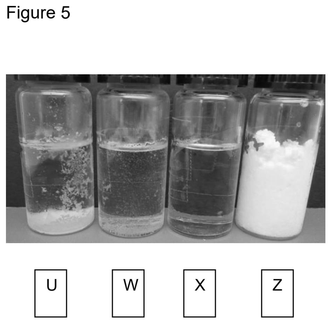

The images of the solutions U, W, X and Z are shown in Figure 5.

After two weeks storage at ambient temperature, it could be observed that solution X (containing 1,2-heptanediol) result in a clear solution without recrystallization in comparison to solution U (containing 1,2-pentanediol), solution W (containing 1,2-hexanediol) and solution Z (containing 1,2-octanediol).

### Example 1.6: Solubility of Bis-Ethylhexyloxyphenol Methoxyphenyl Triazine (Neo Helioplan BMT) in 1,2-hetanediol, 2,3-heptanediol or a mixture of 1,2-heptanediol and 2,3-heptanediol

**Table 8:**

| **Ingredients (w/w %)** | **01** | **02** | **03** | **04** | **05** |
|---|---|---|---|---|---|
| Neo Heliopan BMT (INCI: Bis-Ethylhexyloxyphenol Methoxyphenyl Triazine) | 1.0 | 1.0 | 1.0 | 1.0 | 1.0 |
| 1,2-heptanediol | 99.0 | - | 89.1 | 69.3 | 49.5 |
| 2,3-heptanediol | - | 99.0 | 9.9 | 29.7 | 49.5 |
| SUM | 100.0 | | | | |
| Appearance | R+ | R+ | R | R | C |

Production method: Bis-Ethylhexyloxyphenol Methoxyphenyl Triazine was dissolved in 1,2-heptanediol, 2,3-heptanediol or blends thereof by heating the solution up to 80 °C. The solutions were stirred by using a magnetic stirrer until ambient temperature is achieved.

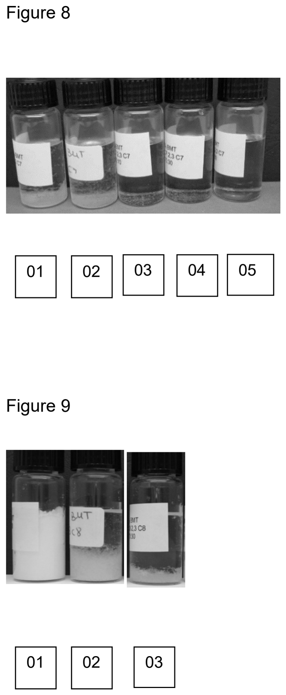

The images of the solutions 01, 02, 03, 04 and 05 are shown in Figure 8.

After one day storage at ambient temperature solution 05 (containing a 1,2-heptanediol and 2,3-heptanediol blend (1 : 1)) appeared as a clear solution without recrystallization. Solutions 03 and 04, containing mixtures of 1,2- and 2,3 heptanediol in the ratio 9 : 1 (solution 03) and in the ratio 7 : 3 (solution 04) showed only slight recrystallization. Comparative solution samples 01 (containing 1,2-heptanediol alone) and solution 02 (containing 2,3-heptanediol alone) showed recrystallization. The blend of 1,2-heptanediol and 2,3-heptanediol leads to a clearly improved solubility of the UV filter in comparison to 1,2-heptanediol and 2,3-heptanediol alone.

### Example 1.7: Solubility of Bis-Ethylhexyloxyphenol Methoxyphenyl Triazine (Neo Helioplan BMT) in 1,2-octanediol, 2,3-octanediol or a mixture of 1,2-octanediol and 2,3-octanediol

**Table 9:**

| Ingredients (w/w %) | 06 | 07 | 08 |
|---|---|---|---|
| Neo Heliopan BMT (INCl: Bis-Ethylhexyloxyphenol Methoxyphenyl Triazine) | 1.0 | 1.0 | 1.0 |
| 1,2-octanediol | 99.0 | - | 69.3 |
| 2,3-octanediol | - | 99.0 | 29.7 |
| SUM | 100.0 | | |
| Appearance | S | R++ | R |

Production method: Bis-Ethylhexyloxyphenol Methoxyphenyl Triazine was dissolved in 1,2-octanediol, 2,3-octanediol or blends thereof by heating the solution up to 80 °C. The solutions were stirred by using a magnetic stirrer until they achieve ambient temperature.

The images of the solutions 06, 07 and 08 are shown in Figure 9.

After one day storage at ambient temperature comparative solution 06 (containing 1,2-octanediol) is completely crystallized (solid) and solution 07 (containing 2,3-octanediol) showed strong recrystallization. Sample 08 containing a blend of 1,2-octanediol and 2,3-octanediol (ratio 70 : 30) is not completely clear but it showed the lowest grade of recrystallization. A synergistic effect referring to solubility improvement could be observed by using a blend of 1,2-octanediol and 2,3-octanediol in comparison of 1,2-octanediol and 2,3-octanediol each alone.

### Example 2: Solubility of solid UV filters in emulsions

The following tests were performed to show the influence of different 1,2-alkanediols on solubility of solid UV filters in emulsions:

(a) 5 % Bis-Ethylhexyloxyphenol Methoxyphenyl Triazine; or (b) 3 % Ethylhexyl Triazone were incorporated into O/W emulsions comprising an alkanediol as specified in the examples below.

The solubility was evaluated by microscope of the emulsion with regard to recrystallization after two weeks storage at room temperature.

### Example 2.1: 5 % Bis-Ethylhexyloxyphenol Methoxyphenyl Triazine in emulsion

(a) 01_A Placebo without diol
(b) 01_B 1.0 % 1,2-pentanediol
(c) 01_C 1.0 % 1,2-hexanediol
(d) 01_D 1.0 % 1,2-heptanediol
(e) 01_E 1.0 % 1,2-octanediol

**Table 10: Emulsion formulation**

| | **Ingredients (w/w %)** | **INCI** | **01_A placebo without 1,2-alkanediol** | **01_B; 01_C; 01_D; 01_E plus 1.0 % 1,2-alkanediol** |
|---|---|---|---|---|
| A. | Dracorin^{®} CE | Glyceryl Stearate Citrate | 1.0 | 1.0 |
| | Lanette O ex BASF | Cetearyl Alcohol | 1.0 | 1.0 |
| | Cutina GMS V ex BASF | Glyceryl Stearate | 0.5 | 0.5 |
| | SymMollient^{®} PDCC | Propanediol Dicaprylate/Caprate | 2.0 | 2.0 |
| | SymMollient^{®} S green | Cetearyl Nonanoate | 2.0 | 2.0 |
| | Avocado Oil | Persea Gratissima (Avocado) Oil | 3.0 | 3.0 |
| | Plantoil Triglyceride | Caprylic/Capric/Triglyceride | 8.0 | 8.0 |
| | Neo Heliopan^{®} BMT | Bis-Ethylhexyloxphenol Methoxyphenyl Triazine | 5.0 | 5.0 |
| B. | Keltrol CG ex Lubrizol | Xanthan Gum | 0.10 | 0.10 |
| | Carbopol Ultrez 10 ex Lubrizol | Carbomer | 0.10 | 0.10 |
| C. | Water, demin. | Water (Aqua) | 75.0 | 74.0 |
| | Custom DMDM ex Custom Ingredients | DMDM Hydantoin | 0.1 | 0.1 |
| | 1,2-Alkanediol (1,2-pentanediol, or 1,2-hexanediol, or 1,2-heptanediol, or 1,2-octanediol) | (Pentylene glycol, 1,2-hexanediol, 1,2-heptanediol, Capryly Glycol) | - | 1.0 |
| | Glycerin 99,5 % | Glycerin | 2.0 | 2.0 |
| D. | NaOH (10 % sol) | Water (aqua), sodium hydroxide | 0.2 | 0.2 |
| **Sum:** | 100.0 | | | |

Production method (200 g were prepared): phases A and C were separately heated up to 80 °C. Phase B was added to A and dispersed with magnetic stirrer (1min). Phase C was added to the mixture of A plus B and emulsified with Ultra Turrax (3 min/6000 rpm). Then, the mixture was allowed to cool down by using a vane stirrer (10 min 150 rpm/10 min 100 rpm). The resulting mixture was neutralized with phase D during stirring and pH adjustment to pH 5.8.

After two weeks storage at ambient temperature, the solubility of the samples was evaluated by microscope with regard to recrystallization.

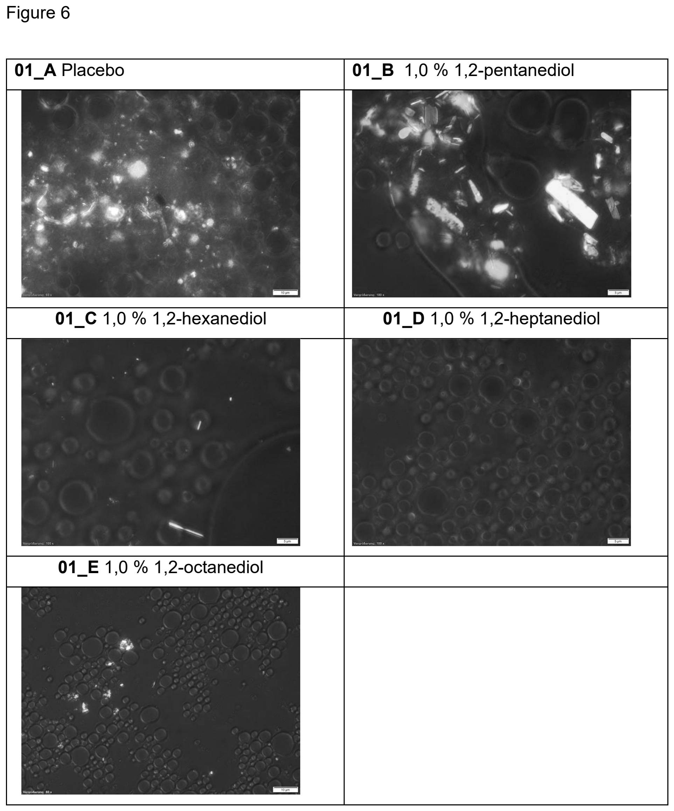

The respective microscopic images of the samples after two weeks storage are shown in Figure 6.

Microscopic images: Olympus IX-70; Image magnification: 60-fold. For crystals: Bright field.

It could be observed, that in emulsion 01_D (containing 1,2-heptanediol) were no crystals, whereas emulsions 01_A (without alkanediol), 01_B (containing 1,2-pentanediol), 01_C (containing 1,2-hexanediol) and 01_E (containing 1,2-octanediol) showed recrystallization.

### Example 2.2: 3 % Ethylhexyl Triazone in emulsion

(a) 01_A Placebo without diol
(b) 01_B 1.0 % 1,2-pentanediol
(c) 01_C 1.0 % 1,2-hexanediol
(d) 01_D 1.0 % 1,2-heptanediol
(e) 01_E 1.0 % 1,2-octanediol

**Table 11: Emulsion formulation**

| | **Ingredients (w/w %)** | **INCI** | **02_A placebo without 1,2-alkanediol** | **02_B; 02_C; 02_D; 02_E plus 1.0 % 1,2-alkanediol** |
|---|---|---|---|---|
| A. | Dracorin^{®} CE | Glyceryl Stearate Citrate | 1.0 | 1.0 |
| | Lanette O ex BASF | Cetearyl Alcohol | 1.0 | 1.0 |
| | Cutina GMS V ex BASF | Glyceryl Stearate | 0.5 | 0.5 |
| | SymMollient^{®} PDCC | Propanediol Dicaprylate/Caprate | 2.0 | 2.0 |
| | SymMollient^{®} S green | Cetearyl Nonanoate | 2.0 | 2.0 |
| | Avocado Oil | Persea Gratissima (Avocado) Oil | 3.0 | 3.0 |
| | Plantoil Triglyceride | Caprylic/Capric/Triglyceride | 8.0 | 8.0 |
| | Uvinul T 150 ex BASF | Ethylhexyl Triazone | 3.0 | 3.0 |
| B. | Keltrol CG ex Lubrizol | Xanthan Gum | 0.10 | 0.10 |
| | Carbopol Ultrez 10 ex Lubrizol | Carbomer | 0.10 | 0.10 |
| C. | Water, demin. | Water (Aqua) | 77.0 | 76.0 |
| | Custom DMDM | MDMD Hydantoin | 0.1 | 0.1 |
| | 1,2-Alkanediol (1,2-pentanediol, or 1,2-hexanediol, or 1,2-heptanediol, or 1,2-octanediol) | (Pentylene glycol, 1,2-hexanediol, 1,2-heptanediol, Capryly Glycol) | - | 1.0 |
| | Glycerin 99,5 % (657319) | Glycerin | 2.0 | 2.0 |
| D. | NaOH (10 % sol) | Water (aqua), sodium hydroxide | 0.2 | 0.2 |
| **Sum:** | | | 100.0 | |

Production method (200 g were prepared): phases A and C were separately heated up to 80 °C. Phase B was added to A and dispersed with magnetic stirrer (1min). Phase C was added to the mixture of A plus B and emulsified with Ultra Turrax (3 min/6000 rpm). Then, the mixture was allowed to cool down by using a vane stirrer (10 min 150 rpm/10 min 100 rpm). The resulting mixture was neutralized with phase D during stirring and pH adjustment to pH 5.8.

After two weeks storage at ambient temperature, the solubility of the samples was evaluated by microscope with regard to recrystallization.

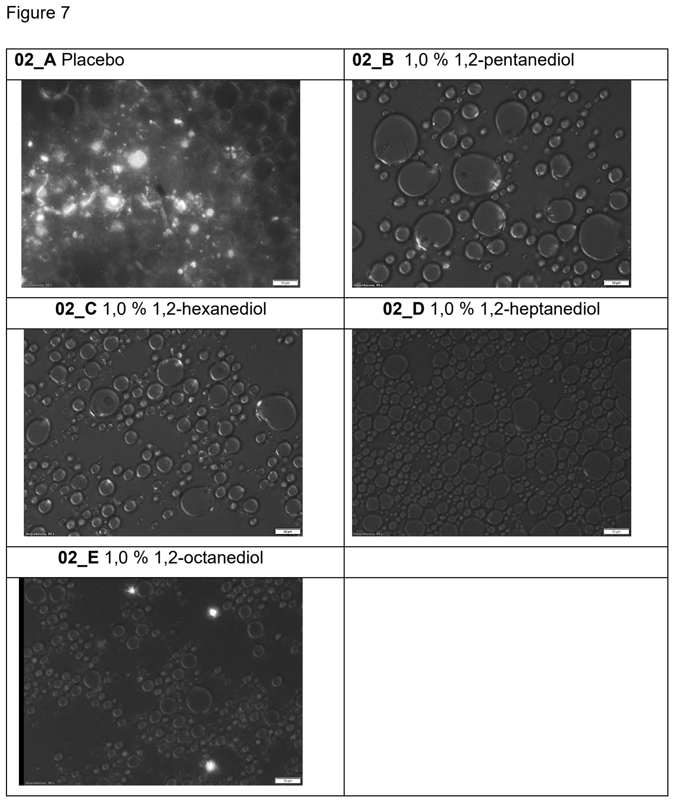

The respective microscopic images of the samples after two weeks storage are shown in Figure 7.

Microscopic images: Olympus IX-70; Image magnification: 60-fold. For crystals: Bright field.

It could be observed, that in emulsion 02_D (containing 1,2-heptanediol) were no crystals, whereas emulsions 02_A (without alkanediol), 02_B (containing 1,2-pentanediol), 02_C (containing 1,2-hexanediol) and 02_E (containing 1,2-octanediol) showed recrystallization.

### Example 3: Solubility of alkanediols in water

For the determination of the water solubility of an alkanediol, water and different concentrations of the respective alkanediol were blended by stirring. The assessment of solubility was carried out at ambient temperature after 24 h storage at 5 °C. The determination of whether the alkanediol has dissolved in water is based entirely on visual observation. An alkanediol compound has dissolved if the mixture is clear and shows no signs of cloudiness or precipitation.

**Table 12:**

| **Tradename/ Chemical name** | **INCI** | **Maximum solubility in water (% by weight)** |
|---|---|---|
| Hydrolite^{®} 5 green | Pentylene Glycol | 50 |
| Hydrolite^{®} 6 | Hexanediol | 50 |
| 1,2-heptanediol | Heptanediol | 2.5 |
| Hydrolite^{®} 8 | Caprylyl Glycol | 0.5 |
| SymDiol^{®} 68 | Hexanediol, Caprylyl Glycol | 1.0 |
| 1,2-nonanediol | Nonanediol | 0.1 |
| SymClariol^{®} | Decylene Glycol | - |
| 1,2-undecanediol | Undecanediol | - |
| 2,3-heptanediol | - | 3.6 |
| 2,3-octanediol | - | 1.5 |
| 2,3-nonanediol | - | 0.3 |
| 2,3-undecanediol | - | - |

### Example 4: Sensory properties of topical formulations comprising an oil component and an alkanediol

For evaluating the sensory behavior, oil components specified in the following Table 13 were blended with 1,2-heptanediol or 2,3-heptanediol or a mixture of 1,2-heptanediol and 2,3-heptanediol or 1,2-octanediol or 2,3-octanediol or a mixture of 1,2-octanediol and 2,3-octanediol (formulation) and assessed by untrained panelists in comparison to the oil component without addition of the specified alkanediols (placebo).

### Test procedure:

- Hands were pre-washed with liquid soap by following a defined procedure;
- 30 µl of corresponding sample was applied on the backside of each hand (randomized);
- After 30 s the spreading behavior of the respective formulation on skin was evaluated versus the respective oil component without 1,2-heptanediol on a scale from 1 to 5 (1 = low spreading; 5 = high spreading);
- The samples were distributed with the fingertips of middle and forefinger with 2 x 5 cycles;
- After 2 minutes, the fatty/greasy skin feeling was evaluated on a scale from 1 to 5 (1 = low fatty/greasy skin feeling; 5 = high fatty/greasy skin feeling);
- Furthermore, the applicants were asked to assess the absorption, i.e. the impression of amount of remaining residue on the skin on a scale from 1 to 5 (1 = low absorption; 5 = high absorption).

**Table 13:**

| | **Oil component** | **alkanediol** | **Spreading** | | | **Fatty/greasy skin feeling** | | | **Absorption/ remaining residue** | | |
|---|---|---|---|---|---|---|---|---|---|---|---|
| | | | **with out** | **with** | **Delta** | **witho ut** | **with** | **Delta** | **witho ut** | **with** | **Delta** |
| Plant Oil | Argan Oil | 1,2-heptanediol | 2 | 4.2 | 2.2 | 4 | 2.4 | 1.6 | 3.8 | 2.8 | 1 |
| Plant Oil | Argan Oil | 2,3-heptanediol | 2 | 3 | 1 | 4 | 2.6 | 1.4 | 3.8 | 2.4 | 1.4 |
| Plant Oil | Argan Oil | 1,2-heptanediol/ 2,3-heptanediol (95:5) | 2 | 3.8 | 1.8 | 4 | 2.6 | 1.4 | 3.8 | 2.4 | 1.4 |
| | | | | | | | | | | | |
| Plant Oil | Argan Oil | 1,2-octanediol | 2 | 3.8 | 1.8 | 4 | 2.6 | 1.4 | 3.8 | 2.6 | 1.2 |
| Plant Oil | Argan Oil | 2,3-octanediol | 2 | 3.4 | 1.4 | 4 | 2.4 | 1.6 | 3.8 | 2.2 | 1.6 |
| Plant Oil | Argan Oil | 1,2-octanediol/ 2,3-octanediol (95:5) | 2 | 4.2 | 2.2 | 4 | 2.2 | 1.8 | 3.8 | 2 | 1.8 |
| | | | | | | | | | | | |
| Plant Oil | Avocado Oil | 1,2-heptanediol | 2.2 | 4.4 | 2.2 | 3.8 | 2.6 | 1.2 | 3.8 | 2.6 | 1.2 |
| Plant Oil | Avocado Oil | 2,3-heptanediol | 2.2 | 3.8 | 1.6 | 3.8 | 2 | 1.8 | 3.8 | 2.4 | 1.4 |
| Plant Oil | Avocado Oil | 1,2-heptanediol/ 2,3-heptanediol (95:5) | 2.2 | 3.6 | 1.4 | 3.8 | 2.2 | 1.6 | 3.8 | 2 | 1.8 |
| | | | | | | | | | | | |
| Plant Oil | Avocado Oil | 1,2-octanediol | 2 | 4 | 2 | 3.8 | 3 | 0.8 | 3.8 | 2.6 | 1.2 |
| Plant Oil | Avocado Oil | 2,3-octanediol | 2 | 3.2 | 1.2 | 3.8 | 2.8 | 1 | 3.8 | 2.4 | 1.4 |
| Plant Oil | Avocado Oil | 1,2-octanediol/ 2,3-octanediol (95:5) | 2 | 3.8 | 1.8 | 3.8 | 2.2 | 1.6 | 3.8 | 2.2 | 1.6 |
| | | | | | | | | | | | |
| Plant Oil | Butyrosperm um Parkii (Shea) Butter | 1,2-heptanediol | 2 | 4 | 2 | 4 | 2 | 2 | 4.2 | 2.7 | 1.5 |
| | | | | | | | | | | | |
| Plant Oil | Cacao butter | 1,2-heptanediol | 1.8 | 3.8 | 2 | 4.2 | 3 | 1.2 | 3.6 | 3.6 | 0 |
| Plant Oil | Cacao butter | 2,3-heptanediol | 1.8 | 3 | 1.2 | 4.2 | 3.4 | 0.8 | 3.6 | 3.2 | 0.4 |
| Plant Oil | Cacao butter | 1,2-heptanediol/ 2,3-heptanediol (95:5) | 1.8 | 4 | 2.2 | 4.2 | 3 | 1.2 | 3.6 | 3 | 0.6 |
| | | | | | | | | | | | |
| Plant Oil | Cacao butter | 1,2-octanediol | 1.8 | 3.8 | 2 | 4.2 | 3.2 | 1 | 3.6 | 3 | 0.6 |
| Plant Oil | Cacao butter | 2,3-octanediol | 1.8 | 3 | 1.2 | 4.2 | 3 | 1.2 | 3.6 | 2.8 | 0.8 |
| Plant Oil | Cacao butter | 1,2-octanediol/ 2,3-octanediol (95:5) | 1.8 | 2.6 | 0.8 | 4.2 | 3.2 | 1 | 3 | 2.6 | 0.4 |
| | | | | | | | | | | | |
| Plant Oil | Caprylic Capric Triglyceride (neutral oil) | 1,2-heptanediol | 1.9 | 3.4 | 1.5 | 3.2 | 2.9 | 0.3 | 3.3 | 2.7 | 0.6 |
| Plant Oil | Caprylic Capric | 2,3-heptanediol | 1.9 | 3.6 | 1.7 | 3.2 | 3.4 | -0.2 | 3.3 | 3 | 0.3 |
| | Triglyceride (neutral oil) | | | | | | | | | | |
| Plant Oil | Caprylic Capric Triglyceride (neutral oil) | 1,2-heptanediol/ 2,3-heptanediol (95:5) | 1.9 | 3.8 | 1.9 | 3.2 | 2.6 | 0.6 | 3.3 | 2.8 | 0.5 |
| | | | | | | | | | | | |
| Plant Oil | Castor Oil | 1,2-heptanediol | 1.2 | 2.2 | 1 | 4.2 | 2.8 | 1.4 | 4.4 | 4 | 0.4 |
| Plant Oil | Castor Oil | 2,3-heptanediol | 1.2 | 1.6 | 0.4 | 4.2 | 3.6 | 0.6 | 4.4 | 3.8 | 0.6 |
| Plant Oil | Castor Oil | 1,2-heptanediol/ 2,3-heptanediol (95:5) | 1.2 | 1.8 | 0.6 | 4.2 | 3.4 | 0.8 | 4.4 | 3.4 | 1 |
| | | | | | | | | | | | |
| Plant Oil | Castor Oil | 1,2-octanediol | 1.2 | 1.6 | 0.4 | 4.2 | 3.6 | 0.6 | 4.4 | 3.4 | 1 |
| Plant Oil | Castor Oil | 2,3-octanediol | 1.2 | 1.8 | 0.6 | 4.2 | 2.8 | 1.4 | 4.4 | 3 | 1.4 |
| Plant Oil | Castor Oil | 1,2-octanediol/ 2,3-octanediol (95:5) | 1.2 | 2 | 0.8 | 4.2 | 3.4 | 0.8 | 4.4 | 3.2 | 1.2 |
| | | | | | | | | | | | |
| Plant oil | Chlorella oil (biotech) | 1,2-heptanediol | 1,3 | 4 | 2,7 | 3 | 2,7 | 0,3 | 3,2 | 3 | 0,2 |
| | | | | | | | | | | | |
| Plant Oil | Coconut Oil | 1,2-heptanediol | 2.4 | 4.2 | 1.8 | 3.2 | 1.8 | 1.4 | 3.4 | 2 | 1.4 |
| Plant Oil | Coconut Oil | 2,3-heptanediol | 2.4 | 3.4 | 1 | 3.2 | 2.4 | 0.8 | 3.4 | 2.4 | 1 |
| Plant Oil | Coconut Oil | 1,2-heptanediol/ 2,3-heptanediol (95:5) | 2.4 | 4.2 | 1.8 | 3.2 | 3 | 0.2 | 3.4 | 2.4 | 1 |
| | | | | | | | | | | | |
| Plant Oil | Coconut Oil | 1,2-octanediol | 2.4 | 4.4 | 2 | 3.6 | 2.6 | 1 | 3.8 | 3 | 0.8 |
| Plant Oil | Coconut Oil | 2,3-octanediol | 2.4 | 4.2 | 1.8 | 3.6 | 2.2 | 1.4 | 3.8 | 2.6 | 1.2 |
| Plant Oil | Coconut Oil | 1,2-octanediol/ 2,3-octanediol (95:5) | 2.4 | 4 | 1.6 | 3.6 | 2.8 | 0.8 | 3.8 | 3 | 0.8 |
| | | | | | | | | | | | |
| Plant Oil | Grapeseed Oil | 1,2-heptanediol | 2 | 3.8 | 1.8 | 4.2 | 2.2 | 2 | 2.6 | 2.8 | 0.2 |
| Plant Oil | Grapeseed Oil | 2,3-heptanediol | 2 | 3.6 | 1.6 | 4.2 | 2.6 | 1.6 | 3 | 2.2 | 0.8 |
| Plant Oil | Grapeseed Oil | 1,2-heptanediol/ 2,3-heptanediol (95:5) | 2 | 3.8 | 1.8 | 4.2 | 2.2 | 2 | 2.6 | 1.6 | 1 |
| | | | | | | | | | | | |
| Plant Oil | Grapeseed Oil | 1,2-octanediol | 2 | 4 | 2 | 4.2 | 2.8 | 1.4 | 2.6 | 1.6 | 1 |
| Plant Oil | Grapeseed Oil | 2,3-octanediol | 2 | 2.8 | 0.8 | 4.2 | 2.8 | 1.4 | 2.6 | 2.4 | 0.2 |
| Plant Oil | Grapeseed Oil | 1,2-octanediol/ 2,3-octanediol (95:5) | 2 | 3.8 | 1.8 | 4.2 | 2.2 | 2 | 2.3 | 1.4 | 0.9 |
| | | | | | | | | | | | |
| Plant Oil | Helianthus Annuus (Sunflower) Seed Oil | 1,2-heptanediol | 1.2 | 4.2 | 3 | 3.8 | 2.3 | 1.5 | 3.3 | 2 | 1.3 |
| Plant Oil | Helianthus Annuus (Sunflower) Seed Oil | 2,3-heptanediol | 1.6 | 3.8 | 2.2 | 3.6 | 2.6 | 1 | 3.6 | 2.6 | 1 |
| Plant Oil | Helianthus Annuus (Sunflower) Seed Oil | 1,2-heptanediol/ 2,3-heptanediol (95:5) | 1.6 | 4.4 | 2.8 | 3.6 | 2.4 | 1.2 | 3.6 | 2 | 1.6 |
| | | | | | | | | | | | |
| Plant Oil | Helianthus Annuus (Sunflower) Seed Oil | 1,2-octanediol | 1.4 | 4.2 | 2.8 | 3.8 | 2 | 1.8 | 3.8 | 2 | 1.8 |
| Plant Oil | Helianthus Annuus (Sunflower) Seed Oil | 2,3-octanediol | 1.4 | 3.4 | 2 | 3.8 | 3 | 0.8 | 3.8 | 3.4 | 0.4 |
| Plant Oil | Helianthus Annuus (Sunflower) Seed Oil | 1,2-octanediol/ 2,3-octanediol (95:5) | 1.4 | 4.4 | 3 | 3.8 | 2.4 | 1.4 | 3.6 | 2.6 | 1 |
| Plant Oil | Helianthus Annuus (Sunflower) Seed Oil | 1,2-heptanediol/ 1,2-octanediol (1:1) | 1.2 | 5 | 3.8 | 3.8 | 2.8 | 1 | 3.3 | 2.2 | 1.1 |
| Plant Oil | Helianthus Annuus (Sunflower) Seed Oil | 1,2-heptanediol/ 1,2-octanediol (95:5) | 1.2 | 4.2 | 3 | 3.8 | 2.8 | 1 | 3.3 | 2.4 | 0.9 |
| | | | | | | | | | | | |
| Plant Oil | Olive Oil | 1,2-heptanediol | 2.2 | 4 | 1.8 | 3.8 | 2 | 1.8 | 4 | 2.4 | 1.6 |
| Plant Oil | Olive Oil | 2,3-heptanediol | 2.2 | 4 | 1.8 | 3.8 | 2.2 | 1.6 | 4 | 2 | 2 |
| Plant Oil | Olive Oil | 1,2-heptanediol/ 2,3-heptanediol (95:5) | 2.2 | 3.8 | 1.6 | 3.8 | 1.8 | 2 | 4 | 2 | 2 |
| | | | | | | | | | | | |
| Plant Oil | Olive Oil | 1,2-octanediol | 2.2 | 4.2 | 2 | 3.8 | 2.6 | 1.2 | 4 | 2.6 | 1.4 |
| Plant Oil | Olive Oil | 2,3-octanediol | 2.4 | 3.2 | 0.8 | 3.8 | 2.4 | 1.4 | 4 | 2.6 | 1.4 |
| Plant Oil | Olive Oil | 1,2-octanediol/ 2,3-octanediol (95:5) | 2.2 | 4.4 | 2.2 | 3.8 | 2.2 | 1.6 | 4 | 2.4 | 1.6 |
| Plant Oil | Prunus Amygdalus Dulcis (Sweet Almond) Oil | 1,2-heptanediol | 1.3 | 3 | 1.7 | 3.3 | 2.5 | 0.8 | 3.7 | 2.2 | 1.5 |
| | | | | | | | | | | | |
| Plant Oil | Simmondsia Chinensis (Jojoba) Seed Oil | 1,2-heptanediol | 1.5 | 4 | 2.5 | 3.8 | 2.8 | 1 | 3.7 | 2.5 | 1.2 |
| | | | | | | | | | | | |
| | | | | | | | | | | | |
| Natural oil | Microalgae oil | 1,2-heptanediol | 2.2 | 3.8 | 1.6 | 3.5 | 2.7 | 0.8 | 3.7 | 2.5 | 1.2 |
| | | | | | | | | | | | |
| | | | | | | | | | | | |
| Fatty alcohol | Hexyldecanol (Eutanol G) | 1,2-heptanediol | 2.5 | 3.5 | 1 | 3 | 3 | 0 | 2.5 | 2.7 | 0.2 |
| | | | | | | | | | | | |
| | | | | | | | | | | | |
| Fatty ester | Cetearyl Nonanoate (SymMollient S) | 1,2-heptanediol | 1.7 | 4.2 | 2.5 | 3.2 | 2.5 | 0.7 | 3.2 | 2 | 1.2 |
| Fatty ester | Propanediol Dicaprylate/D icaprate (Symollient PDCC) | 1,2-heptanediol | 1.6 | 2.9 | 1.3 | 2.7 | 2.5 | 0.2 | 2.5 | 2.4 | 0.1 |
| | | | | | | | | | | | |
| | | | | | | | | | | | |
| UV filter | Ethylhexyl Methoxycinna mate (Neo Heliopan AV) | 1,2-heptanediol | 1.5 | 3.7 | 2.2 | 3.7 | 3.2 | 0.5 | 3.5 | 3 | 0.5 |
| UV filter | Ethylhexyl Salicylate (Neo Heliopan OS) | 1,2-heptanediol | 1.8 | 4.3 | 2.5 | 3.7 | 1.8 | 1.9 | 3.5 | 2.2 | 1.3 |
| UV filter | Homosalate (Neo Heliopan HMS) | 1,2-heptanediol | 2 | 2.8 | 0.8 | 2.7 | 3.2 | 0.5 | 2.8 | 2.5 | 0.3 |
| UV filter | Isoamyl p-Methoxycinna mate (Neo Heliopan E 1000) | 1,2-heptanediol | 1.5 | 2.7 | 1.2 | 4 | 2.7 | 1.3 | 4.2 | 2.5 | 1.7 |
| UV filter | Octocrylene (Neo Heliopan 303) | 1,2-heptanediol | 1 | 2.7 | 1.7 | 1.8 | 3.3 | 1.5 | 4 | 2.7 | 1.3 |

It was found that the use of 1,2-heptanediol or 2,3-heptanediol or a mixture of 1,2-heptanediol and 2,3-heptanediol or 1,2-octanediol or 2,3-octanediol or a mixture of 1,2-octanediol and 2,3-octanediol improves the spreading behavior and/or significantly reduces the fatty/greasy skin feeling of oily ingredients, especially UV filters. In parallel, the absorption (less remaining residue) was improved too.

In particular oil components, especially UV filters, which have a poor spreading by nature, which initial spreading, i.e. without the addition of an alkanediol component, was scored with < 2, show a remarkably improvement in spreading both the tested single alkanediol compound and the tested alkanediol mixture. 1,2-heptanediol and a mixture of 1,2-heptanediol and 2,3-heptanediol perform best.

### Example 5: Formulation examples

In the following formulation examples the following five perfume oils PFO1, PFO2, PFO3, PFO4 or PFO5 were each used as fragrance.

**Table 14: Composition of perfume oil 1; PO1) amounts in ‰ by weight)**

| **Ingredients** | **Amount** |
|---|---|
| Aldehyde c14 so-called | 2.0 |
| Allyl amyl glycolate 10% dpg | 5.0 |
| Anisic aldehyde pure | 5.0 |
| Apple oliffac type | 10.0 |
| Benzylacetate | 50.0 |
| Bergamot identoil^{®} colourless | 15.0 |
| Canthoxal | 5.0 |
| Cetalox 10% ipm | 3.0 |
| Citronellol 950 | 40.0 |
| Damascenone total 1% dpg | 5.0 |
| Damascone alpha 10% dpg | 5.0 |
| Damascone delta 10% dpg | 2.0 |
| Dimethyl benzyl carbinyl butyrate | 2.0 |
| Dipropylene glycol | 178.0 |
| Ebanol | 2.0 |
| Ethyl decadienoate trans cis-2.4 10% ipm | 2.0 |
| Florosa | 5.0 |
| Frambinon^{®} 10% dpg | 7.0 |
| Galaxolide 50% in ipm | 100.0 |
| Galbex type base | 1.0 |
| Geranyl acetate pure | 2.0 |
| Hedione | 30.0 |
| Heliotropin | 10.0 |
| Hexenyl acetate cis-3 10% dpg | 1.0 |
| Hexenyl salicylate cis-3 | 5.0 |
| Hexyl cinnamic aldehyde alpha | 70.0 |
| Hexyl salicylate | 50.0 |
| Hydroxy citronellal | 10.0 |
| Iso e super | 15.0 |
| Isoraldeine 70 | 20.0 |
| Leafovert^{®} | 1.0 |
| Lilial | 60.0 |
| Linalool | 60.0 |
| Linalyl acetate | 20.0 |
| Lyral | 7.0 |
| Manzanate | 2.0 |
| Phenoxanol | 7.0 |
| Phenylethyl alcohol | 120.0 |
| Sandal mysore core | 2.0 |
| Sandranol^{®} | 7.0 |
| Styralyl acetate | 3.0 |
| Tagetes rco 10% tec | 2.0 |
| Terpineol pure | 20.0 |
| Tetrahydrogeraniol 10% dpg | 5.0 |
| Tonalide | 7.0 |
| Vertocitral 10% dpg | 5.0 |
| Vertofix | 15.0 |
| **Total:** | **1000.0** |

**Table 15: Composition of perfume oil 2; PO2 (amounts in ‰ by weight)**

| **Ingredients** | **Amount** |
|---|---|
| Acetophenone. 10% in DPG | 10.0 |
| n-Undecanal | 5.0 |
| Aldehyde C14. so-called (peach aldehyde) | 15.0 |
| Allylamyl glycolate. 10% in DPG | 20.0 |
| Amyl salicylate | 25.0 |
| Benzyl acetate | 60.0 |
| Citronellol | 80.0 |
| d-Limonene | 50.0 |
| Decenol trans-9 | 15.0 |
| Dihydromyrcenol | 50.0 |
| Dimethylbenzylcarbinyl acetate | 30.0 |
| Diphenyloxide | 5.0 |
| Eucalyptol | 10.0 |
| Geraniol | 40.0 |
| Nerol | 20.0 |
| Geranium oil | 15.0 |
| Hexenol cis-3. 10% in DPG | 5.0 |
| Hexenyl salicylate cis-3 | 20.0 |
| Indole. 10% in DPG | 10.0 |
| Alpha-ionone | 15.0 |
| Beta-ionone | 5.0 |
| Lilial^{®} (2-methyl-3-(4-tert-butyl-phenyl)propanal) | 60.0 |
| Linalool | 40.0 |
| Methylphenyl acetate | 10.0 |
| Phenylethyl alcohol | 275.0 |
| Styrolyl acetate | 20.0 |
| Terpineol | 30.0 |
| Tetrahydrolinalool | 50.0 |
| Cinnamyl alcohol | 10.0 |
| **Total:** | **1000.0** |

**Table 16: Composition of perfume oil 3; PO3 (amounts in ‰ by weight)**

| **Ingredients** | **Amount** |
|---|---|
| Benzyl acetate | 60.0 |
| Citronellyl acetate | 60.0 |
| Cyclamenaldehyde (2-methyl-3-(4-isopropylphenyl)propanal | 20.0 |
| Dipropylene glycol (DPG) | 60.0 |
| Ethyllinalool | 40.0 |
| Florol (2-isobutyl-4-methyltetrahydro-2H-pyran-4-ol) | 30.0 |
| Globanone^{®} [(E/Z)-8-cyclohexadecen-1-one] | 180.0 |
| Hedione^{®} (methyldihydrojasmonate) | 140.0 |
| Hexenyl salicylate. cis-3 | 10.0 |
| Vertocitral (2.4-dimethyl-3-cyclohexenecarboxaldehyde) | 5.0 |
| Hydratropaldehyde. 10% in DPG | 5.0 |
| Isodamascone (1-(2.4.4-trimethyl-2-cyclohexen-1-yl)-2-buten-1-one. 10% in DPG | 5.0 |
| Isomuscone (cyclohexadecanone) | 40.0 |
| Jacinthaflor (2-methyl-4-phenyl-1.3-dioxolane) | 10.0 |
| Cis-jasmone. 10% in DPG | 20.0 |
| Linalool | 50.0 |
| Linalyl acetate | 30.0 |
| Methyl benzoate. 10% in DPG | 25.0 |
| para-Methyl cresol. 10% in DPG | 10.0 |
| Nerol | 20.0 |
| Phenylpropylaldehyde | 5.0 |
| 2-Phenylethyl alcohol | 82.0 |
| Tetrahydrogeraniol | 13.0 |
| 2.2-Dimethyl-3-cyclohexyl-1-propanol | 80.0 |
| **Total:** | **1000.0** |

**Table 17: Composition of perfume oil 4; PO4 (amounts in ‰ by weight)**

| **Ingredients** | **Amount** |
|---|---|
| Ambrettolide (macro) | 10.0 |
| Ambroxide 10% in ipm | 10.0 |
| Benzyl acetate | 20.0 |
| Benzyl salicylate | 15.0 |
| Bergamot oil. Bergapten-free | 60.0 |
| Calone^{®} 1951 10% in dpg | 15.0 |
| Coumarin | 5.0 |
| Cyclogalbanate^{®} 10% in dpg | 10.0 |
| Alpha -damascone 1% in dpg | 20.0 |
| Dihydromyrcenol | 10.0 |
| Ethyl linalool | 75.0 |
| Ethyl linalylacetate | 50.0 |
| Ethyl maltol 1% in dep | 10.0 |
| Ethylene brassylate (macro) | 80.0 |
| Florosa | 40.0 |
| Geranylacetate | 10.0 |
| Hedione^{®} hc/30 | 35.0 |
| Hedione^{®} | 210.0 |
| Helional^{®} | 15.0 |
| Helvetolide^{®} (alicyc) | 30.0 |
| Hexenylsalicylate cis-3 | 20.0 |
| Iso e super^{®} | 40.0 |
| Leafovert^{®} 10% in dep | 10.0 |
| Lilial^{®} | 80.0 |
| Lyral^{®} | 20.0 |
| Mandarin oil | 10.0 |
| Styralyl acetate | 5.0 |
| Symrose^{®} | 15.0 |
| Vanillin 10% in dep | 20.0 |
| Dipropylene glycol (dpg) | 50.0 |
| **Total:** | **1000.0** |

**Table 18: Composition of perfume oil 5; PO5 (amounts in ‰ by weight)**

| **Ingredients** | **Amount** |
|---|---|
| Amarocite^{®} | 10.0 |
| Ambrocenide^{®} 10% in dpg | 5.0 |
| Ambroxide | 15.0 |
| Aurelione^{®} (7/8-cyclohexadecenone) (macro) | 70.0 |
| Bergamot oil. Bergapten-free | 90.0 |
| Calone^{®} 1951 10% in dpg | 20.0 |
| Caraway oil | 10.0 |
| Citral | 20.0 |
| Coumarin | 10.0 |
| Alpha-damascone 1% in dpg | 15.0 |
| Dihydromyrcenol | 70.0 |
| Estragon oil | 10.0 |
| Ethyl linalool | 100.0 |
| Ethyl linalylacetate | 90.0 |
| Eugenol | 10.0 |
| Evernyl^{®} | 5.0 |
| Fructate^{®} | 5.0 |
| Geranium oil | 5.0 |
| Hedione^{®} hc/30 | 100.0 |
| Helional^{®} | 10.0 |
| Indole 10% in dpg | 5.0 |
| Iso e super^{®} | 100.0 |
| Kephalis^{®} | 5.0 |
| Lavender oil | 40.0 |
| Citrus oil | 80.0 |
| Lilial^{®} | 30.0 |
| Mandarin oil | 20.0 |
| Muscenone (macro) | 5.0 |
| Sandranol^{®} | 10.0 |
| Vanillin 10% in dpg | 5.0 |
| Dipropylene glycol | 30.0 |
| **Total:** | **1000.0** |

**The** above perfume oils PO1, PO2, PO3, PO4 or PO5 were incorporated into the formulations presented below.

Cosmetic formulations (compositions) (amounts in % by weight for all formulations).

**Table 19: Sunscreen Oil Spray with Ethanol, expected SPF 30, UVA/UVB balanced**

| **Ingredients** | **INCI** | **Amount** |
|---|---|---|
| Neo Heliopan^{®} 357 (622501) | Butyl methoxydibenzoylmethane | 5.0 |
| Neo Heliopan^{®} BMT(102814) | Bis-ethylhexyloxyphenol methoxyphenyl triazine | 3.5 |
| Neo Heliopan^{®} OS (131494) | Ethylhexyl salicylate | 5.0 |
| Uvinul^{®} T 150 | Ethylhexyl triazone | 5.0 |
| SymMollient^{®} PDCC (102119) | Propanediol dicaprylate/caprate | 5.0 |
| Cetiol^{®} Sensoft | Propylheptyl caprylate | 6.0 |
| Cetiol^{®} AB | C12-15 alkyl benzoate | ad 100 |
| Isopropyl Palmitate | Isopropyl palmitate | 20.7 |
| SymDecanox^{™} HA (972276) | Caprylic/capric triglyceride | 1.0 |
| | Hydroxymethoxyphenyl decanone | |
| Ethanol | Alcohol, Aqua | 15.0 |
| Hydrolite^{®} 5 green (996442) | Pentylene glycol | 3.0 |
| 1,2-heptanediol | 1,2-heptanediol | 1.5 |
| 2,3-heptanediol | 2,3-heptanediol | 0.1 |
| Perfume oil PO1, PO2, PO3, PO4, or PO5 | Perfume | 0.3 |

**Table 20: Urban Sun Spray, o/w emulsion, expected SPF 50+**

| **Ingredients** | **INCI** | **amount w/w%** | | |
|---|---|---|---|---|
| Aqua /Water | Aqua | ad 100 | | |
| Vivapur CS Tex sun | Microcrystalline Cellulose, Cellulose Gum | 1.0 | 1.0 | 1.0 |
| Vivapur CS 032 XV | Microcrystalline Cellulose, Xanthan Gum | 1.0 | 1.0 | 1.0 |
| SymOcide PH (973949) | Phenoxyethanol, Hydroxyacetophenone, Caprylyl Glycol, Aqua | 1.4 | 1.4 | 1.4 |
| Emulsiphos (677660) | Potassium cetyl phosphate, Hydrogenatedpalm glycerides | 2.5 | 2.5 | 2.5 |
| Dragoxat 89 (109854) | Ethylhexyl Isononanoate | 2.0 | 2.0 | 2.0 |
| SymMollient PDCC (102119) | Propanediol Dicaprylate/Caprate | 3.5 | 3.5 | 3.5 |
| SymSol PF-3 (358712) | Aqua, Pentylene Glycol, Sodium Lauryl | 2.0 | 2.0 | 2.0 |
| | Sulfoacetate, Sodium Oleoyl Sarcosinate, Sodium Chlrodie, Sodium Oleate | | | |
| Prisorine 3505 | Isostearic acid | 1.5 | 1.5 | 1.5 |
| Neo Heliopan BMT (102814) | Bis-Ethylhexyloxyphenol Methoxypenyl Triazine | 2.5 | 2.5 | 2.5 |
| Neo Heliopan 357 (622501) | Butyl Methoxydibenzoylmeth ane | 4.5 | 4.5 | 4.5 |
| Neo Heliopan 303 (600154) | Octocrylene | 10.0 | 10.0 | 10.0 |
| Neo Heliopan HMS (182573) | Homosalate | 10.0 | 10.0 | 10.0 |
| Neo Heliopan OS (131494) | Ethylhexyl Salicylate | 5.0 | 5.0 | 5.0 |
| Dragosine (844033) | Carnosine | 0.2 | 0.2 | 0.2 |
| SymUrban (386557) | Benzylidene Dimethoxydimetylindan one | 0.2 | 0.2 | 0.2 |
| 1,2-heptanediol | 1,2-heptanediol | 0.5 | - | - |
| 2,3-heptanediol | 2,3-heptanediol | - | 0,1 | - |
| 1,2-heptanediol/2,3-heptanediol (95:5 w/w %) | 1,2-heptanediol/2,3-heptanediol | - | - | 0.5 |
| SymVital AR 3040 (974839) | Zingiber officinale (Ginger) Root extract | 0.1 | 0.1 | 0.1 |
| SymDecanox HA (972276) | Caprylic/Capric Triglyceride, Hydroxymethoxyphenyl decanone | 1.0 | 1.0 | 1.0 |
| Floraesters K-100 Jojoba | Hydrolyzed jojoba esters, Jojoba esters, Aqua | 0.5 | 0.5 | 0.5 |
| EDTA BD | Disodium EDTA | 0.1 | 0.1 | 0.1 |
| Keltrol CG-BT | Xanthan Gum | 0.3 | 0.3 | 0.3 |
| Perfume oil PO1, PO2, PO3, PO4, or PO5 | Perfume oil PO1, PO2, PO3, PO4, or PO5 | 0.5 | 0.5 | 0.5 |
| Farmal MS6135 | Calcium Starch Octenylsuccinate | 2.0 | 2.0 | 2.0 |
| Tapioca Pure | Tapioca starch | 1.5 | 1.5 | 1.5 |

**Table 21: Sunscreen Spray (O/W), expected SPF 30* (cold manufacturing process)**

| **Ingredients** | **INCI** | **Amount** |
|---|---|---|
| Dracorin^{®} GOC (683541) | Glyceryl Oleate Citrate Caprylic/ Capric Triglyceride | 2.0 |
| Dragosine (844033) | Carnosine | 0.2 |
| Neo Heliopan^{®} Flat (294843) | Homosalate, Octocrylene, Bis-Ethylhexyloxyphenol Methoxyphenyl Triazine, Butyl | 25.0 |
| | Methoxydibenzoylmethane Ethylhexyl Salicylate | |
| SymMollient^{®} PDCC (102119) | Propanediol Dicaprylate/Caprate | 7.0 |
| Aristoflex^{®} Silk | Sodium Polyacryloyldiemthyl taurate | 0.6 |
| 2,3-heptanediol | 2,3-heptanediol | 1.0 |
| 1,2-octanediol / 2,3-octanediol (98:2 w/w%) | 1,2-octanediol, 2,3-octanediol | 0.5 |
| Aqua /Water | Aqua | ad 100 |
| Citric Acid 10% Sol. | Aqua, Citric acid | 0.25 |
| SymOcide^{®} PH (973949) | Phenoxyethanol, Hydroxyacetophenone, Caprylyl Glycol, Aqua | 1.45 |
| SymMollient^{®} PDCC (102119) | Propanediol Dicaprylate/ Caprate | 3.0 |
| Glycerin 99.5 % | Glycerin | 3.0 |
| Dragoxat^{®} 89 (109854) | Ethylhexyl Isononanoate | 2.0 |
| PCL-Liquid^{®} 100 (660089) | Cetearyl Ethylhexanoate | 2.0 |
| Edta^{®} BD | Disodium EDTA | 0.1 |
| Farmal MS 6135 | Calcium Starch Octenylsuccinate | 2.0 |
| Keltrol^{®} CG-BT | Xanthan Gum | 0.4 |
| Perfume oil PO1, PO2, PO3, PO4, or PO5 | Perfume | 0.3 |

**Table 22: UV protection water spray, SPF 50**

| **Ingredients** | **INCI** | **Amount** | | |
|---|---|---|---|---|
| Isolan GPS | Isododecane | 10.0 | 10.0 | 10.0 |
| Dermofeel sensolv MB | Diethylhexyl Carboate | 5.0 | 5.0 | 5.0 |
| Tegosoft DC MB | Phenoxyethyl Caprylate | 5.0 | 5.0 | 5.0 |
| Neo Heliopan 357 (622501) | Butyl Methoxydibenzoylmethane | 5.0 | 5.0 | 5.0 |
| Neo Heliopan HMS (182573) | Homosalate | 5.0 | 5.0 | 5.0 |
| Neo Heliopan 303 (600154) | Octocrylene | 5.0 | 5.0 | 5.0 |
| Neo Heliopan OS (131494) | Ethylhexyl Salicylate | 5.0 | 5.0 | 5.0 |
| Neo Heliopan BMT (102814) | Bis-Ethylhexyloxyphenol Methoxypenyl Triazine | 4.0 | 4.0 | 4.0 |
| Uvinul T150 | Ethylhexyl Triazone | 3.0 | 3.0 | 3.0 |
| Dermofeel Toco 70 non GMO | Tocopherol; Helianthus Annuu (Sunflower) Seed oil | 0.5 | 0.5 | 0.5 |
| Tego SP 13 Sun Up | Poly C10-30 Alkyl Acrylate | 0.4 | 0.4 | 0.4 |
| SymEffect Sun (105604) | Cera Alba. Sodium Stearoyl Lactylate | 1.5 | 1.5 | 1.5 |
| SymRepair 100 (445662) | Hexyldecanol. Bisabolol. Cethylhydroxyproline Palmitamide. Stearic acid. Brassica | 1.0 | 1.0 | 1.0 |
| | campestris (Rapeseed) sterols | | | |
| SymDecanox HA (972276) | Caprylic/Capric Triglyceride, Hydroxymethoxyphenyl decanone | 1.0 | 1.0 | 1.0 |
| PCL Liquid 100 (660089) | Cetearyl Ethlyhexanoate | 2.0 | 2.0 | 2.0 |
| 1,2-heptanediol | 1,2-heptanediol | 0.5 | - | - |
| 2,3-heptanediol | 2,3-heptanediol | - | 0.5 | - |
| 1,2-heptanediol/2,3-heptanediol (95:5 w/w %) | 1,2-heptanediol/2,3-heptanediol | - | - | 1.0 |
| Perfume oil PO1; PO2; PO3; PO4; or PO5 | Perfume | 0.5 | 0.5 | 0.5 |
| Dermosoft OMP | Methylpropanediol; Capryly Glycol; Phenylpropanol | 3.0 | 3.0 | 3.0 |
| Water | Water (Aqua) | ad 100 | | |
| Neo Heliopan Hydro (103089) | Phenylbenzimidazole Sulfonic Acid | 2.0 | 2.0 | 2.0 |
| Glycerin | Glycerin, Aqua | 0.5 | 0.5 | 0.5 |
| Sodium hydroxide 10 % sol. | Aqua, Sodium hydroxicde | 0.3 | 0.3 | 0.3 |
| EDTA BD | Disosium EDTA | 0.1 | 0.1 | 0.1 |
| Dragosine (844033) | Carnosine | 0.1 | 0.1 | 0.1 |
| Triethanolamine | Triethanolamine | 0.1 | 0.1 | 0.1 |

**Table 23: Sunscreen Spray, *SPF 50+, very water resistant****

| **Ingredients** | **INCI** | **Amount** |
|---|---|---|
| Emulsiphos^{®} (677660) | Potassium Cetyl Phosphate Hydrogenated Palm Glycerides | 2.0 |
| SymMollient^{®} PDCC (102119) | Propanediol Dicaprylate/ Caprate | 5.0 |
| SymMollient^{®} S green (103227) | Cetearyl Nonanoate | 2.5 |
| Neo Heliopan^{®} 357 (622501) | Butyl Methoxydibenzoylmethane | 5.0 |
| Neo Heliopan^{®} HMS (182573) | Homosalate | 8.0 |
| Neo Heliopan^{®} 303 (600154) | Octocrylene | 8.0 |
| Neo Heliopan^{®} OS (131494) | Ethylhexyl Salicylate | 4.0 |
| Neo Heliopan^{®} BMT (102814) | Bis-Ethylhexyloxyphenol Methoxypenyl Triazine | 4.0 |
| Tocopherol Alpha DL | Tocopherol | 0.2 |
| Edeta^{®} BD | EDTA | 0.1 |
| SymEffect^{®} Sun (105604) | Cera Alba, Sodium Stearoyl Lactylate | 3.0 |
| Keltrol^{®} CG-SFT | Xanthan Gum | 0.2 |
| Water/ Aqua | Aqua | ad 100 |
| Neo Heliopan^{®} Hydro (103089) | Phenylbenzimidazole Sulfonic Acid | 1.5 |
| Biotive^{®} L-Arginine (621277) | Arginine | 1.0 |
| Dragosine^{®} (844033) | Carnosine | 0.1 |
| SymSave^{®} H (979940) | Hydroxyacetophenone | 0.5 |
| Hydrolite^{®} 5 green (996442) | Pentylene glycol | 1.8 |
| SymLite^{®} G8 (104528) | Glyceryl caprylate | 0.6 |
| Cranberry Oil CC (105531) | Vaccinium Macrocappon seed oil | 1.0 |
| Tapioca Pure | Tapioca starch | 2.0 |
| VITACEL^{®} CS 20 FC | Cellulose | 2.0 |
| 1,2-heptanediol | 1,2-heptanediol | 1,5 |
| Perfume oil PO1; PO2; PO3; PO4; or PO5 | Perfume | 0.3 |

**Table 24: Sunscreen Lotion (O/W), expected SPF 50* (cold/cold manufacturing process)**

| **Ingredients** | **INCI** | **Amount** |
|---|---|---|
| Dracorin^{®} GOC (683541) | Glyceryl Oleate Citrate Caprylic/ Capric Triglyceride | 2.0 |
| KP-545 | Cyclopentasiloxane Acrylates/Dimethicone copolymer | 1.0 |
| Neo Heliopan^{®} Flat (294843) | Homosalate, Octocrylene, Bis-Ethylhexyloxyphenol Methoxyphenyl Triazine, Butyl Methoxydibenzoylmethane Ethylhexyl Salicylate | 30.0 |
| SymMollient^{®} PDCC (102119) | Propanediol Dicaprylate/Caprate | 7.0 |
| Aristoflex^{®} Silk | Sodium Polyacryloyldiemthyl taurate | 0.6 |
| 1,2-heptanediol/2,3-heptanediol (95:5 w/w %) | 1,2-heptanediol/2,3-heptanediol | 1,0 |
| 2,3-octanediol | 2,3-octanediol | 0,1 |
| Aqua /Water | Aqua | ad 100 |
| SymOcide^{®} PH (973949) | Phenoxyethanol, Hydroxyacetophenone, Caprylyl Glycol, Aqua | 1,5 |
| Hydrolite^{®} 5 green (996442) | Pentylene glycol | 1.8 |
| Glycerin 99.5 % | Glycerin | 2.0 |
| VITACEL^{®} CS 20 FC | Cellulose | 1.0 |
| Perfume oil PO1, PO2, PO3, PO4, or PO5 | Perfume | 0.3 |

**Table 25: Beach Time Lotion, SPF 30**

| | | A1 | B1 | C1 | D1 |
|---|---|---|---|---|---|
| **Ingredients** | **INCI** | **amount w/w%** | | | |
| Isolan GPS | Polyglyceryl-4 Diisostearate / Polyhydroxystearate / Sebacate | 3.5 | 3.5 | 3.5 | 3.5 |
| Dermofeel sensolv MB | Isoamyl Laurate | 4.5 | 4.5 | 4.5 | 4.5 |
| TEGOSOFT DC MB | Decyl Cocoate | 4.0 | 4.0 | 4.0 | 4 |
| Neo Heliopan^{®} BMT (102814) | Bis-Ethylhexyloxyphenol Methoxypenyl Triazine | 3.0 | 3.0 | 3.0 | 3.0 |
| Uvinul A plus | Diethylamino Hydroxybenzoyl Hexyl Benzoate | 3.0 | 3.0 | 3.0 | 3.0 |
| Neo Heliopan^{®} OS (131494) | Ethylhexyl Salicylate | 4.0 | 4.0 | 4.0 | 4.0 |
| Uvinul T150 | Ethylhexyl Triazone | 3.0 | 3.0 | 3.0 | 3.0 |
| Neo Heliopan HMS (182573) | Homosalate | 5.0 | 5.0 | 5.0 | 5.0 |
| Verstatil TBO | Triethylcitrate; Caprylyl Glycol; benzoic Acid | 1.0 | 1.0 | 1.0 | 1.0 |
| Zinc Stearate | Zinc Stearate | 0.5 | 0.5 | 0.5 | 0.5 |
| 1,2-heptanediol | 1,2-heptanediol | 1,0 | 0.5 | - | - |
| 1,2-heptanediol/2,3-heptanediol (95:5 w/w %) | 1,2-heptanediol/2,3-heptanediol | - | - | - | 0.5 |
| Water | Aqua | ad 100 | | | |
| Glycerin | Glycerin | 3.0 | 3.0 | 3.0 | 3.0 |
| Zinc Sulfate Heptahydrate | Zinc Sulfate Heptahydrate | 1.2 | 1.2 | 1.2 | 1.2 |

**Table 26: Anti-Sweat Sunscreen Lotion**

| **Ingredients** | **INCl** | **Amount** |
|---|---|---|
| Aqua/Water | Aqua | ad 100 |
| 1,2-octanediol / 2,3-octanediol (95:5 w/w %) | 1,2-octanediol/2,3-octanediol | 1,0 |
| 1,2-heptanediol | 1,2-heptanediol | 0.3 |
| Citric Acid 30% Sol. | Aqua, Citric acid | 0.6 |
| Aristoflex^{®} Velvet | Polyacrylate crosspolymer 11 | 0.6 |
| Glycerin 99.5 % | Glycerin, Aqua | 2.0 |
| SymSol^{®} PF-3 (358712) | Aqua, Pentylene glycol, Sodium lauryl sulfoacetate, Sodium oleoyl sarcosinate, Sodium chloride, Sodium oleate | 1.0 |
| SymSave^{®} H (979940) | Hydroxyacetophenone | 0.3 |
| Neo Heliopan^{®} 357 (622501) | Butyl methoxydibenzoylmethane | 3.0 |
| Neo Heliopan^{®} 303 (600154) | Octocrylene | 10.0 |
| Neo Heliopan^{®} OS (131494) | Ethylhexyl salicylate | 5.0 |
| Neo Heliopan^{®} HMS (182573) | Homosalate | 10.0 |
| Neo Heliopan^{®} E1000 (656083) | Isoamyl p-methoxycinnamate | 2.0 |
| Neo Heliopan^{®} BMT(102814) | Bis-ethylhexyloxyphenol methoxyphenyl triazine | 3.0 |
| Floraesters K-100^{®} Jojoba | Hydrolyzed jojoba esters | 2.0 |
| | Jojoba esters | |
| | Aqua | |
| Dragoxat^{®} 89 (109854) | Ethylhexyl isononanoate | 6.0 |
| SymMollient^{®} S (181598) | Cetearyl nonanoate | 1.0 |
| EDTA^{®} BD | Disodium edta | 0.1 |
| SymDecanox^{™} HA (972276) | Caprylic/capric triglyceride | 1.0 |
| | Hydroxymethoxyphenyl decanone | |
| Frescolat^{®} Plus (132553) | Menthol | 0.3 |
| | Menthyl lactate | |
| SymDeo^{®} Plus (763003) | Lauryl alcohol, Phenoxyethanol, 2-benzylheptanol, Decylene glycol | 1.0 |
| Hydroviton^{®} PLUS 2290 (510027) | Aqua, Pentylene glycol, Glycerin, Fructose, Urea, Citric acid, Sodium hydroxide, Maltose, Sodium pca, Sodium chloride, Sodium lactate, Trehalose, Allantoin, Sodium hyaluronate, Glucose | 4.0 |
| Sensocel^{®} 10 | Cellulose | 2.0 |

**Table 27: Sun protection milk (w/o)**

| **Ingredients** | **INCI** | **Amount** |
|---|---|---|
| Dehymuls PGPH | Polyglyceryl-2 dipolyhydroxystearate | 3.0 |
| Beeswax 8100 | Beeswax | 1.0 |
| Monomuls 90-0-18 | Glyceryl oleate | 1.0 |
| Zinc stearate | Zinc stearate | 1.0 |
| Cetiol SN | Cetearyl isononanoate | 5.0 |
| Cetiol OE | Dicaprylyl ether | 5.0 |
| Tegosoft TN | C12-15 alkyl benzoate | 4.0 |
| Vitamin E | Tocopherol | 0.5 |
| Neo Heliopan^{®} OS | Ethylhexyl salicylate | 5.0 |
| Neo Heliopan^{®} AV | Ethylhexyl methoxycinnamate | 7.5 |
| Uvinul^{®} T150 | Ethylhexyl triazone | 1.5 |
| Water, distilled | Water (Aqua) | ad 100 |
| Trilon BD | Disodium EDTA | 0.1 |
| Glycerol | Glycerol | 5.0 |
| Neo Heliopan^{®} AP 10% solution. neutralized with NaOH | Disodium phenyl dibenzimidazole tetrasulfonate | 15.0 |
| Perfume oil PO1, PO2, PO3, PO4, or PO5 | Perfume | 0.3 |
| Alpha bisabolol | Bisabolol | 0.1 |
| SymOcide^{®} PT | Phenoxyethanol. Tropolone | 0.3 |
| 1,2-heptanediol | 1,2-heptanediol | 0.8 |

**Table 28: Protecting Watery Mousse, expected SPF 30***

| **Ingredients** | **INCI** | **Amount** |
|---|---|---|
| Aqua/Water | Aqua | ad 100 |
| Neo Heliopan^{®} Hydro (103089) | Phenylbenzimidazole sulfonic acid | 2.0 |
| Neo Heliopan^{®} AP (106796) | Disodium phenyl dibenzimidazole tetrasulfonate | 1.0 |
| Biotive^{®} L-Arginine (621277) | Arginine | 1.8 |
| SymSol^{®} PF-3 (358712) | Aqua, Pentylene glycol, Sodium lauryl, sulfoacetate, Sodium oleoyl sarcosinate, Sodium chloride, Sodium oleate | 2.5 |
| Dragosine^{®} (844033) | Carnosine | 0.2 |
| Aqua Keep 10SH-NFC | Sodium acrylates crosspolymer-2 | 2.5 |
| SymSave^{®} H (979940) | Hydroxyacetophenone | 0.5 |
| Phenoxyethanol | Phenoxyethanol | 0.5 |
| Hydrolite^{®} 5 (616751) | Pentylene glycol | 1.5 |
| Neo Heliopan^{®} 357 (622501) | Butyl methoxydibenzoylmethane | 3.0 |
| Neo Heliopan^{®} OS (131494) | Ethylhexyl salicylate | 5.0 |
| Neo Heliopan^{®} BMT(102814) | Bis-ethylhexyloxyphenol methoxyphenyl triazine | 3.0 |
| Uvasorb^{®} HEB | Diethylhexyl butamido triazone | 3.0 |
| SymMollient^{®} S (181598) | Cetearyl nonanoate | 3.0 |
| EDTA^{®} BD | Disodium edta | 0.1 |
| Isoadipate (660014) | Diisopropyl adipate | 4.0 |
| Cetiol^{®} AB | C12-15 alkyl benzoate | 7.0 |
| Caprylic/Capric Triglyceride | Caprylic/capric triglyceride | 6.0 |
| Frescolat^{®} ML (620105) | Menthyl lactate | 2.0 |
| SymGlucan^{®} (151719) | Aqua, Glycerin, 1,2-hexanediol, Caprylyl glycol, Beta-glucan | 2.0 |
| 1,2-nonanediol | 1,2-nonanediol | 0,1 |
| Color | - | 0.2 |
| 1,2-hexanediol | 1,2-hexanediol | 0.5 |
| 1,2-heptanediol/2,3-heptanediol (95:5 w/w %) | 1,2-heptanediol, 2,3-heptanediol | 1,0 |

**Table 29: Full protection emulsion, expected SPF 50**

| **Ingredients** | **INCI** | **Amount** |
|---|---|---|
| Dracorin^{®} GOC (683541) | Glyceryl oleate citrate, Caprylic/capric triglyceride | 2.5 |
| Neo Heliopan^{®} 303 (600154) | Octocrylene | 8.0 |
| Neo Heliopan^{®} 357 (622501) | Butyl methoxydibenzoylmethane | 4.5 |
| Neo Heliopan^{®} HMS (182573) | Homosalate | 10.0 |
| Neo Heliopan^{®} OS (131494) | Ethylhexyl salicylate | 5.0 |
| Neo Heliopan^{®} BMT(102814) | Bis-ethylhexyloxyphenol methoxyphenyl triazine | 5.0 |
| SymMollient^{®} PDCC (102119) | Propanediol dicaprylate/caprate | 5.0 |
| Velvesil^{®} DM | Dimethicone, Cetearyl dimethicone crosspolymer | 3.0 |
| Dow Corning^{®} 9041 Silicone Elastomer Blend | Dimethicone, Dimethicone crosspolymer | 2.0 |
| EDTA^{®} BD | Disodium edta | 0.1 |
| Keltrol^{®} CG-SFT | Xanthan gum | 0.2 |
| Pemulen^{™} TR-2 Polymeric Emulsifier | Acrylates/c10-30 alkyl acrylate crosspolymer | 0.1 |
| Aqua/Water | Aqua | ad 100 |
| Glycerin 99.5 % | Glycerin, Aqua | 3.0 |
| Dragosine^{®} (844033) | Carnosine | 0.2 |
| SymSave^{®} H (979940) | Hydroxyacetophenone | 0.5 |
| Hydrolite^{®} 5 green (996442) | Pentylene glycol | 0.8 |
| Hydrolite^{®} CG (199602) | Caprylyl glycol | 0.3 |
| 1,2-octanediol/2,3-octanediol (95:5 w/w %) | 1,2-octanediol, 2,3-octanediol | 0.8 |
| SymVital^{®} AR (974839) | Zingiber officinale (ginger) root extract | 0.2 |
| Tapioca Pure | Tapioca starch | 2.0 |
| Perfume oil PO1, PO2, PO3, PO4, or PO5 | Perfume | 0.5 |

**Table 30: Whitening emulsion, expected SPF 50plus***

| **Ingredients** | **INCI** | **Amount** |
|---|---|---|
| Emulsiphos^{®} (677660) | Potassium cetyl phosphate | 2.0 |
| | Hydrogenated palm glycerides | |
| Lanette^{®} 16 | Cetyl alcohol | 0.5 |
| Neo Heliopan^{®} 357 (622501) | Butyl methoxydibenzoylmethane | 4.9 |
| Neo Heliopan^{®} HMS (182573) | Homosalate | 10.0 |
| Neo Heliopan^{®} 303 (600154) | Octocrylene | 10.0 |
| Neo Heliopan^{®} OS (131494) | Ethylhexyl salicylate | 5.0 |
| Neo Heliopan^{®} BMT(102814) | Bis-ethylhexyloxyphenol methoxyphenyl triazine | 4.0 |
| SymBright^{™} 2036 (352400) | Sclareolide | 0.2 |
| SymWhite^{®} PLUS (510494) | Caprylic/capric triglyceride, Pentylene glycol Phenylethyl resorcinol, Bisabolol, Butyl methoxydibenzoylmethane | 1.0 |
| Isoadipate (660014) | Diisopropyl adipate | 5.0 |
| KP-545 | Cyclopentasiloxane | 1.0 |
| | Acrylates/dimethicone copolymer | |
| Velvesil^{®} DM | Dimethicone | 2.0 |
| | Cetearyl dimethicone crosspolymer | |
| Prisorine^{®} 3505 | Isostearic acid | 1.0 |
| EDTA^{®} BD | Disodium edta | 0.1 |
| Hydrolite^{®} 5 green (996442) | Pentylene glycol | 3.0 |
| Biotive^{®} Resveratrol (903268) | Resveratrol | 0.1 |
| Keltrol^{®} CG-BT | Xanthan gum | 0.5 |
| Pemulen^{®} EZ-4U | Acrylates/c10-30 alkyl acrylate crosspolymer | 0.1 |
| Aqua/Water | Aqua | ad 100 |
| Biotive^{®} L-Arginine (621277) | Arginine | 0.2 |
| Extrapone^{®} Pearl GW (156742) | Aqua, Glycerin, Hydrolyzed pearl Xanthan gum | 0.5 |
| SymSave^{®} H (979940) | Hydroxyacetophenone | 0.5 |
| VITACEL^{®} CS 20 FC | Cellulose | 2.0 |
| 1,2-heptanediol | 1,2-heptanediol | 0.3 |
| 1,2-octanediol | 1,2-octanediol | 0.2 |
| SymOcide^{®} C (510633) | O-cymen-5-ol | 0.1 |
| Propylene Glycol (160221) | Propylene glycol | 2.0 |
| Cosmetic Color Pearlescent Silver AA (100012) | Mica, Titanium dioxide | 3.0 |
| Perfume oil PO1, PO2, PO3, PO4, or PO5 | Perfume | 0.5 |

**Table 31: Sunscreen Fluid for acne-prone Skin, expected SPF 30**

| **Ingredients** | **INCI** | **Amount** |
|---|---|---|
| Dracorin^{®} GOC (683541) | Glyceryl oleate citrate | 2.5 |
| | Caprylic/capric triglyceride | |
| Neo Heliopan^{®} 357 (622501) | Butyl methoxydibenzoylmethane | 4.2 |
| Neo Heliopan^{®} OS (131494) | Ethylhexyl salicylate | 5.0 |
| Neo Heliopan^{®} BMT(102814) | Bis-ethylhexyloxyphenol methoxyphenyl triazine | 3.5 |
| Uvasorb^{®} HEB | Diethylhexyl butamido triazone | 7.0 |
| SymMollient^{®} S (181598) | Cetearyl nonanoate | 1.0 |
| Caprylic/Capric Triglyceride | Caprylic/capric triglyceride | 8.0 |
| EDTA^{®} BD | Disodium edta | 0.1 |
| SymDecanox^{™} HA (972276) | Caprylic/capric triglyceride | 1.0 |
| | Hydroxymethoxyphenyl decanone | |
| Isoadipate (660014) | Diisopropyl adipate | 4.0 |
| SymClariol^{®} (344028) | Decylene glycol | 0.3 |
| Cetiol^{®} AB | C12-15 alkyl benzoate | 13.5 |
| Keltrol^{®} CG-SFT | Xanthan gum | 0.2 |
| Keltrol^{®} CG-BT | Xanthan gum | 0.1 |
| Food Color Brown E172+E171 Powder (656623) | Titanium dioxides (ci 77891), Iron oxides (ci 77492), Iron oxides (ci 77491), Iron oxides (ci. 77499) | 3.0 |
| Eusolex^{®} T-AVO | Titanium dioxide, Silica | 2.0 |
| Aqua/Water | Aqua | ad 100 |
| Glycerin 99.5 % | Glycerin, Aqua | 3.0 |
| Lanette^{®} E | Sodium cetearyl sulfate | 0.8 |
| Neo Heliopan^{®} Hydro (103089) | Phenylbenzimidazole sulfonic acid | 2.0 |
| Biotive^{®} L-Arginine (621277) | Arginine | 1.0 |
| Sodium Hydroxide 10% solution | Aqua | 0.7 |
| | Sodium hydroxide | |
| SymSave^{®} H (979940) | Hydroxyacetophenone | 0.5 |
| Hydrolite^{®} 5 (616751) | Pentylene glycol | 1.5 |
| 2,3-octanediol | 2,3-octanediol | 0.05 |
| 1,2-heptanediol/2,3-heptanediol (95:5 w/w %) | 1,2-heptanediol, 2,3-heptanediol | 1.5 |
| Phenoxyethanol | Phenoxyethanol | 0.5 |
| SymGlucan^{®} (151719) | Aqua, Glycerin, 1,2-hexanediol, Caprylyl glycol, Beta-glucan | 2.0 |
| Sensocel^{®} 10 | Cellulose | 2.0 |

**Table 32: No More Shine Fluid, expected SPF50**

| **Ingredients** | **INCI** | **Amount** |
|---|---|---|
| Dracorin^{®} GOC (683541) | Glyceryl oleate citrate | 2.0 |
| | Caprylic/capric triglyceride | |
| Neo Heliopan^{®} 357 (622501) | Butyl methoxydibenzoylmethane | 4.5 |
| Neo Heliopan^{®} OS (131494) | Ethylhexyl salicylate | 5.0 |
| Neo Heliopan^{®} HMS (182573) | Homosalate | 10.0 |
| Neo Heliopan^{®} 303 (600154) | Octocrylene | 8.0 |
| Neo Heliopan^{®} BMT(102814) | Bis-ethylhexyloxyphenol methoxyphenyl triazine | 3.0 |
| SymMollient^{®} PDCC (102119) | Propanediol dicaprylate/caprate | 5.0 |
| KP-545 L | Dimethicone | 1.0 |
| | Acrylates/dimethicone copolymer | |
| EDTA^{®} BD | Disodium edta | 0.1 |
| Velvesil^{®} DM | Dimethicone | 2.5 |
| | Cetearyl dimethicone crosspolymer | |
| Silsoft^{™} 034 | Caprylyl methicone | 1.0 |
| Keltrol^{®} CG-BT | Xanthan gum | 0.3 |
| Aristoflex^{®} Silk | Sodium polyacryloyldimethyl taurate | 0.5 |
| Aqua/Water | Aqua | ad 100 |
| Neo Heliopan^{®} Hydro (103089) | Phenylbenzimidazole sulfonic acid | 1.0 |
| Biotive^{®} L-Arginine (621277) | Arginine | 0.7 |
| Lanette^{®} E | Sodium cetearyl sulfate | 0.8 |
| Dragosine^{®} (844033) | Carnosine | 0.2 |
| SymSave^{®} H (979940) | Hydroxyacetophenone | 0.5 |
| Hydrolite^{®} 5 green | Pentylene glycol | 1.0 |
| 2,3-octanediol | 2,3-octanediol | 0.05 |
| 1,2-heptanediol/2,3-heptanediol (96:4 w/w %) | 1,2-heptanediol, 2,3-heptanediol | 1.0 |
| Hydrolite^{®} CG (199602) | Caprylyl glycol | 0.3 |
| Glycerin 99.5 % | Glycerin, Aqua | 3.0 |
| Food color brown e172+e171 powder (656623) | Titanium dioxides (ci 77891), Iron oxides (ci 77492), Iron oxides (ci 77491), Iron oxides (ci. 77499) | 2.5 |
| Cosmetic color titanium dioxide ci77891 (137877) | Titanium dioxide | 4.0 |
| SymDecanox^{™} HA (972276) | Caprylic/capric triglyceride | 0.5 |
| | Hydroxymethoxyphenyl decanone | |
| Sensocel^{®} 10 | Cellulose | 1.5 |
| Phytoconcentrole^{®} Carrot (659772) | Helianthus annuus seed oil, Beta-carotene Daucus carota sativa root extract | 0.3 |
| Perfume oil PO1, PO2, PO3, PO4, or PO5 | Perfume | 0.3 |
| Citric Acid 10% Sol. | Aqua, Citric acid | 0.2 |

**Table 33: Light Sunscreen Fluid, expected SPF 50**

| **Ingredients** | **INCI** | **Amount** |
|---|---|---|
| Aqua/Water | Aqua | ad 100 |
| Glycerin 99.5 % | Glycerin, Aqua | 3.0 |
| Hydrolite^{®} 5 green (996442) | Pentylene glycol | 0.8 |
| 2,3-octanediol | 2,3-octanediol | 0,05 |
| 1,2-heptanediol | 1,3-heptanediol | 0,8 |
| SymSave^{®} H (979940) | Hydroxyacetophenone | 0.5 |
| Hydrolite^{®} CG (199602) | Caprylyl glycol | 0.3 |
| Neo Heliopan^{®} Hydro (103089) | Phenylbenzimidazole sulfonic acid | 2.0 |
| Biotive^{®} L-Arginine (621277) | Arginine | 1.4 |
| Emulsiphos^{®} (677660) | Potassium cetyl phosphate | 1.0 |
| | Hydrogenated palm glycerides | |
| Neo Heliopan^{®} 303 (600154) | Octocrylene | 10.0 |
| Neo Heliopan^{®} 357 (622501) | Butyl methoxydibenzoylmethane | 5.0 |
| Neo Heliopan^{®} HMS (182573) | Homosalate | 10.0 |
| Neo Heliopan^{®} BMT(102814) | Bis-ethylhexyloxyphenol methoxyphenyl triazine | 4.0 |
| Neo Heliopan^{®} OS (131494) | Ethylhexyl salicylate | 5.0 |
| Neo Heliopan^{®} E1000 (656083) | Isoamyl p-methoxycinnamate | 2.0 |
| Dragoxat^{®} 89 (109854) | Ethylhexyl isononanoate | 3.0 |
| Tocopheryl Acetate | Tocopheryl acetate | 0.5 |
| Keltrol^{®} CG-BT | Xanthan gum | 0.1 |
| Keltrol^{®} CG-SFT | Xanthan gum | 0.3 |
| VITACEL^{®} CS 20 FC | Cellulose | 2.0 |
| Tapioca Pure | Tapioca starch | 2.0 |
| Ethanol 96 % | Alcohol denat. | 5.0 |
| Silsoft^{™} 034 | Caprylyl methicone | 3.0 |
| Perfume oil PO1, PO2, PO3, PO4, or PO5 | Perfume | 0.3 |

**Table 34: Refreshing Gel Sunscreen, expected SPF 30, UVA/UVB balanced**

| **Ingredients** | **INCI** | **Amount** |
|---|---|---|
| Aqua/Water | Aqua | ad 100 |
| 1,2-heptanediol/2,3-heptanediol (95:5 w/w %) | 1,2-heptanediol, 2,3-heptanediol | 1,0 |
| 2,3-octanediol | 2,3-octanediol | 0.3 |
| Neo Heliopan^{®} Hydro (103089) | Phenylbenzimidazole sulfonic acid | 2.0 |
| Biotive^{®} L-Arginine (621277) | Arginine | 1.0 |
| Sodium Hydroxide 10% solution | Aqua, Sodium hydroxide | 0.2 |
| Dragosine^{®} (844033) | Carnosine | 0.2 |
| SymGuard^{®} CD (675075) | Phenylpropanol, O-cymen-5-ol, Decylene glycol | 0.5 |
| SymDeo^{®} B125 (399939) | 2-methyl 5-cyclohexylpentanol | 0.5 |
| SymSave^{®} H (979940) | Hydroxyacetophenone | 0.5 |
| SymSol^{®} PF-3 (358712) | Aqua, Pentylene glycol, Sodium lauryl sulfoacetate, Sodium oleoyl sarcosinate, Sodium chlorid, Sodium oleate | 1.5 |
| Neo Heliopan^{®} 357 (622501) | Butyl methoxydibenzoylmethane | 3.0 |
| Neo Heliopan^{®} OS (131494) | Ethylhexyl salicylate | 5.0 |
| Neo Heliopan^{®} BMT(102814) | Bis-ethylhexyloxyphenol methoxyphenyl triazine | 1.5 |
| Neo Heliopan^{®} HMS (182573) | Homosalate | 2.5 |
| Neo Heliopan^{®} 303 (600154) | Octocrylene | 10.0 |
| EDTA^{®} BD | Disodium edta | 0.1 |
| SymMollient^{®} PDCC (102119) | Propanediol dicaprylate/caprate | 2.0 |
| Floraesters K-100^{®} Jojoba | Hydrolyzed jojoba esters, Jojoba esters, Aqua | 1.0 |
| Frescolat^{®} X-Cool (370610) | Menthyl ethylamido oxalate | 0.8 |
| Aristoflex^{®} AVC | Ammonium acryloyoldimethyltaurate/vp copolymer | 1.4 |
| Keltrol^{®} CG-T | Xanthan gum | 0.1 |
| Sensocel^{®} 10 | Cellulose | 3.0 |
| Perfume oil PO1, PO2, PO3, PO4, or PO5 | Perfume | 0.4 |
| Phytoconcentrole^{®} Carrot (659772) | Helianthus annuus seed oil, Beta-carotene, Daucus carota sativa root extract | 0.3 |

**Table 35: Sun protection balm, SPF 40**

| **Ingredients** | **INCI** | **Amount** |
|---|---|---|
| Aqua/Water | Aqua | ad 100 |
| Neo Heliopan^{®} Hydro (622501) | Phenylbenzimidazole sulfonic acid | 1.0 |
| 1,2-heptanediol | 1,2-heptanediol | 1,0 |
| 1,2-octanediol / 2,3-octanediol blend (98:2 w/w% | 1,2-octanedio, 2,3-octanediol | 0,5 |
| Hydrolite^{®}-5 | Pentylene glycol | 2.0 |
| Biotive^{®} L-Arginine | Arginine | 0.6 |
| Hydrolite^{®} CG | Caprylyl glycol | 0.25 |
| EDTA^{®} BD | Disodium EDTA | 0.1 |
| SymSave^{®} H (979940) | Hydroxyacetophenone | 0.3 |
| Hyaluronic Acid | Hyaluronic Acid | 0.1 |
| Dragosine^{®} | Carnosine | 0.2 |
| Aristoflex^{®} Silk | Sodium Polyacrydimethyl Taurate | 1.1 |
| Neo Heliopan^{®} 357 (622501) | Butyl methoxydibenzoylmethane | 5.0 |
| Neo Heliopan^{®} 303 (600154) | Octocrylene | 8.0 |
| Neo Heliopan^{®} OS (131494) | Ethylhexyl salicylate | 5.0 |
| Neo Heliopan^{®} HMS (182573) | Homosalate | 3.0 |
| Neo Heliopan^{®} BMT(102814) | Bis-ethylhexyloxyphenol methoxyphenyl triazine | 3.0 |
| Dragoxat^{®} 89 (109854) | Ethylhexyl isononanoate | 2.0 |
| SymMollient^{®} PDCC | Cetearyl nonanoate | 3.0 |
| Vitacel^{®} CS 20 FC | Cellulose | 2.0 |

**Table 36: Anti-Pollution Hair Care, SPF 15**

| **Ingredients** | **INCI** | **Amount** |
|---|---|---|
| Caprylic/Capric Triglyceride | Caprylic/capric triglyceride | 18.0 |
| SymOleo@ Vita7 (637260) | Glycine soja oil, Gossypium herbaceum seed oil, Mangifera indica seed butter, Olea europaea fruit oil, Persea gratissima oil, Prunus amygdalus dulcis (sweet almond) oil, Theobroma cacao seed butter | 2.0 |
| SymMollient^{®} PDCC (102119) | Propanediol dicaprylate/caprate | 5.0 |
| Neo Heliopan^{®} BMT 0(102814) | Bis-ethylhexyloxyphenol methoxyphenyl triazine | 2.0 |
| Neo Heliopan^{®} 303 (600154) | Octocrylene | 5.0 |
| Neo Heliopan^{®} OS (131494) | Ethylhexyl salicylate | 5.0 |
| Cetiol^{®} CC | Dicaprylyl carbonate | 5.0 |
| Sweet Almond Oil (659831) | Prunus amygdalus dulcis (sweet almond) oil | 15.0 |
| Cotton Seed Oil (628612) | Gossypium herbaceum seed oil | 15.0 |
| Grape Seed Oil (Refined) (604680) | Vitis vinifera seed oil | 12.0 |
| Xiameter^{®} PMX-345 | Cyclopentasiloxane, Cyclohexasiloxane | 10.0 |
| SymHair^{®} Shape & Color (805457) | Cetearyl nonanoate, Triticum vulgare germ oil, Caprylic/capric triglyceride, Linoleic acid, Triticum vulgare bran extract, Triticum vulgare germ extract Camellia oleifera seed oil | 1.0 |
| SymDecanox^{™} HA (972276) | Caprylic/capric triglyceride, Hydroxymethoxyphenyl decanone | 2.0 |
| 1,2-heptanediol | 1,2-heptanediol | 1.5 |
| 2,3-heptanediol | 2,3-heptanediol | 0.05 |
| Perfume oil PO1, PO2, PO3, PO4, or PO5 | Perfume | 2.4 |

**Table 37: Perfect Hand emulsion, expected SPF 15, UVA/UVB balanced**

| **Ingredients** | **INCI** | **Amount** |
|---|---|---|
| Emulsiphos^{®} (677660) | Potassium cetyl phosphate | 1.5 |
| | Hydrogenated palm glycerides | |
| Lanette^{®} O | Cetearyl alcohol | 1.0 |
| SymWhite 377 | Phenylethyl Resorcinol | 0.4 |
| Tegosoft^{®} MM | Myristyl myristate | 0.5 |
| Neo Heliopan^{®} BMT(102814) | Bis-ethylhexyloxyphenol methoxyphenyl triazine | 2.0 |
| Neo Heliopan^{®} OS (131494) | Ethylhexyl salicylate | 5.0 |
| Edeta^{®} BD | Disodium EDTA | 0.1 |
| Dragoxat^{®} 89 (109854) | Ethylhexyl isononanoate | 2.0 |
| Isoadipate (660014) | Diisopropyl adipate | 6.0 |
| PCL-Liquid^{®} 100 (660089) | Cetearyl ethylhexanoate | 3.0 |
| Corapan^{®} TQ (182585) | Diethylhexyl 2,6-naphthalate | 2.0 |
| Cetiol^{®} SB 45 | Butyrospermum parkii butter | 1.5 |
| SymBright^{™} 2036 (352400) | Sclareolide | 0.2 |
| SymDecanox^{™} HA (972276) | Caprylic/capric triglyceride, Hydroxymethoxyphenyl decanone | 1.0 |
| SymCare^{®} O (510634) | Hexyldecanol, Pentylene glycol, 4-t-butylcyclohexanol, Bisabolol, Cetylhydroxyproline palmitamide, Hydroxyphenyl propamidobenzoic acid, Stearic acid, Brassica campestris (rapeseed) sterols, Zingiber officinale (ginger) root extract | 2.0 |
| SymDetox^{®} (355267) | Methylcyclohexyl Succinate Dimethylamide | 0.5 |
| Genuvisco^{®} Carrageenan CG-131 | Chondrus crispus powder | 0.4 |
| Keltrol^{®} CG-SFT | Xanthan gum | 0.2 |
| Aqua/Water | Aqua | ad 100 |
| 1,2-heptanediol / 2,3-heptanediol blend (95:5 w/w%) | 1,2-heptanediol; 2,3-octanediol | 0,5 |
| 2,3-octanediol | 2,3-octanediol | 0.3 |
| 1,2-nonanediol | 1,2-nonanediol | 0,2 |
| Neo Heliopan^{®} Hydro (103089) | Phenylbenzimidazole sulfonic acid | 2.5 |
| Biotive^{®} L-Arginine (621277) | Arginine | 1.0 |
| Sodium Hydroxide 10% solution | Aqua | 1.3 |
| | Sodium hydroxide | |
| Glycerin (657319) | Glycerin | 2.0 |
| SymSave^{®} H (979940) | Hydroxyacetophenone | 0.5 |
| SymDiol^{®} 68 (108580) | 1,2-hexanediol, Caprylyl glycol | 0.5 |
| RonaFlair^{®} Flawless | Silica | 1.5 |
| | Ci 77892 | |
| | Ci 77491 | |
| Extrapone^{®} Moroccan Rose GW (111872) | Aqua, Glycerin, Peg-40 hydrogenated castor oil, Rosa damascena flower extract | 1.0 |
| Tego^{®} Feel C10 | Cellulose | 1.5 |
| Perfume oil PO1, PO2, PO3, PO4, or PO5 | Perfume | 0.5 |

**Table 38: Tinted Sunscreen cream against hyperpigmentation**

| **Ingredients** | **INCI** | **Amount** |
|---|---|---|
| Emulsiphos^{®} (677660) | Potassium cetyl phosphate | 2.0 |
| | Hydrogenated palm glycerides | |
| Neo Heliopan^{®} 357 (622501) | Butyl methoxydibenzoylmethane | 4.2 |
| Neo Heliopan^{®} OS (131494) | Ethylhexyl salicylate | 5.0 |
| Neo Heliopan^{®} BMT(102814) | Bis-ethylhexyloxyphenol methoxyphenyl triazine | 3.5 |
| Uvasorb^{®} HEB | Diethylhexyl butamido triazone | 7.0 |
| SymMollient^{®} S (181598) | Cetearyl nonanoate | 1.0 |
| Isoadipate (660014) | Diisopropyl adipate | 4.0 |
| SymBright^{™} 2036 (352400) | Sclareolide | 0.2 |
| SymSitive^{®} 1609 (399944) | Pentylene glycol, 4-t-butylcyclohexanol | 1.0 |
| Caprylic/Capric Triglyceride | Caprylic/capric triglyceride | 8.0 |
| SymDecanox^{™} HA (972276) | Caprylic/capric triglyceride | 1.0 |
| | Hydroxymethoxyphenyl decanone | |
| Cetiol^{®} AB | C12-15 alkyl benzoate | 13.5 |
| EDTA^{®} BD | Disodium edta | 0.1 |
| Keltrol^{®} CG-BT | Xanthan gum | 0.3 |
| Genuvisco^{®} Carrageenan CG-131 | Chondrus crispus powder | 0.2 |
| Food Color Brown E172+E171 Powder (656623) | Titanium dioxides (ci 77891), Iron oxides (ci 77492), Iron oxides (ci 77491), Iron oxides | 2.0 |
| | (ci. 77499) | |
| Eusolex^{®} T-AVO | Titanium dioxide, Silica | 2.0 |
| Aqua/Water | Aqua | 31.4 |
| Neo Heliopan^{®} Hydro (103089) | Phenylbenzimidazole sulfonic acid | 2.0 |
| Biotive^{®} L-Arginine (621277) | Arginine | 1.0 |
| Sodium Hydroxide 10% solution | Aqua, Sodium hydroxide | 0.7 |
| Dragosine^{®} (844033) | Carnosine | 0.2 |
| Glycerin 99.5 % | Glycerin | 3.0 |
| | Aqua | |
| SymSave^{®} H (979940) | Hydroxyacetophenone | 0.5 |
| Hydrolite^{®} 5 green (996442) | Pentylene glycol | 1.5 |
| 1,2-hexanediol | 1,2-hexanediol | 0.2 |
| 2,3-octanediol | 2,3-octanediol | 0.3 |
| Phenoxyethanol | Phenoxyethanol | 0.5 |
| Lanette^{®} E | Sodium cetearyl sulfate | 0.8 |
| Perfume oil PO1, PO2, PO3, PO4, or PO5 | Perfume | 0.5 |
| SymOat PLUS (400124) | Aqua, Glycerin, Maltodextrin, 1,2-hexanediol, Beta-glucan, Caprylyl glycol | 2.0 |
| Sensocel^{®} 10 | Cellulose | 1.0 |

**Table 39: BB Cream Dark Tone, expected SPF-20**

| **Ingredients** | **INCI** | **Amount** |
|---|---|---|
| Emulsiphos^{®} (677660) | Potassium cetyl phosphate Hydrogenated palm glycerides | 2.5 |
| Eumulgin^{®} SG | Sodium stearoyl glutamate | 0.5 |
| PCL-Liquid^{®} 100 (660089) | Cetearyl ethylhexanoate | 2.0 |
| Lanette^{®} O | Cetearyl alcohol | 1.0 |
| Dragoxat^{®} 89 (109854) | Ethylhexyl isononanoate | 1.0 |
| Cutina^{®} PES | Pentaerythrityl distearate | 1.0 |
| SymMollient^{®} S (181598) | Cetearyl nonanoate | 1.0 |
| Xiameter^{®} PMX-0245 Cyclopentasiloxane | Cyclopentasiloxane | 2.0 |
| Xiameter^{®} PMX-200 Silicone Fluid 50 cs | Dimethicone | 1.0 |
| Neo Heliopan^{®} 357 (622501) | Butyl methoxydibenzoylmethane | 3.0 |
| Neo Heliopan^{®} 303 (600154) | Octocrylene | 7.0 |
| Neo Heliopan^{®} HMS (182573) | Homosalate | 7.0 |
| Neo Heliopan^{®} BMT(102814) | Bis-ethylhexyloxyphenol methoxyphenyl triazine | 1.0 |
| Neo Heliopan^{®} OS (131494) | Ethylhexyl salicylate | 5.0 |
| SymDecanox^{™} HA (972276) | Caprylic/capric triglyceride Hydroxymethoxyphenyl decanone | 2.0 |
| Talcum | Talc | 2.0 |
| Food Color Titanium Dioxide Powder E171 (656838) | Ci 77891 | 0.8 |
| Keltrol^{®} CG-SFT | Xanthan gum | 0.4 |
| Carbopol^{®} Ultrez 20 Polymer | Acrylates/c10-30 alkyl acrylate crosspolymer | 0.3 |
| EDTA NA2 (800130) | Disodium EDTA | 0.2 |
| Aqua/Water | Aqua | ad 100 |
| Neo Heliopan^{®} Hydro (103089) | Phenylbenzimidazole sulfonic acid | 1.0 |
| Biotive^{®} L-Arginine (621277) | Arginine | 1.0 |
| Sodium Hydroxide 10% solution | Aqua | 0.7 |
| | Sodium hydroxide | |
| COSMETIC COLOR BROWN | Ci 77891 | 1.5 |
| POWDER (656509) | Ci 77492 | |
| | Ci 77491 | |
| | Ci 77499 | |
| Food Color Iron Oxide Yellow E172 (656835) | Ci 77492 | 0.3 |
| Glycerin (657319) | Glycerin | 3.0 |
| Hydrolite^{®} 5 (616751) | Pentylene glycol | 1.5 |
| Hydrolite^{®} CG (199602) | Caprylyl glycol | 0.2 |
| SymSave^{®} H (979940) | Hydroxyacetophenone | 0.5 |
| 2,3-heptanediol | 2,3-heptanediol | 0.5 |
| 1,2-heptanediol / 2,3-heptanediol blend (95:5) | 1,2-heptanediol, 2,3-heptanediol | 0.5 |
| Hydroviton^{®} PLUS 2290 (510027) | Aqua, Pentylene glycol, Glycerin, Fructose, Urea, Citric acid, Sodium hydroxide, Maltose, Sodium pca, Sodium chloride, Sodium lactate, Trehalose, Allantoin, Sodium hyaluronate, Glucose | 2.0 |
| Perfume oil PO1, PO2, PO3, PO4, or PO5 | Perfume | 0.5 |

**Table 40: Daily Well Aging, SPF 30, UVA/UVB balanced, w/o Octocrylene**

| **Ingredients** | **INCI** | **Amount** |
|---|---|---|
| Lanette^{®} O | Cetearyl alcohol | 1.5 |
| Emulsiphos^{®} (677660) | Potassium cetyl phosphate | 2.5 |
| | Hydrogenated palm glycerides | |
| SymUrban^{®} (386557) | Benzylidene dimethoxydimethylindanone | 0.3 |
| Neo Heliopan^{®} 357 (622501) | Butyl methoxydibenzoylmethane | 4.0 |
| Neo Heliopan^{®} OS (131494) | Ethylhexyl salicylate | 5.0 |
| Neo Heliopan^{®} BMT(102814) | Bis-ethylhexyloxyphenol methoxyphenyl triazine | 3.0 |
| Uvasorb^{®} HEB | Diethylhexyl butamido triazone | 2.0 |
| SymMollient^{®} S (181598) | Cetearyl nonanoate | 1.0 |
| Cetiol^{®} Sensoft | Propylheptyl caprylate | 4.0 |
| Tegosoft^{®} TN | C12-15 alkyl benzoate | 11.0 |
| SymDecanox^{™} HA (972276) | Caprylic/capric triglyceride | 1.0 |
| | Hydroxymethoxyphenyl decanone | |
| Isoadipate (660014) | Diisopropyl adipate | 7.5 |
| EDTA^{®} BD | Disodium edta | 0.1 |
| Carbopol^{®} ETD 2050 Polymer | Carbomer | 0.3 |
| Keltrol^{®} CG-T | Xanthan gum | 0.2 |
| Aqua/Water | Aqua | ad 100 |
| 1,2-heptanediol | 1,2-heptanediol | 0.7 |
| 2,3-octanediol | 2,3-octanediol | 0.3 |
| Glycerin 99.5 % | Glycerin, Aqua | 1.0 |
| Lanette^{®} E | Sodium cetearyl sulfate | 0.8 |
| Neo Heliopan^{®} Hydro (103089) | Phenylbenzimidazole sulfonic acid | 3.0 |
| Biotive^{®} L-Arginine (621277) | Arginine | 1.5 |
| Propylene Glycol (160221) | Propylene glycol | 3.0 |
| Sodium Hydroxide 10% solution | Aqua, Sodium hydroxide | 2.0 |
| SymSave^{®} H (979940) | Hydroxyacetophenone | 0.6 |
| Phenoxyethanol | Phenoxyethanol | 0.5 |
| Dragosine^{®} (844033) | Carnosine | 0.2 |
| SymVital^{®} AR (974839) | Zingiber officinale (ginger) root extract | 0.2 |
| Frescolat^{®} ML Cryst (399952) | Menthyl lactate | 0.5 |
| Perfume oil PO1, PO2, PO3, PO4, or PO5 | Perfume | 1.0 |
| Tapioca Pure | Tapioca starch | 2.0 |

**Table 41: Anti-Wrinkle Day Emulsion, expected SPF 15**

| **Ingredients** | **INCI** | **Amount** |
|---|---|---|
| Emulsiphos^{®} (677660) | Potassium cetyl phosphate, Hydrogenated palm glycerides | 2.5 |
| Neo Heliopan^{®} BMT(102814) | Bis-ethylhexyloxyphenol methoxyphenyl triazine | 2.0 |
| Neo Heliopan^{®} 303 (600154) | Octocrylene | 2.0 |
| Neo Heliopan^{®} OS (131494) | Ethylhexyl salicylate | 5.0 |
| Isoadipate (660014) | Diisopropyl adipate | 2.0 |
| SymMollient^{®} S (181598) | Cetearyl nonanoate | 2.0 |
| Caprylic/Capric Triglyceride | Caprylic/capric triglyceride | 3.0 |
| Dragoxat^{®} 89 (109854) | Ethylhexyl isononanoate | 1.0 |
| PCL-Liquid^{®} 100 (660089) | Cetearyl ethylhexanoate | 3.0 |
| SymUrban^{®} (386557) | Benzylidene dimethoxydimethylindanone | 0.3 |
| SymDecanox^{™} HA (972276) | Caprylic/capric triglyceride | 2.0 |
| | Hydroxymethoxyphenyl decanone | |
| Edeta^{®} BD | Disodium edta | 0.1 |
| Keltrol^{®} CG-SFT | Xanthan gum | 0.2 |
| Cosmedia^{®} SP | Sodium Polyacrylate | 0.4 |
| Aqua/Water | Aqua | ad 100 |
| 2,3-heptanediol | 2,3-heptanediol | 0.3 |
| 1,2-octanediol | 1,2-octanediol | 0.2 |
| Glycerin (657319) | Glycerin | 3.0 |
| Neo Heliopan^{®} Hydro (103089) | Phenylbenzimidazole sulfonic acid | 2.0 |
| Biotive^{®} L-Arginine (621277) | Arginine | 1.0 |
| Sodium Hydroxide 10% solution | Aqua, Sodium hydroxide | 1.2 |
| Dragosine^{®} (844033) | Carnosine | 0.2 |
| SymSave^{®} H (979940) | Hydroxyacetophenone | 0.5 |
| SymDiol^{®} 68 (108580) | 1,2-hexanediol, Caprylyl glycol | 0.3 |
| Phenoxyethanol (130089) | Phenoxyethanol | 0.2 |
| Hydroviton^{®} PLUS 2290 (510027) | Aqua, Pentylene glycol, Glycerin, Fructose, Urea, Citric acid, Sodium hydroxide, Maltose, Sodium pca Sodium chloride, Sodium lactate, Trehalose, Allantoin, Sodium hyaluronate, Glucose | 3.0 |
| SymLift^{®} (745396) | Aqua, Trehalose, Glycerin, Pentylene glycol, Beta-glucan, Hordeum vulgare seed extract, Sodium hyaluronate, 1,2-hexanediol, Caprylyl glycol | 5.0 |
| Macadamia Oil (refined) (613139) | Macadamia integrifolia seed oil | 0.2 |
| SymVital^{®} AR (974839) | Zingiber officinale (ginger) root extract | 0.2 |
| Actipone^{®} Laminaria Saccharina GW (146705) | Glycerin, Aqua, Laminaria saccharina extract | 1.0 |
| Ethanol 96 % | Alcohol denat. | 3.0 |
| Sensocel^{®} 10 | Cellulose | 1.0 |
| Sipernat^{®} 11 PC | Hydrated silica | 1.0 |
| Perfume oil PO1, PO2, PO3, PO4, or PO5 | Perfume oil PO1, PO2, PO3, PO4, or PO5 | 0.4 |

**Table 42: Daily Tattoo Care, expected SPF 10, UVA/UVB balanced**

| **Ingredients** | **INCI** | **Amount** |
|---|---|---|
| Aqua/Water | Aqua | ad 100 |
| 1,2-heptanediol/ 2,3-heptanediol (95:5 w/w %) | 1,2-heptanediol, 2,3-heptanediol | 0.5 |
| Neo Heliopan^{®} Hydro (103089) | Phenylbenzimidazole sulfonic acid | 2.5 |
| Biotive^{®} L-Arginine (621277) | Arginine | 1.0 |
| Sodium Hydroxide 10% solution | Aqua, Sodium hydroxide | 2.8 |
| SymSave^{®} H (979940) | Hydroxyacetophenone | 0.5 |
| SymDiol^{®} 68 (108580) | 1,2-hexanediol, Caprylyl glycol | 0.5 |
| Edeta^{®} BD | Disodium EDTA | 0.1 |
| Emulsiphos^{®} (677660) | Potassium cetyl phosphate | 2.5 |
| | Hydrogenated palm glycerides | |
| Cetiol^{®} SB 45 | Butyrospermum parkii butter | 2.0 |
| Lanette^{®} 16 | Cetyl alcohol | 2.0 |
| Lanette^{®} O | Cetearyl alcohol | 3.5 |
| SymMollient^{®} S (181598) | Cetearyl nonanoate | 1.0 |
| Caprylic/Capric Trigjyceride | Caprylic/capric triglyceride | 6.0 |
| Corapan^{®} TQ (182585) | Diethylhexyl 2,6-naphthalate | 1.0 |
| Dragoxat^{®} 89 (109854) | Ethylhexyl isononanoate | 2.0 |
| Isoadipate (660014) | Diisopropyl adipate | 3.0 |
| Neo Heliopan^{®} BMT(102814) | Bis-ethylhexyloxyphenol methoxyphenyl triazine | 2.0 |
| Neo Heliopan^{®} OS (131494) | Ethylhexyl salicylate | 5.0 |
| SymCare^{®} O (510634) | Hexyldecanol, Pentylene glycol, 4-t-butylcyclohexanol, Bisabolol, Cetylhydroxyproline palmitamide, Hydroxyphenyl propamidobenzoic acid, Stearic acid, Brassica campestris (rapeseed) sterols, Zingiber officinale (ginger) root extract | 4.0 |
| SymDecanox^{™} HA (972276) | Caprylic/capric triglyceride Hydroxymethoxyphenyl decanone | 1.0 |
| Cetiol^{®} Ultimate | Tridecane, Undecane | 4.0 |
| Carbopol^{®} Ultrez 21 Polymer | Acrylates/c10-30 alkyl acrylate crosspolymer | 0.4 |
| Keltrol^{®} CG-T | Xanthan gum | 0.2 |
| SymGlucan^{®} (151719) | Aqua, Glycerin, 1,2-hexanediol, Caprylyl glycol, Beta-glucan | 2.0 |
| Perfume oil PO1, PO2, PO3, PO4, or PO5 | Perfume | 0.5 |
| Tapioca Pure | Tapioca starch | 2.0 |

**Table 43: Sensitive Day Care Cream, expected SPF 15**

| **Ingredients** | **INCI** | **Amount** |
|---|---|---|
| Lanette^{®} O | Cetearyl alcohol | 4.0 |
| Emulsiphos^{®} (677660) | Potassium cetyl phosphate, Hydrogenated palm glycerides | 2.0 |
| Cetiol^{®} AB | C12-15 alkyl benzoate | 1.0 |
| Cetiol^{®} OE | Dicaprylyl ether | 1.0 |
| SymMollient^{®} S (181598) | Cetearyl nonanoate | 1.0 |
| Neo Heliopan^{®} BMT(102814) | Bis-ethylhexyloxyphenol methoxyphenyl triazine | 2.0 |
| Neo Heliopan^{®} 303 (600154) | Octocrylene | 3.0 |
| Grape Seed Oil (Refined) (604680) | Vitis vinifera seed oil | 1.0 |
| Corapan^{®} TQ (182585) | Diethylhexyl 2,6-naphthalate | 3.0 |
| Eumulgin^{®} SG | Sodium stearoyl glutamate | 1.0 |
| SymCare^{®} O (510634) | Hexyldecanol, Pentylene glycol, 4-t-butylcyclohexanol, Bisabolol, Cetylhydroxyproline palmitamide, Hydroxyphenyl propamidobenzoic acid, | 3.0 |
| | Stearic acid, Brassica campestris (rapeseed) sterols, Zingiber officinale (ginger) root extract | |
| Softisan^{®} 100 | Hydrogenated coco-glycerides | 1.0 |
| Eutanol^{®} G | Octyldodecanol | 2.0 |
| Caprylic/Capric Triglyceride | Caprylic/capric triglyceride | 5.0 |
| Carbopol^{®} Ultrez 10 Polymer | Carbomer | 0.3 |
| Aqua/Water | Aqua | ad 100 |
| 1,2-octanediol | 1,2-octanediol | 0.2 |
| 1,2-heptanediol | 1,2-heptanediol | 0,5 |
| Neo Heliopan^{®} Hydro (103089) | Phenylbenzimidazole sulfonic acid | 3.0 |
| Biotive^{®} L-Arginine (621277) | Arginine | 1.0 |
| Sodium Hydroxide 10% solution | Aqua, Sodium hydroxide | 2.0 |
| Edeta^{®} BD | Disodium EDTA | 0.1 |
| Glycerin (657319) | Glycerin | 5.0 |
| SymSave^{®} H (979940) | Hydroxyacetophenone | 0.5 |
| Phenoxyethanol (130089) | Phenoxyethanol | 0.5 |
| Hydroviton^{®} PLUS 2290 (510027) | Aqua, Pentylene glycol, Glycerin, Fructose, Urea, Citric acid, Sodium hydroxide, Maltose, Sodium pca, Sodium chloride, Sodium lactate, Trehalose, Allantoin, Sodium hyaluronate, Glucose | 3.0 |
| D-Panthenol 75 W | Panthenol, Aqua | 2.0 |
| Tapioca Pure | Tapioca starch | 4.0 |
| Actipone^{®} Licorice (102908) | Aqua, Butylene glycol, Glycyrrhiza glabra root extract | 2.0 |
| Sodium Hydroxide 10% solution | Aqua, Sodium hydroxide | 1.5 |

**Table 44: Cream, expected SPF 15, UVA/UVB**

| **Ingredients** | **INCI** | **Amount** |
|---|---|---|
| Aqua/Water | Aqua | ad 100 |
| 2,3-octanediol | 2,3-octanediol | 0.7 |
| SymSol^{®} PF-3 (358712) | Aqua, Pentylene glycol, Sodium lauryl sulfoacetate, Sodium oleoyl sarcosinate, Sodium chloride, Sodium oleate | 2.0 |
| Neo Heliopan^{®} Hydro (103089) | Phenylbenzimidazole sulfonic acid | 2.5 |
| Biotive^{®} L-Arginine (621277) | Arginine | 1.0 |
| Sodium Hydroxide 10% solution | Aqua, Sodium hydroxide | 1.3 |
| Aqua Keep 10SH-NFC | Sodium acrylates crosspolymer-2 | 2.0 |
| SymSave^{®} H (979940) | Hydroxyacetophenone | 0.5 |
| Dragosine^{®} (844033) | Carnosine | 0.1 |
| Hydrolite^{®} 5 (616751) | Pentylene glycol | 2.5 |
| Neo Heliopan^{®} OS (131494) | Ethylhexyl salicylate | 3.0 |
| Neo Heliopan^{®} 303 (600154) | Octocrylene | 3.0 |
| Neo Heliopan^{®} BMT(102814) | Bis-ethylhexyloxyphenol methoxyphenyl triazine | 1.5 |
| Neo Heliopan^{®} HMS (182573) | Homosalate | 3.0 |
| SymMollient^{®} S (181598) | Cetearyl nonanoate | 1.0 |
| Isoadipate (660014) | Diisopropyl adipate | 2.5 |
| SymRelief^{®} 100 (367692) | Bisabolol | 0.2 |
| | Zingiber officinale root extract | |
| SymDecanox^{™} HA (972276) | Caprylic/capric triglyceride | 1.5 |
| | Hydroxymethoxyphenyl decanone | |
| SymDetox^{®} (355267) | 3,3,5-trimethylcyclohexyl succinate dimethylamide | 0.5 |
| SymUrban^{®} (386557) | Benzylidene dimethoxydimethylindanone | 0.5 |
| Edeta^{®} BD | Disodium edta | 0.1 |
| Frescolat^{®} X-Cool (370610) | Menthyl ethylamido oxalate | 0.5 |
| SymBright^{™} 2036 (352400) | Sclareolide | 0.1 |
| SymWhite^{®} PLUS (510494) | Caprylic/capric triglyceride, Pentylene glycol Phenylethyl resorcinol, Bisabolol, Butyl methoxydibenzoylmethane | 1.3 |
| Keltrol^{®} CG-BT | Xanthan gum | 0.2 |
| FOOD COLOR BROWN E172+E171 POWDER (656623) | Titanium dioxides (ci 77891), Iron oxides (ci 77492), Iron oxides (ci 77491), Iron oxides (ci. 77499) | 2.0 |
| Perfume oil PO1, PO2, PO3, PO4, or PO5 | Perfume | 0.5 |
| SymGlucan^{®} (151719) | Aqua, Glycerin, 1,2-hexanediol, Caprylyl glycol, Beta-glucan | 5.0 |

**Table 45: Air freshener A/F spray water based)**

| **Ingredients** | **Amount** |
|---|---|
| Fragrance | 4 |
| Neo Heliopan^{®} Flat (INCI: Homosalate, Octocrylene, Bis-Ethylhexyloxyphenol Methoxyphenyl Triazine, Butyl Methoxydibenzoylmethane, Ethylhexyl Salicylate) | 1.0 |
| Ethanol | 8 |
| PEG-40 Hydrogenated Castor Oil | 5 |
| 1,2-heptanediol | 1 |
| Aqua | add to 100 |

| | |
|---|---|
| Colorless liquid, pH neutral | |

**Table 61: All-purpose cleaner**

| **No** | **Ingredients** | **Amount** |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 1,2-heptanediol | 1 |
| 6 | Aqua | add to 100 |

| | | |
|---|---|---|
| Colorless liquid, pH 7 | | |

**Table 46: All-purpose cleaner**

| **No** | **Ingredients** | **Amount** |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 2,3-octanediol | 0.1 |
| 6 | 1,2-heptanediol | 0.9 |
| 7 | Aqua | add to 100 |

| | | |
|---|---|---|
| Colorless liquid, pH 7 | | |

**Table 47: All-purpose cleaner, alkaline**

| **No** | **Ingredients** | **Amount** |
|---|---|---|
| 1 | Coco-Glycoside | 1 |
| 2 | Benzalkonium Chloride | 0.2 |
| 3 | Sodiumbicarbonat | 0.1 |
| 4 | Tri-sodiumcitrate-dihydrate | 0.1 |
| 5 | Methylglycinediacetic acid | 0.1 |
| 6 | 1,2-Benzisothiazol-3(2H)-on | 0.1 |
| 7 | Fragrance | 0.1 |
| 8 | 1,2-heptanediol | 0.5 |
| 9 | Aqua | add to 100 |

| | | |
|---|---|---|
| Colorless liquid, pH 9 | | |

**Table 48: Cleaner, liquid, citric acid**

| **No** | **Ingredients** | **Amount** |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol | 0.5 |
| 5 | Fragrance | 0.3 |
| 6 | Aqua | add 100 |

| | | |
|---|---|---|
| Liquid of low viscosity, pH 2 | | |

**Table 49: Cleaner, liquid, citric acid**

| **No** | **Ingredient** | **Amount** |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol/2,3-heptanediol 90 : 10 | 0.5 |
| 5 | Fragrance | 0.3 |
| 6 | Aqua | add 100 |

| | | |
|---|---|---|
| Liquid of low viscosity, pH 2 | | |

**Table 50: Detergent liquid light duty**

| **No** | **Ingredients** | **Amount** |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-octanediol | 0.1 |
| 9 | 1,2-heptanediol | 0.9 |
| 10 | Fragrance | 0.5 |
| 11 | Aqua | add to 100 |

| | | |
|---|---|---|
| Liquid, pH 7.3 | | |

**Table 51: Detergent liquid light duty**

| **No** | **Ingredients** | **Amount** |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-heptanediol | 0.1 |
| 9 | 1,2-heptanediol | 0.9 |
| 10 | Fragrance | 0.5 |
| 11 | Aqua | add to 100 |

| | | |
|---|---|---|
| Liquid, pH 7.3 | | |

**Table 52: Dishwash liquid manual**

| **No** | **Ingredients** | **Amount** |
|---|---|---|
| 1 | Sodium Laureth Sulfate | add 100 |
| 2 | Ethanol | 2.9 |
| 3 | lauramine oxide | 7.7 |
| 4 | propylene glycol | 1.9 |
| 5 | Phenoxyethanol | 0.1 |
| 6 | Benzisothiazolinone, Methylisothiazolinone, Laurylamine Dipropylenediamine | 0.1 |
| 7 | 1,2-heptanediol | 0.5 |
| 8 | sodium chloride | 3.85 |
| 9 | Fragrance | 0.3 |

| | | |
|---|---|---|
| Colorless liquid, pH 8.5 | | |

**Table 53: Fabric softener**

| **No** | **Ingredients** | **Amount** |
|---|---|---|
| 1 | Di-(Talg Carboxyethyl) Hydroxyethyl methylammonium-methosulfat | 5.5 |
| 2 | Simethicone | 0.3 |
| 3 | 2-octyl-2H-isothiazol-3-one | 0.1 |
| 4 | 1,2-heptanediol | 0.5 |
| 5 | Fragrance | 0.2 |
| 6 | Neo Heliopan^{®} Flat (INCI: Homosalate, Octocrylene, Bis-Ethylhexyloxyphenol Methoxyphenyl Triazine, Butyl Methoxydibenzoylmethane, Ethylhexyl Salicylate) | 0.5 |
| 7 | Aqua | add 100 |

| | | |
|---|---|---|
| Liquid, pH 3 | | |

**Table 54: Fabric softener concentrate, encapsulated**

| **No** | **Ingredients** | **Amount** |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 1,2-heptanediol | 0.5 |
| 8 | Neo Heliopan^{®} Flat (INCI: Homosalate, Octocrylene, Bis-Ethylhexyloxyphenol Methoxyphenyl Triazine, Butyl Methoxydibenzoylmethane, Ethylhexyl Salicylate) | 2.0 |
| 9 | Fragrance | 0.6 |
| 10 | Aqua | add 100 |

| | | |
|---|---|---|
| White liquid, pH 2.5 | | |

**Table 55: Fabric softener concentrate, encapsulated**

| **No** | **Ingredients** | **Amount** |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 2,3-heptanediol | 0.05 |
| 8 | 1,2-heptanediol | 0.5 |
| 9 | Triasorb | 0.2 |
| 10 | Fragrance | 0.6 |
| 11 | Aqua | add 100 |

| | | |
|---|---|---|
| White liquid, pH 2.5 | | |

**Table 56: Hand soap, liquid**

| **No** | **Ingredients** | **Amount** |
|---|---|---|
| 1 | Sodium Laureth Sulfate | 18 |
| 2 | Cocoamidopropylbetaine | 6 |
| 3 | Cocamide DEA | 3 |
| 4 | citric acid | 0.5 |
| 5 | sodium chloride | 1.9 |
| 6 | Glycerol | 2 |
| 7 | Fragrance | 0.2 |
| 8 | 1,2-heptanediol | 0.25 |
| 9 | Aqua | add 100 |

| | | |
|---|---|---|
| Slightly colored liquid, pH 6 | | |

**Table 57: Rim block gel**

| **No** | **Ingredients** | **Amount** |
|---|---|---|
| 1 | Sodium Laureth Sulfate | 11 |
| 2 | Trideceth-9 | 2 |
| 3 | Xanthan gum | 2 |
| 4 | Hydroxyethylcellulose | 0.5 |
| 5 | Citric Acid | 0.4 |
| 6 | Ethanol | 6 |
| 7 | Fragrance | 5 |
| 8 | 1,2-heptanediol | 0.5 |
| 9 | Neo Heliopan^{®} BMT (Bis-Ethylhexyloxyphenol Methoxyphenyl Triazine) | 1.0 |
| 10 | Aqua | add 100 |

| | | |
|---|---|---|
| Colorless paste/gel, pH = 4.5 | | |

**Table 58: Scent Lotion with capsules**

| **No** | **Ingredients** | **Amount** |
|---|---|---|
| 1 | microcrystalline cellulose/cellulose gum | 1 |
| 2 | PEG-40 hydrogenated Castor oil/ Trideceth-9 | 0.5 |
| 3 | encapsulated perfume oil | 1 |
| 4 | 2-octyl-2H-isothiazol-3-one | 0.1 |
| 5 | Fragrance | 5 |
| 6 | 1,2-heptanediol | 1 |
| 7 | Neo Heliopan^{®} BMT (Bis-Ethylhexyloxyphenol Methoxyphenyl Triazine) | 2.0 |
| 8 | Aqua | add 100 |

| | | |
|---|---|---|
| White emulsion, pH neutral | | |

**Table 59: Cleaner, liquid, lactic acid**

| **No** | **Ingredients** | **Amount** |
|---|---|---|
| 1 | Sodium Laureth Sulfate | 1.8 |
| 2 | Trideceth-9 | 2.1 |
| 3 | Xanthan gum | 0.35 |
| 4 | Lactic Acid | 2,8 |
| 5 | 1,2-heptanediol | 1 |
| 6 | Fragrance | 0.3 |
| 7 | Aqua | add 100 |

| | | |
|---|---|---|
| Colorless liquid, pH 2,0 | | |

**Table 60: Cleaner, liquid, citric acid**

| **No** | **Ingredients** | **Amount** |
|---|---|---|
| 1 | Sulfonic acid, C 13-17-sec-Alkyl-, Sodium salt | 1 |
| 2 | Xanthan gum | 0-3 |
| 3 | citric acid | 5 |
| 4 | 1,2heptanediol | 0.25 |
| 5 | Fragrance | 0.3 |
| 6 | Aqua | add 100 |

| | | |
|---|---|---|
| Liquid, pH 2 | | |

## Claims

1. A cosmetic or pharmaceutical composition or homecare product, comprising or consisting of
(a1) 1,2-heptanediol;
(b1) at least one solid UV filter; and
(c1) optionally at least one active substance for a cosmetic or pharmaceutical composition or a homecare product and/or additive;
or
(a2) 2,3-heptanediol;
(b2) at least one solid UV filter; and
(c2) optionally at least one active substance for a cosmetic or pharmaceutical composition or a homecare product and/or additive.

2. Cosmetic or pharmaceutical composition or homecare product according to claim 1, wherein the at least one solid UV filter is selected from the group consisting of:
- 1-(4-tert-Butylphenyl)-3-(4-methoxyphenyl)-propane-1,3-dione (INCI: Butyl Methoxydibenzoylmethane; Avobenzon),
- Sodium salt of 2,2'-bis(1,4-phenylene)-1H-benzimidazole-4,6-disulfonic acid (INCI: Disodium Phenyl Dibenzimidazole Tetrasulfonate),
- 2-Hydroxy-4-methoxybenzophenone (INCI: Benzophenone-3),
- 2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol),
- Isopentyl-4-methoxycinnamate (INCI: Isoamyl p-Methoxycinnamate; Amiloxate),
- 2-Phenylbenzimidazole-5-sulfonic acid and its potassium, sodium and triethanolamine salts (INCI: Phenylbenzimidazole Sulfonic Acid),
- 3-(4-Methylbenzylidene)-d1 camphor (INCI: 4-Methylbenzylidene Camphor),
- 1,3,5-Triazine-2,4,6-tris[1,1'-biphenyl]-4-yl (INCI: Tris-biphenyl Triazine, incl. Nanomaterial),
- 2-Hydroxy-4-methoxybenzophenone- 5-sulfonic acid and its sodium salt (INCI: Benzophenone-4/Benzophenone-5), and/or
- Zinc Oxide (INCI: Zinc Oxide),
- Benzoic acid, 2-[-4-(diethylamino)-2-hydroxybenzoyl]-hexylester (INCI: Diethylamino Hydroxybenzoyl Hexyl Benzoate),
- 2,4,6-Trianilino-(p-carbo-2'-ethylhexyl-1'-oxy)-1,3,5-triazine (INCI: Ethylhexyl Triazone),
- alpha-(2-Oxoborn-3-ylidene)-toluene-4-sulphonic acid and its salts (INCI: Benzylidene Camphor Sulfonic Acid),
- Polymer of N-{(2 and 4)-[(2-oxoborn-3-ylidene)-methyl]-benzyl}-acrylamide (INCI: Polyacrylamido Methyl Benzylidene Camphor),
- 3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid),
- Ethoxylated Ethyl-4-aminobenzoate (INCI: PEG-25 PABA),
- Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone),
- Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane),
- 3-Benzylidene camphor (INCI: 3-Benzylidene Camphor), and/or
- Phenylene-bis-diphenyltriazine (Triasorb B),
- Neo Heliopan^{®} Flat, and/or
- Titanium dioxide (INCI: Titanium Dioxide),
and mixtures of two or more of the aforesaid UV filters.

3. Cosmetic or pharmaceutical composition or homecare product according to claim 1 or claim 2, wherein the at least one active substance for a cosmetic or pharmaceutical composition or homecare product and/or additive is selected from the group consisting of agents against ageing of the skin, anti-microbial agents, antioxidants, chelating agents, emulsifiers, preservatives, green and synthetic polymers, skin-cooling agents, rheology additives, oils, fragrances or perfume oils, polyols, and mixtures of two or more of the aforementioned substances.

4. Cosmetic or pharmaceutical composition or homecare product according to any one of claims 1 to 3, further comprising at least one additional UVA filter, and/or further comprising at least one additional UVB filter, and/or further comprising at least one additional broadband filter, and/or further comprising at least one pigment, or mixtures thereof.

5. Cosmetic or pharmaceutical composition or homecare product according to any one of claims 1 to 4, further comprising at least one oil component selected from the group consisting of plant oils, hydrocarbons, fatty alcohols, fatty acid esters, and mixtures of two or more of the aforesaid liquid oil components.

6. Cosmetic or pharmaceutical composition or homecare product according to any one of claims 1 to 5, comprising the 1,2-heptanediol or the 2,3-heptanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, most preferred in an amount of 0.5 to 1.0 % by weight, based on the total weight of the composition or homecare product.

7. Cosmetic or pharmaceutical composition or homecare product according to any one of claims 1 to 6, comprising
- the 2,3-heptanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, most preferred 0.5 to 1.0 % by weight, based on the total weight of the composition or homecare product; preferably in an amount of 0.001 to 0.5 % by weight, more preferably in an amount of 0.005 to 0.1 % by weight and most preferably in an amount of 0.01 to 0.075 % by weight, based on the total weight of the composition or homecare product; or
- the 2,3-alkanediol as linear alkanediol or of the mixture comprising at least one first linear alkanediol and one or more second linear alkanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, most preferred in an amount of 0.5 to 1.0 % by weight, based on the total weight of the composition or homecare product;
preferably in an amount of 0.001 to 0.1 % by weight, more preferably in an amount of 0.005 to 0.1 % by weight and most preferably in an amount of 0.01 to 0.075 % by weight, based on the total weight of the composition or homecare product.

8. Cosmetic or pharmaceutical composition according to any one of claims 1 to7, comprising the at least one solid UV filter and optionally further UV filters in an amount of 0.1 to 50.0 % by weight, particularly in an amount of 0.5 to 45.0 % by weight, most particularly in an amount of 1.0 to 40 % by weight, based on the total weight of the composition or homecare product.

9. Cosmetic or pharmaceutical composition according to any one of claims 1 to 8 in the form of
i. a dispersion comprising the at least one oil component in an amount of ≥ 1% by weight, in particular in an amount of ≥ 3 % by weight, in particular wherein the cosmetic or pharmaceutical composition is in the form of an emulsion, in particular a O/W emulsion, a W/O emulsion, a multiple emulsion, a hydrodispersion gel, a balm, a multiple emulsion of the water-in-oil type (W/O/W) or of the oil-in-water type (O/W/O), PIT emulsion, Pickering emulsion a micro-emulsion, a liquid, a lotion, a suspension, a milk, an ointment, a paste, a gel, a cream based, an oil based or a liposome-based formulation;
ii. a water free formulation; or
iii. an aqueous or aqueous/alcoholic, in particular aqueous/ethanolic, or an aqueous/glycolic solution, comprising the alcohol or glycol in an amount of 0.1 % to ≤ 50 % by weight, based on the total weight of the solution.

10. Cosmetic or pharmaceutical composition according to any one of claims 1 to 9 as a cosmetic, for personal care, in particular for skin care, hair care, scalp care or nail care, as a pharmaceutical, or for animal care.

11. Use of 1,2-heptanediol or 2,3-heptanediol for improving the solubility of a solid UV filter, preferably a UV filter according to claim 2, in a cosmetic or pharmaceutical composition or homecare product; and/or for improving the availability of a solid UV filter, preferably a UV filter according to claim 2, in a cosmetic or pharmaceutical composition on skin upon application of the cosmetic of pharmaceutical composition.
